(19) Europäisches Patentamt
European Patent Office
Office européen des brevets

(11) **EP 2 725 357 A2**

(12) **EUROPEAN PATENT APPLICATION**
published in accordance with Art. 153(4) EPC


(43) Date of publication:
**30.04.2014 Bulletin 2014/18**

(21) Application number: **12802627.5**

(22) Date of filing: **25.06.2012**

(51) Int Cl.:
***G01N 33/52*** *(2006.01)* ***G01N 33/53*** *(2006.01)*
***G01N 33/68*** *(2006.01)* ***G01N 21/64*** *(2006.01)*
***C09K 11/06*** *(2006.01)*

(86) International application number:
**PCT/KR2012/005009**

(87) International publication number:
**WO 2012/177106 (27.12.2012 Gazette 2012/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 KR 20110061404**



(71) Applicant: **Korea Research Institute Of Bioscience And Biotechnology**
**Yuseong-gu, Daejeon 305-806 (KR)**

(72) Inventors:
- **CHUNG, Sang Jeon**
**Daejeon 305-806 (KR)**
- **KIM, Ju Hwan**
**Daejeon 305-806 (KR)**
- **RUCHKINA, Elena**
**Daejeon 305-806 (KR)**
- **KANG, Hyo Jin**
**Daejeon 305-806 (KR)**

(74) Representative: **Forrest, Graham Robert et al**
**Mewburn Ellis LLP**
**33 Gutter Lane**
**London**
**EC2V 8AS (GB)**


(54) **PROBE FOR IFRET AND USAGE OF SAME**


(57) The present invention relates to a probe for iFRET and use thereof. Specifically, the present invention relates to a novel probe for iFRET, a method for preparing the probe for iFRET, a method for searching a target protein-specific binding site or a molecule having the binding site using the probe for iFRET, and a method for imaging the target protein using the probe for iFRET. The probe for iFRET according to the present invention utilizes an amino acid in a protein as a fluorescent donor, unlike the conventional FRET method. Therefore, only one fluorescent material is used, and its emission wavelength is distinct from the intrinsic fluorescence of the protein. Thus, high specificity and sensitivity are ensured, and the quantity, activity and mechanism of various proteins can be analyzed in an easy and accurate manner.


[FIG. 2]

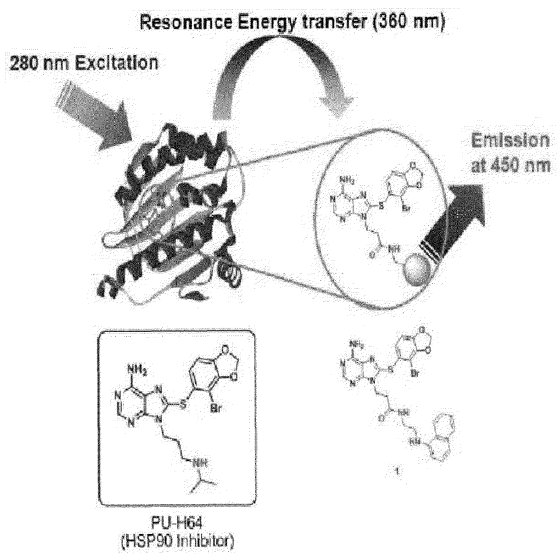


EP 2 725 357 A2

## Description

## BACKGROUND OF THE INVENTION

### 1. Field of the Invention

The present invention relates to a probe for iFRET and use thereof. Specifically, the present invention relates to a novel probe for iFRET, a method for preparing the probe for iFRET, a method of searching for a target protein-specific binding site or a molecule having the binding site using the probe for iFRET, and a method for detecting or imaging the target protein using the probe for iFRET.

### 2. Description of the Related Art

Many different materials have been used as a probe or a reporter for analyzing biomaterials in vivo or in vitro in a variety of fields related to life science. These probe or reporter systems can be largely divided into a method of measuring coloration, color development, or luminescence which is caused by existence of a particular substrate itself or degradation or deformation due to enzymatic reaction of the substrate, and a method of observing a fluorophore or chromophore itself.

The former methods have been used in the target protein-fused form, and applied to ex vivo observations such as Western/Northern blotting, ELISA (enzyme linked immunosorbent assay) or the like as well as in vivo observations such as measurement of protein expression levels or promoter strength, selection of recombinant strains or the like. Representative examples thereof include enzymes such as alkaline phosphatase, peroxidase and xanthine oxidase or the like, and substrates such as luminol, lucigenin, acridinium ester, biotin, fluorescein, and dioxigenin or the like.

However, these compounds are disadvantageous in that their intracellular expressions cause a risk of disturbing in vivo metabolic pathways, which can thereby inhibit cell growth or to induce cell death. In addition, problems due to catalytic properties of the enzymes and low stability of the substrates to be used are a leading cause of experimental errors. Most of the compounds are not biomaterials but chemical compounds, and thus they may be harmful to cells. In this respect, it is another drawback that it is difficult to continuously observe these compounds in vivo. For this reason, the above described probes or reporters have been mainly used as ex vivo probes rather than in vivo probes.

In order to solve the above problems, a reporter protein that itself acts as a probe has been actively researched. A representative example of the protein that itself acts as a probe is a fluorescent protein having the property of emitting energy in the form of light, wherein the energy is emitted during a process in which fluorophores that are excited by absorption of light at a particular wavelength return to the ground state of the lower energy. Representative examples thereof include a green fluorescent protein (GFP) derived from the Aequorea Victoria, jellyfish, and a Discosoma red fluorescent protein (DsRed) derived from the Discosoma sp.

Because these fluorescent proteins themselves emit fluorescence without a substrate or a cofactor, the metabolic disturbance and interference with cytophysiological factors found in the former case are low. Thus, it is possible to apply these proteins in various probe fields, in which the former enzymes are used, and in other fields. That is, the proteins can be used in the fields in which application of the above enzymes and substrates is limited due to their drawbacks. In particular, these proteins generate fluorescence having an intrinsic wavelength by the light of a particular wavelength and thus can be used for directly monitoring complex changes in living cells such as migration, the activities and pathway of a particular protein, cell division and differentiation, etc. under normal physiological conditions without destruction of a cell or an organism. For this reason, the fluorescent proteins have been recognized as the best factors from among the existing methods. Further, the fluorescent proteins have a rigid tertiary structure having fluorophores inside the β-barrel structure. Owing to this structure, the fluorescent proteins are very stable in terms of physicochemical properties and have excellent light stability. Therefore, the proteins can be applied under a wide range of pH and temperature conditions and in the presence of a denaturant or an organic solvent, and experimental reproducibility is maintained in repeated experiments. Thus, the fluorescent proteins are advantageous in that stable experimental results can be obtained, compared to the known probes. They are also advantageous in that when the wild fluorescent proteins are used in combinations with red fluorescent protein (RFP), yellow fluorescent protein (YFP), cyan fluorescent protein (CFP), or blue fluorescent protein (BFP) developed to emit lights at different wavelengths by protein engineering, it is possible to observe various phenomena at the same time, and FRET which takes place when fluorescent proteins emitting lights at different wavelengths are placed close to each other or expressed in a fusion form can be utilized in various fields such as exploration of the presence of a particular substance in a sample, observation of protein-protein interactions, etc.

Because the irradiated lights for GFP, YFP and CFP are similar in terms of wavelength, fluorescence at different wavelengths can be measured at the same time by using the light of a single wavelength for excitation. However, there is a disadvantage in that expensive equipment is required in order to measure each of fluorescences when they are

used together, because the wavelength of emitted light is similar to that of GFP. Further, all proteins having the GFP scaffold need maturation time for fluorophore formation, and it is difficult to observe them at the time when expression has started, and also difficult to use them in an anoxic environment because their structure formation requires oxygen. Furthermore, reactive oxygen species (ROS) generated during oxidation is problematically toxic to biomaterials (fatty acids, amino acids, proteins, nucleic acids). These problems greatly limit the range of application.

Meanwhile, fluorescence resonance energy transfer refers to a phenomenon wherein, if a shorter wavelength dye as an energy donor absorbs the external energy, the excitation energy of the donor is not released as light energy, but is transferred to a longer wavelength excitation dye as an acceptor which is located within a predetermined distance (<10 nm) via a radiationless process, and thus only the fluorescence of the acceptor is emitted at longer wavelength. Miyawaki et al., (Nature, 1997, 388:882-887) developed a new concept of protein interaction analysis system by analyzing the interaction between calmodulin and calmodulin-binding peptide using the principle of FRET. Fluorescent proteins are excited by absorption of light at different intrinsic wavelengths, and the energy is dissipated as light or heat. When the fluorescent protein returns to the ground state, light is emitted at a characteristic wavelength. The principle of fluorescence resonance energy transfer is a phenomenon that light emitted after excitation of a shorter wavelength fluorescent protein induces excitation of a longer wavelength fluorescent protein to emit fluorescence, when two different fluorescent proteins are within the range of 10 to 100 Å. That is, two different fluorescent proteins are attached to the ends of two proteins to be analyzed for their interaction. When two fluorescent proteins are in close proximity within 10 to 100 Å by protein interaction, fluorescence resonance energy transfer takes place. At this time, the protein interaction can be analyzed by detecting the fluorescence. For example, a fluorescent protein excited by light of 488 nm wavelength emits fluorescence of 520 nm wavelength, which acts to excite other coupled fluorescent protein to emit fluorescence of 630 nm wavelength. Therefore, the interaction between two proteins can be analyzed by excitation at 488 nm and detection of fluorescence at 630 nm wavelength. This system has an advantage of analyzing dynamic protein interactions in living cells. However, the detection is possible only when two fluorescent proteins are in close proximity, and excessive protein expression is often needed to detect subtle changes in fluorescence wavelength.

In order to solve the above problems, recent studies have been conducted on the use of protein intrinsic fluorescence. There is a report that the amino acid tryptophan can be applied to FRET (Angew. Chem. Int. Ed. 2006, 45, 4562-4588), and that a multimeric tryptophan biosensor technology for measuring FRET by using tryptophan-binding domains and fluorescent moieties that permit detection thereof is known (US Patent Publication NO. 2008/0311047).

However, the known probes in FRET technology using protein intrinsic fluorescence are disadvantageous in that emission spectra of the protein itself and fluorescent molecule overlap to cause detection of unnecessary signals, leading to a reduction in sensitivity and specificity of the measurement result.

Accordingly, the present inventors have continued to research in order to solve the problems of a wide range of the known probe systems. As a result, they identified new fluorescent molecules using protein intrinsic fluorescence and developed a probe system by conjugation of the fluorescent molecule with a target protein binding site or a molecule having the binding site, thereby completing the present invention.

## SUMMARY OF THE INVENTION

An object of the present invention is to provide a method for searching for a target protein-specific binding site or a molecule having the binding site, comprising: the first step of preparing a probe for intrinsic fluorescence resonance energy transfer (iFRET) by selecting the target protein-specific binding site or the molecule having the binding site from various candidate groups and linking the same with a fluorescent molecule acting as an acceptor for protein intrinsic fluorescence through a direct bond or a linker; the second step of treating the target protein with the probe for iFRET prepared in the first step; and the third step of irradiating light comprising a wavelength for excitation of the amino acids in the target protein and detecting the light-emitting probe for iFRET.

Another object of the present invention is to provide a method for preparing a probe for iFRET, comprising: the first step of preparing a first probe for iFRET by obtaining the target protein-specific binding site or the molecule having the binding site from various candidate groups and linking the same with a first fluorescent molecule acting as an acceptor for protein intrinsic fluorescence through a direct bond or a linker; the second step of treating the target protein with the first probe for iFRET; the third step of irradiating light comprising a wavelength for excitation of the amino acids in the target protein and detecting the light-emitting probe for iFRET; and the fourth step of preparing a second probe for iFRET by linking the target protein-specific binding site or the molecule having the binding site, composing the first probe for iFRET light-emitting from the third step, with a second fluorescent molecule acting as an acceptor for protein intrinsic fluorescence through a direct bond or a linker.

Still another object of the present invention is to provide a probe for iFRET that is prepared by linking a target protein-specific binding site or a molecule having the binding site with a fluorescent molecule acting as an iFRET acceptor selected from the group consisting of the compounds represented by the following Chemical Formulae I to XXII, through a direct bond or a linker.

Still another object of the present invention is to provide a probe for iFRET that is prepared by linking a target protein-specific binding site or a molecule having the binding site with a fluorescent molecule acting as an iFRET acceptor through a direct bond or a linker, wherein R0 value, a distance between fluorescence donor and fluorescence acceptor at which the FRET efficiency is 50%, ranges from 1 to 4 nm.

Still another object of the present invention is to provide a method for detecting or imaging a target protein by iFRET, comprising: the first step of adding the second probe for iFRET prepared by the method of any one of claims 7 to 13 or the probe for iFRET of any one of claims 14 to 18 to a sample containing the target protein; and the second step of irradiating light comprising a wavelength for excitation of the amino acids in the target protein and detecting or tracking the emitted light from the probe for iFRET.

Still another object of the present invention is to provide a compound that is represented by Chemical Formula selected from the group consisting of the following Chemical Formula XXIII, XXIV, or XXVI to XXXII according to the above preparation method, and a salt thereof.

Still another object of the present invention is to provide a method for detecting or imaging a target protein using a probe for intrinsic fluorescence resonance energy transfer (iFRET), wherein the probe for iFRET is prepared by linking a target protein-specific binding site or a molecule having the binding site with a fluorescent molecule acting as an acceptor for the intrinsic fluorescence of the target protein through a direct bond or a linker, and the method comprises the first step of introducing the probe for iFRET into a sample containing the target protein; the second step of alternately irradiating a first light with a wavelength for excitation of the amino acids in the target protein and a second light with a wavelength for excitation of the fluorescent molecule of the probe for iFRET onto the sample prepared in the first step; and the third step of calculating a third emission signal by ratiometric measurement of the first and second emission signals, by analyzing a first emission signal that is generated from the probe for iFRET by irradiating the first light with a wavelength for excitation of the amino acids in the target protein to the sample and a second emission signal that is generated from the probe for iFRET by irradiating the second light with a wavelength for excitation of the fluorescent molecule of the probe for iFRET.

## BRIEF DESCRIPTION OF THE DRAWINGS

FIG. 1 is a diagram showing the action mechanism of the compound of Chemical Formula XXIII exhibiting iFRET;
FIG. 2 is a diagram showing the action mechanism of the compound of Chemical Formula XXIV exhibiting iFRET;
FIGs. 3 to 15 are graphs showing fluorescent characteristics of the fluorescent molecules of the present invention, namely, the compounds represented by Chemical Formulae I to XXII;
FIG. 16 is a graph showing the result of caspase-3 detection using the compound of Chemical Formula XXIII according to the present invention;
FIG. 17 is a spectrum showing fluorescent characteristics of Compound XXIV;
FIG. 18 is a graph showing the result of detecting IFRET signals using Compound XXIV and HSP90 (heat shock protein 90) protein;
FIG. 19 is a graph showing the result of HSP90 detection using the compound of Chemical Formula XXIV;
FIG. 20 is a graph showing changes in iFRET signals of the competitive compound XXIV and HSP90 protein depending on the presence of a binding competitor in a competition assay using PU-H64 and geldanamycin known to bind to HSP90;
FIG. 21 is a graph showing the result of quantitative analysis of HSP90 using the compound of Chemical Formula XXIV according to the present invention;
FIG. 22 is a graph of mass spectrometric analysis of the compound of Chemical Formula XXIII according to the present invention;
FIG. 23 shows the result of detecting streptavidin using the compound of Chemical Formula XXV according to the present invention as the probe for iFRET;
FIG. 24 shows the result of detecting streptavidin using the compound of Chemical Formula XXVI according to the present invention as the probe for iFRET;
FIG. 25 shows the result of detecting streptavidin using the compound of Chemical Formula XXVII according to the present invention as the probe for iFRET; and
FIG. 26 shows the result of detecting streptavidin using the compound of Chemical Formula XXVIII according to the present invention as the probe for iFRET.

## DETAILED DESCRIPTION OF THE PREFERRED EMBODIMENTS

The first aspect of the present invention provides a method for searching for a target protein-specific binding

site or a molecule having the binding site, comprising: the first step of preparing a probe for intrinsic fluorescence resonance energy transfer (iFRET) by selecting the target protein-specific binding site or the molecule having the binding site from various candidate groups and linking the same with a fluorescent molecule acting as an acceptor for protein intrinsic fluorescence through a direct bond or a linker; the second step of treating the target protein with the probe for iFRET prepared in the first step; and the third step of irradiating light comprising a wavelength for excitation of the amino acids in the target protein and detecting the light-emitting probe for iFRET.

As used herein, "FRET" is a non-radiative energy transfer between two fluorophores that have different emission wavelengths, in which the fluorescence donor in an excitation state transfers its excitation energy to the fluorescence acceptor, and emission from the fluorescence acceptor is observed or fluorescence quenching of the fluorescence donor is observed (Lakowicz, J.R. Principles of Fluorescence Spectroscopy, 2nd ed., New York:Plenum Press, 1999).

FRET is also called resonance energy transfer, because the emission spectrum of the fluorescence donor generally overlaps with the absorption spectrum of the fluorescence acceptor and it occurs without the appearance of photons. FRET is the result of long-range dipole-dipole interactions between fluorescence donor and fluorescence acceptor. The FRET energy transfer efficiency varies depending on overlap of emission spectrum of the fluorescence donor and the absorption spectrum of the fluorescence acceptor, quantum efficiency of the fluorescence donor, relative orientation of the transition dipoles of the fluorescence donor and fluorescence acceptor, and distance between the fluorescence donor and fluorescence acceptor. Therefore, the FRET energy transfer efficiency differs depending on the distance between the fluorescence donor and fluorescence acceptor and the relative orientation thereof, and is expressed as follows according to the Forster's Equation.

[Equation 1]

$$E = R_0^6 / (R^6 + R_0^6)$$

wherein E represents FRET efficiency, and R represents the distance between the fluorescence donor and the fluorescence acceptor and is generally 2 to 9 mm, although it varies depending on the kind of fluorescent material. Also, $R_0$ represents the distance between the fluorescence donor and the fluorescence acceptor, at which FRET efficiency is 50%, and it is generally called Forster distance or Forster radius. $R_0$ is expressed as the following Equation.

[Equation 2]

$$R_0 = 0.211[k^2 n^{-4} Q_D J(\lambda)]^{1/6} \text{ (in Å)}$$

wherein $k^2$ is an orientation factor that is usually calculated as 2/3, and has a value in the range from 0 to 4 depending on the relative orientation of emission of the fluorescence donor and absorption of the fluorescence acceptor. n is the refractive index of a medium and is usually approximately 1.334 for water at 25°C, and Qn is the quantum efficiency of the fluorescence donor. J(λ) is the degree of overlap between the emission spectrum of the fluorescence donor and the absorption spectrum of the fluorescence acceptor and is expressed in the unit of $M^{-1}cm^{-1}nm^4$ (Lakowicz, J.R. Principles of Fluorescence Spectroscopy, 2nd ed., New York:Plenum Press, 1999; Patterson et al., Anal. Biochem. 284: 438, 2000; Patterson et al., J. of Cell Sci. 114: 837, 2001).

As used herein, "fluorescence donor" is an amino acid that is involved in the exhibition of protein intrinsic fluorescence. It has been known that most proteins exhibit intrinsic fluorescence and amino acids having aromatic ring structures are involved therein. Examples thereof include tryptophan, tyrosine, and phenylalanine. Tryptophan, tyrosine and phenylalanine are excited at the wavelength between 260 and 300 nm and emit light at the wavelength between 290 to 400 nm, and the light affects up to the wavelength of 440 nm.

As used herein, "fluorescence acceptor" and "fluorescence receptor" are used interchangeably, and mean fluorescent molecules that are excited by protein intrinsic fluorescence to emit light.

The probe for iFRET according to the present invention includes a target protein-specific binding site or a molecule having the binding site (hereinafter, abbreviated to 'binding molecule') and a fluorescent molecule acting as an iFRET acceptor, in which the binding molecule and fluorescent molecule are linked to each other through a direct bond or a linker.

The binding molecule region induces specific binding of the probe for iFRET to the target protein.

In the present invention, the target protein-specific binding site or the molecule having the binding site also comprises a binding site common to two or more of target proteins or a molecule having this binding site.

The binding molecule may be a known compound specifically binding to the target protein, and the target protein-specific binding site or the molecule having the binding site can selected from various candidate groups by the

method for searching for binding molecules according to the present invention.

The fluorescent molecule acting as the acceptor for protein intrinsic fluorescence according to the present invention is preferably excited by the light at the wavelength between 300 to 400 nm which is the emission wavelength of tryptophan, tyrosine and/or phenylalanine exhibiting protein intrinsic fluorescence, and more preferably, it has the emission wavelength above 450 nm which is distinct from the wavelength of protein intrinsic fluorescence.

Meanwhile, when detecting or tracking the emitted light of the probe for iFRET, all ranges of emission wavelengths of the probe for iFRET can be measured, but it is preferable that the light with the longer wavelength at 450 nm or above among the emitted lights of the probe for iFRET is measured. In cells or tissues, cellular autofluorescent materials exhibiting fluorescence by lights with wavelength at 260 to 400 nm, such as nucleic acids, NAD(P)H, collagen or cellular proteins exist, and fluorescence emissions thereof are monitored between 300 to 450 nm. Accordingly, when the probe for iFRET of the present invention is used in cell imaging or in staining of cell extract or tissue, the emission wavelength of the probe for iFRET of the present invention is preferably 450 nm or longer in order to avoid their interference.

Through experiments, the present inventors confirmed that the compounds represented by Chemical Formulae I to XXII of the following Table 1 can be used as the fluorescent molecule acting as an acceptor for protein intrinsic fluorescence (see Examples 1 to 7).

[Table 1]

| Chemical Formula | Structure | Excitation wavelength (nm) | Emission wavelength (nm) |
|---|---|---|---|
| I | 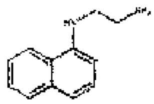 | 340 | 430 |
| II | 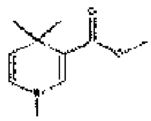 | 360 | 450 |
| III | 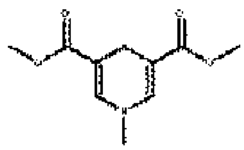 | 390 ~ 400 | 480 |
| IV | 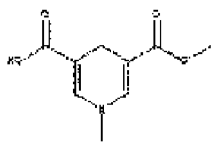 | 390 ~ 400 | 480 |
| V | 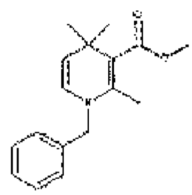 | 330 | 430 |
| VI | 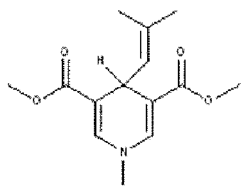 | 390 | 450 |

(continued)

| Chemical Formula | Structure | Excitation wavelength (nm) | Emission wavelength (nm) |
|---|---|---|---|
| VII | 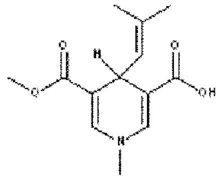 | 380 | 450 |
| VIII | 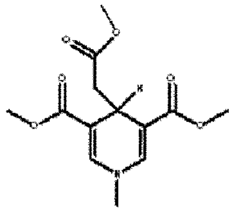 | 370 | 450 |
| IX | 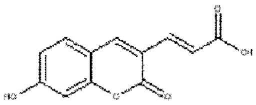 | 370 ~ 380 | 480 |
| X | 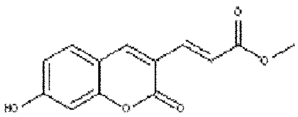 | 430 | 510 |
| XI | 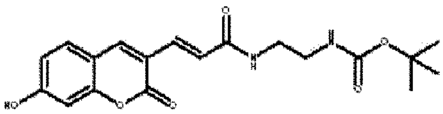 | 380 ~ 420 | 480 |
| XII | 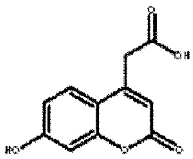 | 330 | 460 |
| XIII | 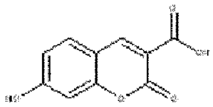 | 400 | 450 |
| XIV | 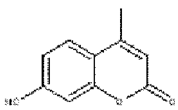 | 370 | 450 |
| XV | 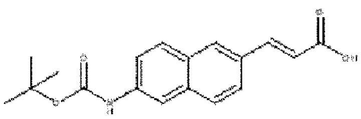 | 330 | 450 |
| XVI | 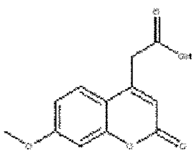 | 330 | 390 |

(continued)

| Chemical Formula | Structure | Excitation wavelength (nm) | Emission wavelength (nm) |
|---|---|---|---|
| XVII | 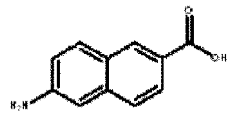 | 330 | 500 |
| XVIII | 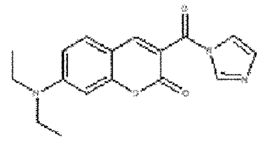 | 400 | 470 |
| XIX | 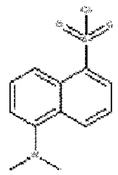 | 340 | 520 |
| XX | 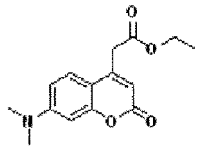 | 370 | 490 |
| XXI | 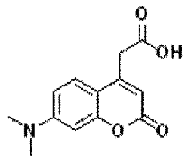 | 370 | 480 |
| XXII | 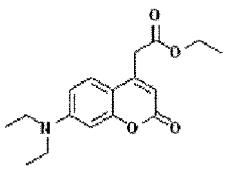 | 370 | 450 |

Derivatives of the compounds represented by Chemical Formulae I to XXII and salts thereof are included in the scope of the present invention, as long as they function as the fluorescent molecules acting as an acceptor for protein intrinsic fluorescence.

Examples of the linker include a variety of alkyls ($-(CH_2)_n-$), aryls, and ethylene glycols ($-(CH_2CH_2O)_n-$; n = 1 to 20) having a proper functional group ($-NH_2$, $CO_2H$, $-N_3$, -C≡C-, etc.) at both ends. These linkers can be linked to the fluorescent molecules possibly through all types of bonds such as carbon-carbon bonds, carbon-oxygen bonds, carbon-nitrogen bonds or the like. Preferably, the linker plays a role in orientation so that the fluorescent molecule of the probe for iFRET quenches to the maximum the intrinsic fluorescence of the target protein emitted at or near the binding site when the binding molecule of the probe for iFRET according to the present invention binds to the target protein. The linker can be properly designed through simulation of the three dimensional orientation, after linking the binding molecule to the target protein.

Meanwhile, when the target protein is treated with the probe for iFRET according to the present invention, typical conditions for fluorescent material treatment may be used in order to detect the light-emitting probe for iFRET after irradiating light for exciting the amino acids in the target protein. Preferably, it may be performed in a solvent selected from the group consisting of water, buffer solution, lower alcohol having 1 to 6 carbon atoms and mixtures thereof; on a biochip, microparticles, or nanoparticles; or in cells or tissues.

In order to detect the light-emitting probe for iFRET after irradiating UV light for exciting the amino acids in the target protein, a microscopic apparatus described in the Korean Patent (a microscoping apparatus for detecting or imaging protein using probe for intrinsic fluorescence resonance energy transfer and method for detecting or imaging

protein using the same) applied on the same date by the present inventors may be used, but typical fluorescence methods may be used. For example, a device for fluorescence analysis may be used. The device for fluorescence analysis may include a filter type- and a monochrome type-fluorescence spectrometer, and so forth. The disclosure of the above Korean patent applied on the same date is herein incorporated in its entirety.

The target protein-specific binding molecule or the molecule having the binding site identified by the method for searching for the target protein-specific binding molecule according to the present invention can be used as a substance for preventing, ameliorating, or treating the target protein-associated diseases or as a part thereof.

The second aspect of the present invention provides a method for preparing a probe for iFRET, comprising: the first step of preparing a first probe for iFRET by obtaining the target protein-specific binding site or the molecule having the binding site from various candidate groups and linking the same with a first fluorescent molecule acting as an acceptor for protein intrinsic fluorescence through a direct bond or a linker; the second step of treating the target protein with the first probe for iFRET; the third step of irradiating light comprising a wavelength for excitation of the amino acids in the target protein and detecting the light-emitting probe for iFRET; and the fourth step of preparing a second probe for iFRET by linking the target protein-specific binding site or the molecule having the binding site, composing the first probe for iFRET light-emitting from the third step, with a second fluorescent molecule acting as an acceptor for protein intrinsic fluorescence through a direct bond or a linker.

Descriptions of the first to third steps of the second aspect of the present invention are the same as in those of the first aspect of the present invention.

The fourth step of the second aspect of the present invention is a step of preparing a second probe for iFRET by linking the target protein-specific binding site or the molecule having the binding site, obtained from the third step, with a second fluorescent molecule acting as an acceptor for protein intrinsic fluorescence through a direct bond or a linker. The first fluorescent molecule of the first probe for iFRET used for searching for the target protein-specific binding molecule according to the present invention and the second fluorescent molecule of the second probe for iFRET can be the same as or different from each other.

Preferably, the first fluorescent molecule and the second fluorescent molecule acting as an acceptor for intrinsic fluorescence of the target protein are each independently excited by the light at the wavelength between 300 to 400 nm which is the emission wavelength of tryptophan, tyrosine and/or phenylalanine exhibiting protein intrinsic fluorescence, and more preferably, they have the emission wavelength at 450 nm and above which is distinct from the wavelength of protein intrinsic fluorescence.

Each of the first fluorescent molecule and the second fluorescent molecule is independently any one of the compounds represented by Chemical Formulae I to XXII of Table 1, but are not limited thereto.

The third aspect of the present invention provides a probe for iFRET that is prepared by linking a target protein-specific binding site or a molecule having the binding site with a fluorescent molecule acting as an iFRET acceptor selected from the group consisting of the compounds represented by the following Chemical Formulae I to XXII, through a direct bond or a linker.

Preferably, the probe for iFRET of the present invention emits light at 450 nm or longer wavelength.

The fourth aspect of the present invention provides a probe for iFRET that is prepared by linking a target protein-specific binding site or a molecule having the binding site with a fluorescent molecule acting as an iFRET acceptor through a direct bond or a linker, wherein R0 value, a distance between fluorescence donor and fluorescence acceptor at which the FRET efficiency is 50%, ranges from 1 to 4 nm.

$R_0$ value of the conventional FRET pairs is approximately 5 nm or more, whereas the iFRET probe of the present invention can be designed so that $R_0$ value between the probe and the fluorescence donor such as Trp etc. in the target protein is 1 to 4 nm. Compared to the conventional FRET probes, the iFRET probe of the present invention can be detected when the probe and the fluorescence donor in the target protein are in close proximity, that is, they are within a distance of approximately 2 nm. Therefore, possible noise due to non-specific binding of low-molecular-weight materials in the general FRET detection can be excluded.

Further, the fifth aspect of the present invention provides a novel compound that is represented by the following Chemical Formula XXIII, XXIV, or XXVI to XXXII, or a salt thereof.

[Chemical Formula XXIII]

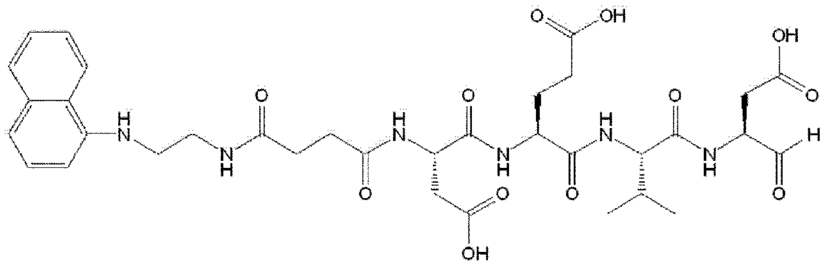

[Chemical Formula XXIV]

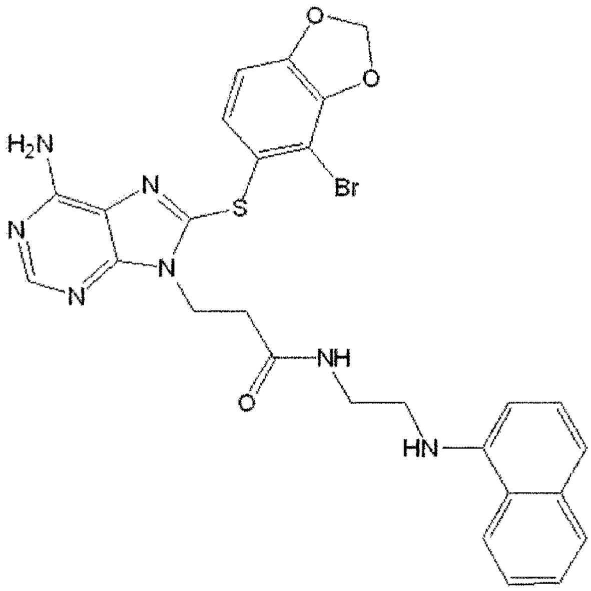

[Chemical Formula XXVI]

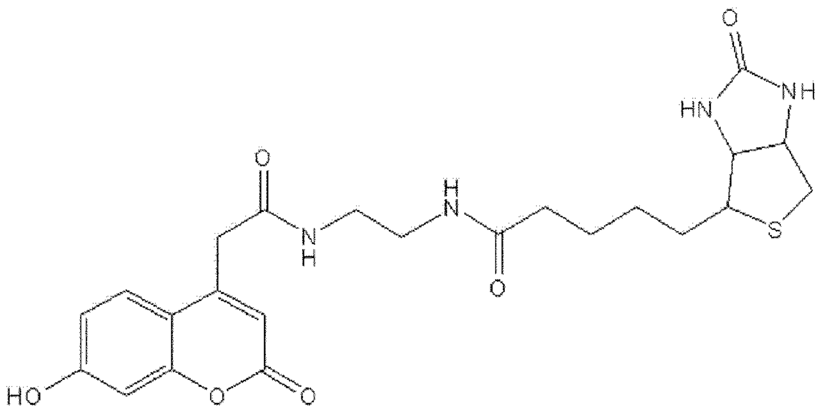

[Chemical Formula XXVII]

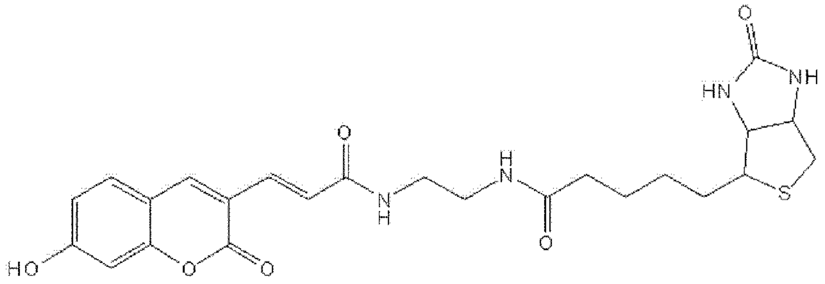

[Chemical Formula XXVIII]

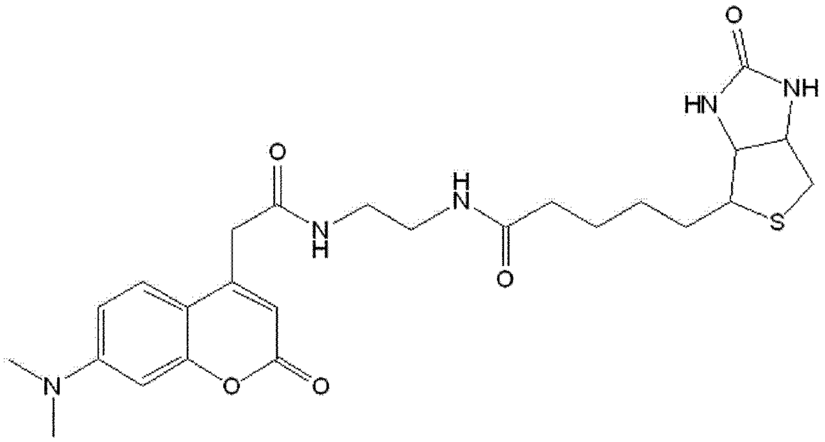

[Chemical Formula XXIX]

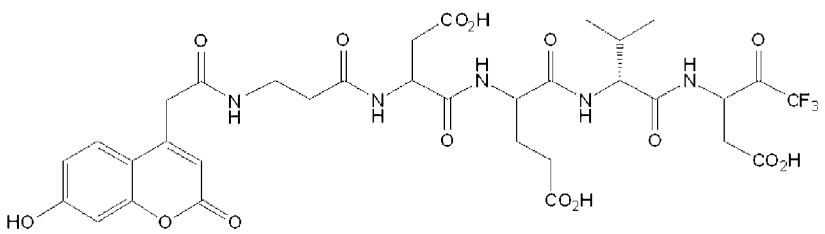

[Chemical Formula XXX]

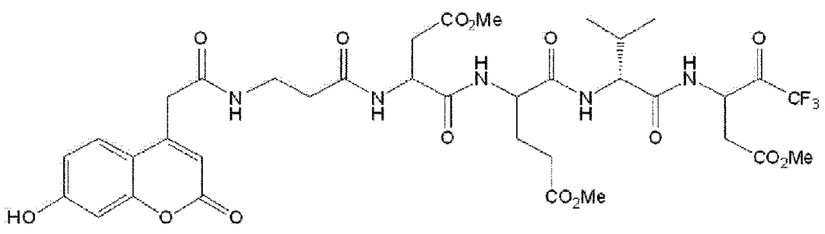

[Chemical Formula XXXI]

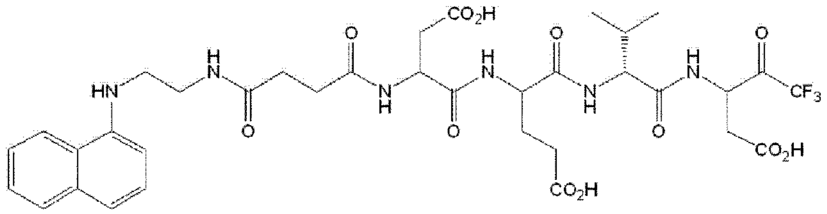

[Chemical Formula XXXII]

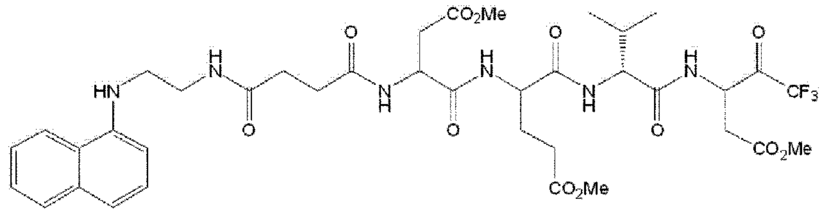

The compound represented by Chemical Formula XXIII, XXIX, XXX, XXXI or XXXII or the salt thereof; the compound represented by Chemical Formula XXVI, XXVII or XXVIII, or the salt thereof; and the compound represented by Chemical Formula XXIV or the salt thereof may be used as the probe for iFRET which targets caspase-3, streptavidin and HSP90 (heat shock protein 90), respectively.

Also, derivatives of the compound represented by Chemical Formula XXIII, XXIV, or XXVI to XXXII are included in the scope of the present invention.

The compound represented by Chemical Formula XXIII or the salt thereof; and the compound represented by Chemical Formula XXIV or the salt thereof utilize 1-naphthylethylenediamine of the following Chemical Formula I as the fluorescent molecule and are prepared by linking the binding molecules capable of binding to the target proteins, caspase-3 and HSP90 with the fluorescent molecule through a direct bond or a linker, respectively. Thus, they can be used as the probes for iFRET that are specific to caspase-3 and HSP90.

[Chemical Formula I]

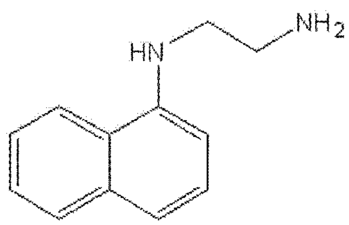

The compound represented by Chemical Formula XXVI, XXVII or XXVIII, or the salt thereof utilizes hydroxycumarin acetic acid of the following Chemical Formula XII, hydroxycumarin acrylic acid of the following Chemical Formula IX, or dimethylcumarin acetic acid of the following Chemical Formula XXI as the fluorescent molecule and is prepared by linking biotin as the binding molecule capable of specifically binding to the target protein streptavidin with the fluorescent molecule through a direct bond or a linker, respectively. Thus, the compound represented by Chemical Formula XXVI, XXVII or XXVIII, or the salt thereof can be used as the probe for iFRET that is specific to streptavidin.

[Chemical Formula XII]

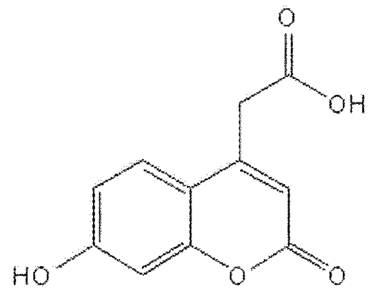

[Chemical Formula IX]

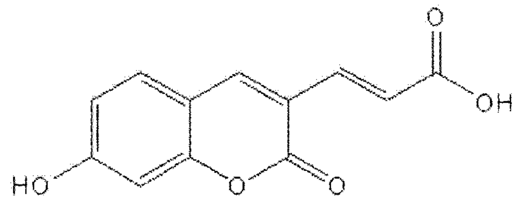

[Chemical Formula XXI]

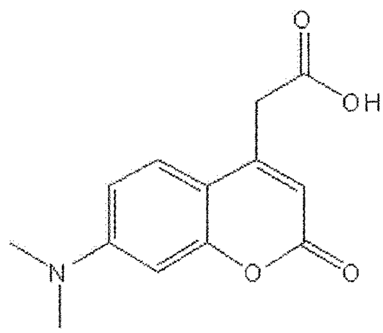

The compound represented by Chemical Formula XXIX, XXX, XXXI or XXXII, or the salt thereof utilizes 1-naphthylethylenediamine (XXXI and XXXII) of Chemical Formula I, or hydroxycumarin acetic acid (XXIX and XXX) of Chemical Formula XII as the fluorescent molecule and is prepared by linking Asp-L-Glu-L-Val-Asp-(ketone)-$CF_3$ (XXIX and XXXI) or Asp($CO_2$Me)-L-Glu($CO_2$Me)-L-Val-Asp($CO_2$Me)-(ketone)-$CF_3$ (XXX and XXXII) as the binding molecule capable of specifically binding to the target protein caspase-3 with the fluorescent molecule through a direct bond or a linker, respectively. Thus, the compound represented by Chemical Formula XXIX, XXX, XXXI or XXXII, or the salt thereof can be used as the probe for iFRET that is specific to caspase-3.

Generally, as fluorophores have absorbance and emission in longer wavelength regions, that is, they can be excited by lower energy, and a less expensive light source can be selected, thereby reducing the manufacturing cost for fabrication of measuring equipment. To prevent the spectral overlap between cellular autofluorescence and emission spectra of fluorophore, it is very important to develop a fluorophore of which emitting wavelength does not overlap with that of autofluorescence, in order to increase sensitivity of the fluorophore.

1-Naphthylethylenediamine of Chemical Formula I can be effectively detected by LED (light-emitting diode), and used as a fluorescent probe. In addition, because this compound has an excitation wavelength of 340 nm to 380 nm and an emission wavelength of 400 nm to 600 nm, it has longer emission wavelength than the known fluorophores, and therefore, it can be detected as a fluorescent molecule distinct from the cellular autofluorescence.

The compound of the present invention can be prepared in the form of a salt thereof and a solvate thereof according to a typical method known in the art.

For the salts, an acid-addition salt thereof formed by a free acid thereof is useful and can be prepared by the conventional method. For example, after dissolving the compound in an excess amount of acid solution, the salts are precipitated by the water-miscible organic solvent such as methanol, ethanol, acetone or acetonitrile to prepare acid addition salt thereof, and a further equivalent amount of compound and acid in water or alcohol (e.g., glycol monomethylether) can be heated and subsequently, the mixture is dried by evaporation or the precipitated salt is filtrated under reduced pressure. As the free acid, organic acid or inorganic acid can be used. The inorganic acid may include hydrochloric acid, phosphoric acid, sulfuric acid, nitric acid, tartaric acid or the like, and the organic acid may include methanesulfonic acid, p-toluene sulfonic acid, acetic acid, trifluoroacetic acid, citric acid, maleic acid, succinic acid, oxalic acid, benzoic acid, tartaric acid, fumaric acid, manderic acid, propionic acid, lactic acid, glycolic acid, gluconic acid, galacturonic acid, glutamic acid, glutaric acid, glucuronic acid, aspartic acid, ascorbic acid, vanillic acid, hydroiodic acid or the like, but are not limited thereto.

Further, a pharmaceutically acceptable metal salt may be prepared by using a base. The alkali metal or alkali-earth metal salt thereof can be prepared by the method, for example, after dissolving the compound in an excess amount of alkali metal hydroxide or alkali-earth metal hydroxide solution, the insoluble salts are filtered and remaining filtrate is subjected to evaporation and drying to obtain the metal salt thereof. As the metal salt, sodium, potassium or calcium salt are pharmaceutically suitable and the corresponding silver salt can be prepared by reacting alkali metal salt or alkali-earth metal salt with suitable silver salt (e.g., silver nitrate).

The salts of the compounds of Chemical Formula XXIII, XXIV, or XXVI to XXXII include all the acidic or basic salt which may be present at the compounds of Chemical Formula XXIII, XXIV, or XXVI to XXXII, if it does not indicated specifically herein. For example, the salts include the salt of hydroxyl group such as the sodium, calcium and potassium salt thereof; the salt of amino group such as the hydrochloride, hydrobromide, sulfuric acid salt, hydrogen sulfuric acid salt, phosphate salt, hydrogen phosphate salt, dihydrophosphate salt, acetate salt, succinate salt, citrate salt, tartarate salt, lactate salt, mandelate salt, methanesulfonate(mesylate) salt and p-toluenesulfonate (tosylate) salt etc, which can be prepared by the salt preparation method or process known in the art.

The sixth aspect of the present invention provides a method for detecting or imaging a target protein by iFRET, comprising the first step of adding the second probe for iFRET prepared according to the second aspect of the present invention or the probe for iFRET according to the third or the fourth aspect of the present invention to a sample containing the target protein; and the second step of irradiating light comprising a wavelength for excitation of the amino acids in the target protein and detecting or tracking the emitted light from the probe for iFRET.

Further, the seventh aspect of the present invention provides a method for detecting or imaging a target protein using a probe for intrinsic fluorescence resonance energy transfer (iFRET), wherein the probe for iFRET is prepared by linking a target protein-specific binding site or a molecule having the binding site with a fluorescent molecule acting as an acceptor for the intrinsic fluorescence of the target protein through a direct bond or a linker, and the method comprises the first step of introducing the probe for iFRET into a sample containing the target protein; the second step of alternately irradiating a first light with a wavelength for excitation of the amino acids in the target protein and a second light with a wavelength for excitation of the fluorescent molecule of the probe for iFRET onto the sample prepared in the first step; and the third step of calculating a third emission signal by ratiometric measurement of the first and second emission signals, by analyzing a first emission signal that is generated from the probe for iFRET by irradiating the first light with a wavelength for excitation of the amino acids in the target protein to the sample and a second emission signal that is generated from the probe for iFRET by irradiating the second light with a wavelength for excitation of the fluorescent molecule of the probe for iFRET.

As used herein, the term "specimen" or "sample" means a composition to be analyzed, which contains or is suspected of containing the target protein, and it may be collected from any one or more of a target protein itself, cell, blood, feces, water, soil, air, food, waste product, animal/plant intestinal material, animal/plant tissue, and an organism itself, but is not limited thereto. The specimen or sample may be water, buffer, lower alcohol having 1 to 6 carbon atoms, or mixtures thereof, a biochip or (nano)microparticles containing the target protein.

The seventh aspect of the present invention is characterized in that a first light with a wavelength for excitation of the amino acids in the target protein and a second light with a wavelength for excitation of the fluorescent molecule of the probe for iFRET are alternately irradiated onto the sample containing the target protein and the probe for iFRET bound thereto.

The second light with a wavelength for excitation of the fluorescent molecule of the probe for iFRET does not excite the amino acids in the target protein but directly excites the fluorescent molecule of the probe for iFRET.

In the seventh aspect of the present invention, a third emission signal can be calculated by ratiometric measurement of the first and second emission signals, by analyzing a first emission signal that is generated from the probe for iFRET by irradiating the first light with a wavelength for excitation of the amino acids in the target protein to the sample and a second emission signal that is generated from the probe for iFRET by irradiating the second light with a wavelength for excitation of the fluorescent molecule of the probe for iFRET.

The third emission signal is a ratio of the amount of the probe for iFRET (first emission signal) that binds to the target protein to the amount of the probe for iFRET (second emission signal) that is present in the test sample (e.g., cell, test liquid, etc.). A reference value (second emission signal) is provided regardless of temporal and spatial changes in the measurement, makes it possible to quantify the binding of the target protein and the probe for iFRET. By using the ratiometric measurement, correction is also possible even though the amount of light illuminated to the sample is changed.

The third emission signal can be calculated by the following Equation.

[Equation 3]

Third emission signal = First emission signal ÷ Second emission signal

According to the ratiometric measurement, intensity of the first emission signal is converted into the third emission signal by using the second emission signal as reference to minimize measurement errors due to localization of the probe for iFRET. Therefore, clear images can be acquired, and it is possible to quantify the amount of the target protein and binding of a drug to the target protein and to track spatial migration of the target protein.

The third emission signal can be calculated using the conventional software for ratiometric measurement.

Meanwhile, the probe for iFRET is prepared by linking the target protein-specific binding molecule with the fluorescent molecule acting as an iFRET acceptor through a direct bond or a linker. Therefore, the binding molecule region induces the specific binding of the probe for iFRET to the target protein, which makes it possible to quantify the target protein using iFRET. In addition, the probe for iFRET moves together with the target protein, and thus behavior of the target protein can be observed. In the method for detecting or imaging the target protein according to the seventh aspect of the present invention, the first emission signal, the second emission signal, and the third emission signal may be two-dimensional images corresponding to each two-dimensional point of the sample.

In the seventh aspect of the present invention, the wavelength of the first light is preferably in the range of 260 nm to 300 nm, and the wavelength of the second light is preferably in the range of 300 nm to 400 nm.

Also, in the seventh aspect of the present invention, when the first light with a wavelength for excitation of the amino acids in the target protein and the second light with a wavelength for excitation of the fluorescent molecule of the probe for iFRET are alternately irradiated at a predetermined time interval, the third emission signals (C1, C2, C3,...) are calculated and accumulated by ratiometric measurement at a predetermined time interval from the first emission signals (A1, A2, A3, ...) that are generated from the probe for iFRET by irradiating the first light with a wavelength for excitation of the amino acids in the target protein to the sample and the second emission signals (B1, B2, B3, ...) that are generated from the probe for iFRET by irradiating the second light with a wavelength for excitation of the fluorescent molecule of the probe for iFRET, thereby monitoring changes in the localization or quantity of the target protein or both of them over time.

Accordingly, intracellular localization and spatial migration of the target protein, changes in the quantity and activity thereof, and binding degree of a drug over time, or all of the above are changes that can be monitored. Changes of the target protein over time can be given as moving images.

The quantity or activity of the target protein can be measured or the action mechanism or migration of the target protein can be tracked by the method for detecting or imaging the target protein according to the sixth or seventh aspect of the present invention.

The method for detecting or imaging the target protein according to the sixth or seventh aspect of the present invention can be used as a method for examining the functions of the target protein, a method for detecting disease-associated target proteins in various specimens for disease diagnosis, or a method for detecting microorganisms or target proteins derived from microorganisms in food, feed, or drink.

Meanwhile, when the emitting light of the probe for iFRET is detected or tracked, it is preferable that the light with the longer wavelength at 450 nm or above among the emitting lights of the probe for iFRET is measured. In cells or tissues, cellular autofluorescent materials exhibiting fluorescence by lights with wavelength at 260 to 400 nm, such as nucleic acids, NAD(P)H, collagen or cellular proteins exist, and fluorescence emissions thereof are monitored between 300 to 450 nm. Accordingly, when the probe for iFRET of the present invention is used in cell imaging or in staining of cell extract or tissue, the emission wavelength of the probe for iFRET of the present invention is preferably 450 nm or longer in order to avoid their interference.

The method for detecting or imaging the target protein according to the sixth or seventh aspect of the present invention further comprises the step of adding a particular material in combination with the probe for iFRET to the sample, irradiating light with a wavelength for excitation of the amino acids in the target protein, and detecting or tracking the emitted light from the probe for iFRET, and according to the presence of the particular material, screening the particular material having a predetermined function for the target protein or examining the function of the particular material for the target protein by irradiating light with a wavelength for excitation of the amino acids in the target protein and then examining changes in the emitted light from the probe for iFRET.

In this regard, the particular material identified by the method can be used as a drug or food additive for preventing, treating or ameliorating the target protein-associated diseases.

The method for detecting or imaging the target protein according to the sixth or seventh aspect of the present invention may be performed by a typical fluorescence imaging method such as imaging using a fluorescence microscope and rapid immunofluorescence chromatography, but it may be preferably performed by an automated workstation.

Hereinafter, the present invention will be described in more detail with reference to Examples. However, these Examples are for illustrative purposes only, and the invention is not intended to be limited by these Examples.

**<Preparation of fluorescent molecule for iFRET probe>**

**Example 1: Preparation method of Chemical Formula II**

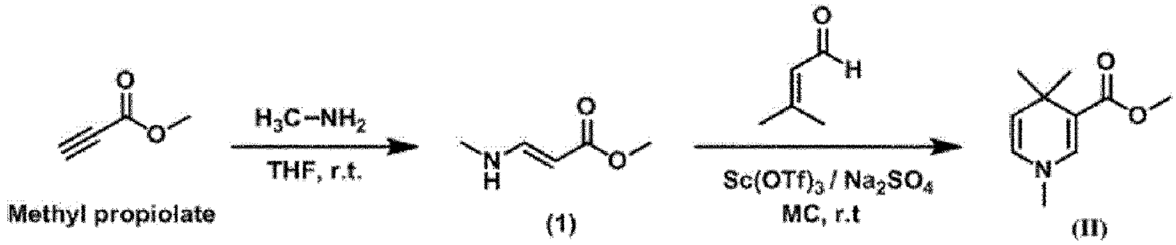


**Synthesis of methyl-3-(methylamino)acrylate (1)**

2 M methylamine-tetrahydrofuran solution (2 M methylamine in THF) (12.8 ml) was added dropwise to methyl propiolate (2.89 ml) and THF (15 ml) solution at 0°C, and stirred at room temperature for 8 hours. The volatile solvent and methyl propiolate were removed under reduced pressure and dried under vacuum to prepare 2.2 g of Compound 1 (yield: 81%). The structure of the product was identified by $^1$H-NMR.
$^1$H-NMR (600 MHz, $CDCl_3$): 7.648-7.574(b.s,1H), 6.621-6.568(q,1H), 4.483-4.468(d,1H), 3.654(s,3H), 2.971-2.957(d,2H).

**Synthesis of methyl 1,4,4-trimethyl-1,4-dihydropyridine-3-carboxylate (II)**

Compound 1 (2.1 g), 3-methyl-2-butenal (1.04 ml), anhydrous sodium sulfate (3 g), and scandium trifluoromethanesulfonate (scandium triflate, 267 mg) were suspended in dichloromethane (80 ml), and then stirred at room temperature for 24 hours. The solution was concentrated under reduced pressure, and the residue was purified by silica gel chromatography (ethyl acetate:hexane=3:7) to prepare 470 mg of Compound II (yield: 14%). The structure of the product was identified by $^1$H-NMR.
$^1$H-NMR (600 MHz, $CDCl_3$): 7.031-7.027(d,1H), 5.592-5.568(q,1H), 4.481-4.462 (d,1H), 3.657(s,3H), 2.963(s,3H), 1.341(s,6H).

**Example 2: Preparation method of Chemical Formulae III and IV**

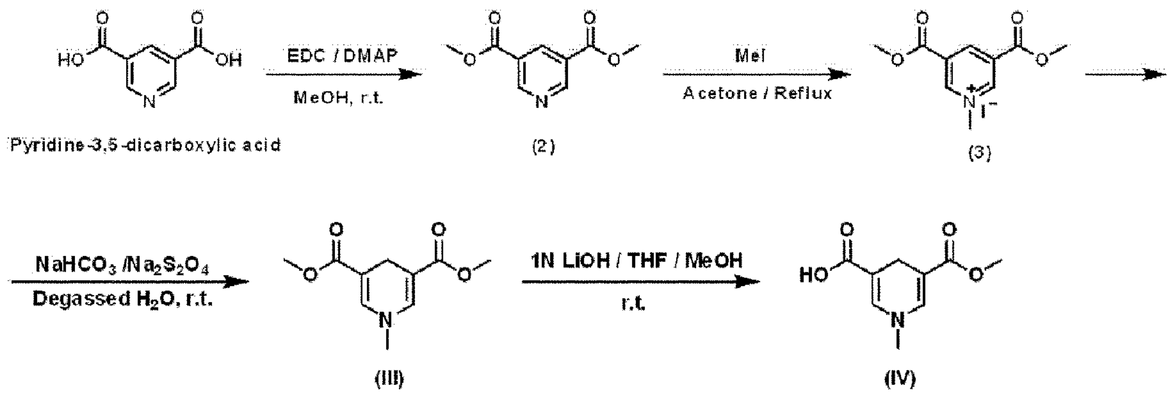

**Synthesis of dimethyl pyridine-3,5-dicarboxylate (2)**

pyridine-3,5-dicarboxylic acid (7.94 g), 1-ethyl-3-(3-dimethylaminopropyl)carbodiimide (EDC, 20 g) and 2.2 g of (4-dimethylamino)pyridine (DMAP) were added to methanol (150 ml), and then stirred at room temperature for 10 hours. The solution was concentrated under reduced pressure, and the residue was diluted with dichloromethane, and then washed with 10% citric acid aqueous solution. The organic layer was further washed with a saturated sodium chloride solution, and dried over anhydrous magnesium sulfate (anhydrous $MgSO_4$). The solution was concentrated under reduced pressure and the residue was dried under vacuum to prepare 8.2 g of Compound 2 (yield: 88%). The structure of the product was identified by $^1$H-NMR.
$^1$H-NMR (400 MHz, $CDCl_3$): 9.418(s,2H), 8.920(s,1H), 4.046(s,3H).

**Synthesis of 3,5-bis(methoxycarbonyl)-1-methylpyridinium (3)**

Methyl iodide (7.77 ml) was added to an acetone solution (40 ml) of Compound 2 (8.1 g), and refluxed with stirring for 20 hours. The reactant was cooled to room temperature and the solvent was removed under reduced pressure. The desired crystalline product thus formed was washed with acetone three times, and dried under vacuum to prepare 13.2 g of Compound 3 (yield: 95%). The structure of the product was identified by $^1$H-NMR.
$^1$H-NMR (400 MHz, DMSO): 9.773(s,2H), 9.119(s,1H), 4.500(s,2H), 4.020(s,6H).

**Synthesis of dimethyl 1-methyl-1,4-dihydropyridine-3,5-dicarboxylate (III)**

An aqueous solution (50 ml) of Compound 3 (13.2 g) was added dropwise to an aqueous solution (150 ml) of sodium bicarbonate (16.5 g) and sodium hydrosulfite (20.5 g) at 0°C under argon atmosphere. The reaction solution was stirred at room temperature for 3 hours, and then the product was extracted with dichloromethane. The extract was concentrated under reduced pressure, and then dried under vacuum to prepare 6.92 g of Compound III (yield: 84%). The structure of the product was identified by $^1$H-NMR.
$^1$H-NMR (600 MHz, $CDCl_3$): 6.918(s,1H), 3.722(s,6H), 3.191(s,2H), 3.108(s,3H).

**Synthesis of 5-(methoxycarbonyl)-1-methyl-1,4-dihydropyridine-3-carboxylic acid (IV)**

Compound III (6 g) was dissolved in 1 N lithium hydroxide solution (40 ml), THF solution (250 ml), and methanol (150 ml) and then stirred at room temperature for 24 hours. The reactant was acidified with a 1 N hydrochloric acid solution, and extracted with ethyl acetate three times. The organic layer was washed with an aqueous solution three times, and further washed with the saturated sodium chloride solution once, and then dried over anhydrous magnesium sulfate. The solution was concentrated under reduced pressure and the residue was purified by silica gel chromatography (chloroform:methanol=10:1) to prepare 3.5 g of Compound IV (yield: 62.5%). The structure of the product was identified by $^1$H-NMR.
H-NMR (600 MHz, $CD_3OD$): 7.015(s,2H), 3.672(s,3H), 3.105(s,2H), 3.067(s,3H).

__**Example 3: Preparation method of Chemical Formula V**__

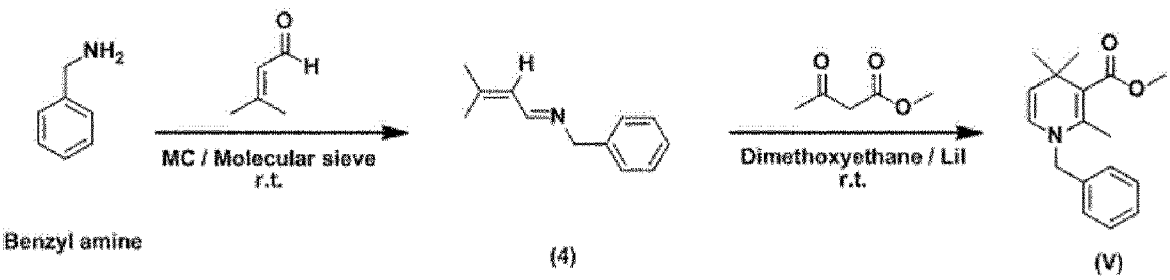


Benzyl amine (4) (V)

**Synthesis of N-(3-methyl-2butenylidene)-1-phenylmethanamine (4)**

Benzylamine (5.3 ml), 3-methyl-2-butenal (4.54 ml), and a molecular sieve (30 g) were added to dichloromethane (60 ml), and then stirred at room temperature for 12 hours. After removing the molecular sieve, the solution was concentrated under reduced pressure, and the residue was purified by silica gel chromatography (ethyl acetate:hexane=1:1)

to prepare 3.4 g of Compound 4 (yield: 38%). The structure of the product was identified by $^1$H-NMR.
$^1$H-NMR (300 MHz, $CDCl_3$): 8.302-8.261(d,1H), 7.336-7.192(m,5H), 6.089-6.040(d,1H), 4.627(s,2H), 1.918(s,3H), 1.864(s,3H).

**Synthesis of methyl 1-benzyl-2,4,4-trimethyl-1,4-dihydropyridine-3-carboxylate (V)**

Compound 4 (1.73 g), methyl-3-oxobutanoate (1.08 ml), lithium iodide (134 mg) were dissolved in 1,2-dimethoxyethane (15 ml), and then stirred at room temperature for 24 hours. The solution was concentrated under reduced pressure, and the residue was purified by silica gel chromatography (diethylether:hexane=1:1) to prepare 200 mg of Compound V (yield: 7.4%). The structure of the product was identified by $^1$H-NMR.
$^1$H-NMR (600 MHz, $CDCl_3$): 7.355-7.330(m,2H), 7.268-7.259(m,1H), 7.213-7.200(d,2H), 5.781-5.768(d,1H), 4.478(s,3H), 4.195-4.159(q,2H), 1.957(s,3H), 1.311-1.274(t,9H).

**Example 4: Preparation method of Chemical Formulae VI and VII**

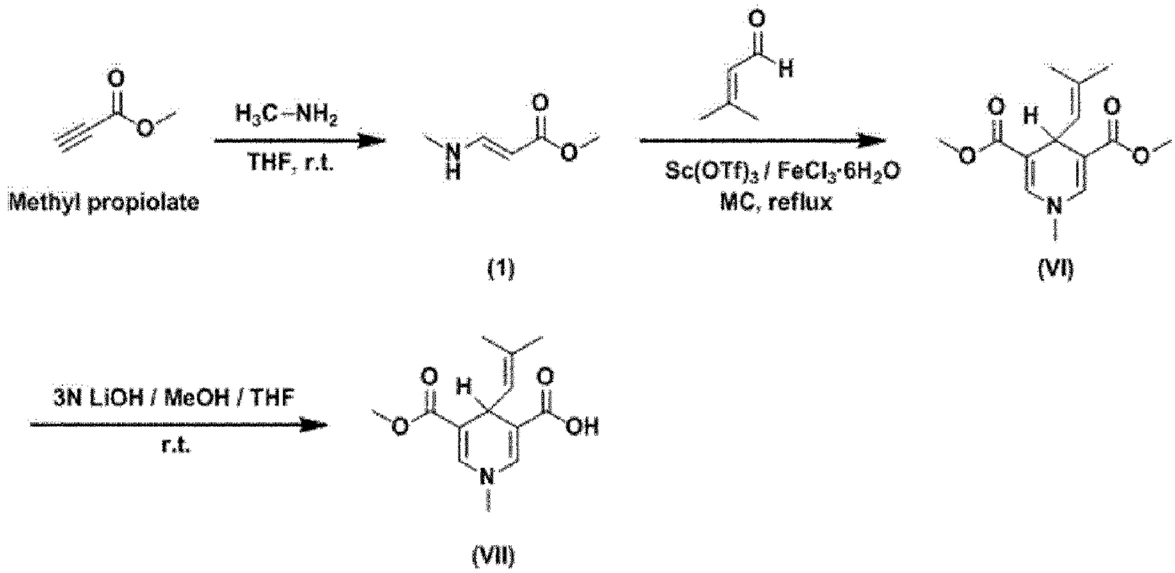


**Synthesis of dimethyl 1-methyl-4-(2-methyl-1-propenyl)-1,4-dihydropyridine-3,5-dicarboxylate (VI)**

Compound 1 (2 g), 3-methyl-2-butenal (2.5 ml), anhydrous sodium sulfate (25 g), and Iron(III) chloride hexahydrate (235 mg) were suspended in dichloromethane (120 ml) and then refluxed with stirring for 44 hours. The solution was concentrated under reduced pressure, and the residue was purified by silica gel chromatography (dichloromethane:hexane=1:1) to prepare 26 mg of Compound VI (yield: 0.6%). The structure of the product was identified by $^1$H-NMR.
$^1$H-NMR (600 MHz, $CDCl_3$): 7.272(s,2H), 4.891-4.870(d,1H), 4.489-4.472(d,1H), 3.711(s,6H), 3.168(s,3H), 1.827(s,3H), 1.628(s,3H).

**Synthesis of 5-(methoxycarbonyl)-1-methyl-4-(2-methyl-1-propenyl)-1,4-dihydropyridine-3-carboxylic acid (VII)**

Compound VI (19 mg) was dissolved in 3 N lithium hydroxide aqueous solution (0.12 ml), THF (0.1 ml) solution, and methanol (0.1 ml), and then stirred at room temperature for 48 hours. The reactant was acidified with 1 N hydrochloric acid solution, and then extracted with ethyl acetate three times. The organic layer was further washed with the aqueous solution three times and with the saturated sodium chloride solution once, and then dried over anhydrous magnesium sulfate. The solution was concentrated under reduced pressure and purified by silica gel chromatography (ethyl acetate:hexane=3:7) to prepare 12 mg of Compound VII (yield: 67%). The structure of the product was identified by $^1$H-NMR.
$^1$H-NMR (600 MHz, $CDCl_3$): 7.121(s,1H), 7.025(s,1H), 4.898-4.880(q,1H), 4.469-4.453(d,1H), 3.673(s,3H), 3.183(s,3H), 1.811(s,3H), 1.629(s,3H).

**Example 5: Preparation method of Chemical Formula VIII**

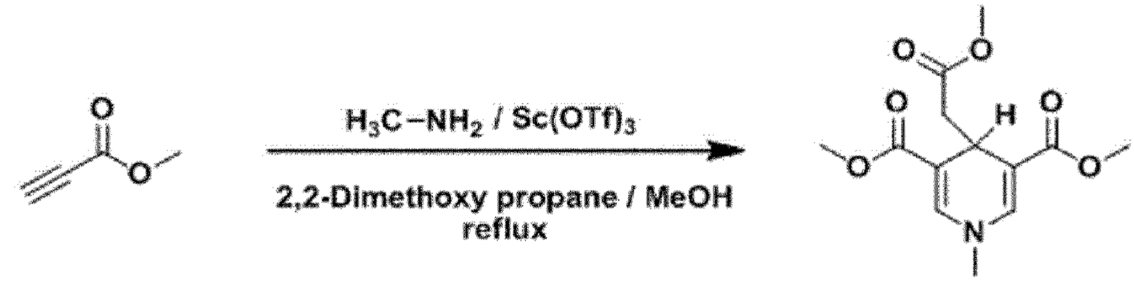


Methyl propiolate

(VIII)

**Synthesis of dimethyl 4-(2-methoxy-2-oxoethyl)-1-methyl-1,4-dihydropyridine-3,5-dicarboxylate (VIII)**

2 M methylamine-tetrahydrofuran solution (0.62 ml) was added dropwise to 2,2-dimethoxypropane (3.9 ml), methanol (1.3 ml), methyl propiolate (0.8 ml) and scandium trifluoromethanesulfonate (78 mg), and refluxed with stirring for 18 hours. The solvent was removed under reduced pressure and the residue was purified by silica gel chromatography (ethyl acetate:hexane=2:8) to prepare 24 mg of Compound VIII (yield: 28%). The structure of the product was identified by $^1$H-NMR.

$^1$H-NMR (300 MHz, $CDCl_3$): 7.120(s,2H), 4.200-4.165(t,1H), 3.793(s,6H), 3.599(s,3H), 3.180(s,3H), 2.467-2.449(d,2H).

**Example 6: Preparation method of Chemical Formulae IX to XI**

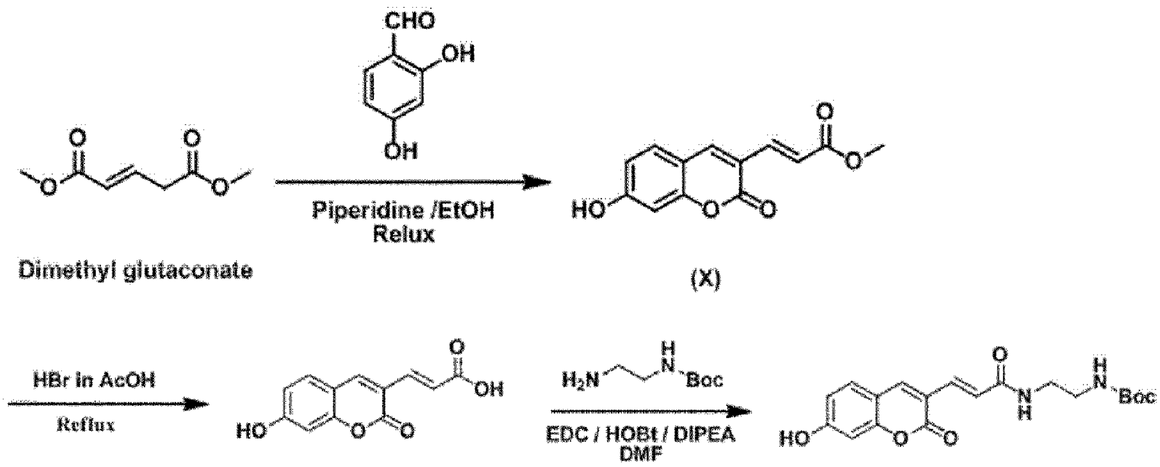


Dimethyl glutaconate

(X)

(IX) (5) (XI)

**Synthesis of methyl 3-(7-hydroxy-2-oxo-2H-chromen-3-yl)acrylate (X)**

Dimethyl glutaconate (5.63 ml), 2,4-dihydroxybenzaldehyde (5 g), and piperidine (0.36 ml) were dissolved in ethanol (100 ml), and then refluxed with stirring for 16 hours. The reactant was cooled to room temperature and the solvent was removed under reduced pressure, and then the desired crystalline product thus formed was washed with ethanol three times, and dried under vacuum to prepare 8.01 g of Compound X (yield: 90%). The structure of the product was identified by $^1$H-NMR.

$^1$H-NMR (600 MHz, DMSO): 8.445(s,1H), 7.833-7.806(d,1H), 7.614-7.600(d,1H), 7.124-7.097(d,1H), 6.893-6.881(d,1H), 6.774(s,1H), 3.77(s,3H).

**Synthesis of 3-(7-hydroxy-2-oxo-2H-chromen-3-yl)acrylic acid (IX)**

Compound X (16 g) was dissolved in 33% hydrogen bromide in acetic acid (150 ml) and then refluxed with stirring for 18 hours. The reactant was cooled to room temperature and the solvent was removed under reduced pressure, and then the crystalline product thus formed was washed with the aqueous solution three times, and dried under vacuum to prepare 13.5 g of Compound IX (yield: 90%). The structure of the product was identified by $^1$H-NMR.
$^1$H-NMR (600 MHz, DMSO): 8.413(s,1H), 7.587-7.566(d,1H), 7.486-7.446(d,1H), 6.862-6.860(dd,1H), 6.824-6.784(d,1H), 6.757(s,1H).

**Synthesis of tert-butyl 2-aminoethylcarbamate (5)**

Di-tert-butyl-dicarbonate (2.2 g) in dichloromethane (20 ml) was added dropwise to ethylenediamine (4 ml) in dichloromethane (140 ml) for 6 hours, and then stirred at room temperature for 18 hours. The solution was concentrated under reduced pressure, and the residue was diluted with dichloromethane, and then washed with 2 M sodium carbonate solution. The organic layer was further washed with the saturated sodium chloride solution and dried over anhydrous magnesium sulfate. The solution was concentrated under reduced pressure, and the residue was dried under vacuum to prepare 1.4 g of Compound 5 (yield: 88%). The structure of the product was identified by $^1$H-NMR.
$^1$H-NMR (600 MHz, $CDCl_3$): 5.01(br s, 1H), 3.20-3.12(m,2H), 2.78(t,2H), 1.43(s,9H), 1.31(s,2H).

**Synthesis of tert-butyl 2-(3-(7-hydroxy-2-oxo-2H-chromen-3-yl)acrylamido)ethylcarbamate (XI)**

Compound 5 (41 mg), Product IX (50 mg), and N,N-diisopropylethylamine (0.19 ml) were dissolved in N,N-dimethylformamide (DMF, 1 ml) at 0°C, and 15 minutes later, EDC (50 mg) and hydroxybenzotriazole (HOBt, 40 mg) were added thereto and stirred at room temperature for 20 hours. After concentration under reduced pressure, the residue was diluted with ethyl acetate, and then washed with the aqueous solution and 10% citric acid aqueous solution. The organic layer was further washed with the saturated sodium chloride solution, and then dried over anhydrous magnesium sulfate. The solution was concentrated under reduced pressure and the residue was dried under vacuum to prepare 75 mg of Compound XI (yield: 91%). The structure of the product was identified by $^1$H-NMR.
$^1$H-NMR (600 MHz, DMSO): 8.252(s,2H), 7.584-7.563(d,1H), 7.305-7.267(d,1H), 6.847-6.842(d,1H), 6.826-6.820(q,2H,), 6.746(d,1H), 3.208-3.161(q,2H), 3.040-2.994(q,2H), 1.379(s,9H).

<u>Example 7: Preparation of Chemical Formulae XX to XXII</u>

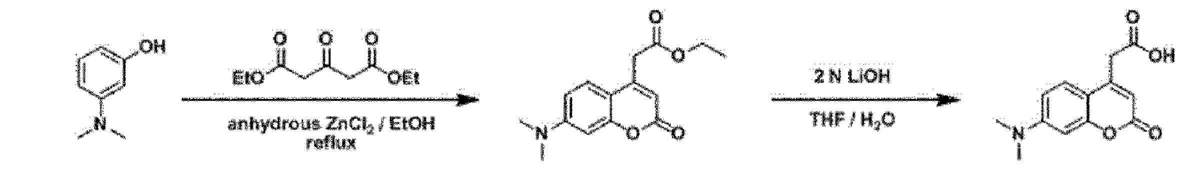


3-(Dimethylamino)phenol (XX) (XXI)

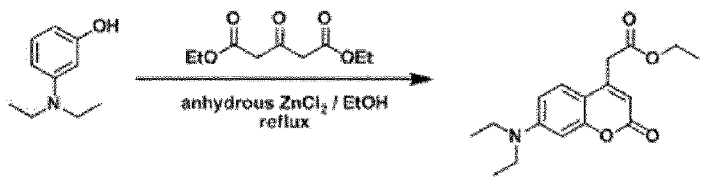


3-(Diethylamino)phenol (XXII)

Synthesis of ethyl 2-(7-(dimethylamino)-2-oxo-2H-chromen-4-yl)acetate (XX)

3-(Dimethylamino)phenol (6.86 g), diethyl-1,3-acetonedicarboxylate (10 ml), and anhydrous zinc chloride (anhydrous $ZnCl_2$, 8.2 g) were dissolved in ethanol (30 ml) and then refluxed with stirring for 12 hours. The crystalline product formed from the reactant in 4°C aqueous solution was recrystallized with chloroform and diethylether, and then dried under vacuum to prepare 3.4 g of Compound XX (yield: 25%). The structure of the product was identified by $^1$H-NMR.

$^1$H-NMR (400 MHz, $CDCl_3$): 7.415-7.393 (d, 1H), 6.633-6.604(dd, 1H), 6.526-6.517(d, 1H), 6.059(s, 1H), 4.207-4.153(q,2H), 3.677(s,2H), 3.060(s,6H), 1.268-1.232(t,3H).

**Synthesis of 2-(7-(dimethylamino)-2-oxo-2H-chromen-4-yl)acetic acid (XXI)**

Compound XX (1 g) was dissolved in 2 N lithium hydroxide aqueous solution (3.6 ml), THF solution (4.5 ml), and methanol (1.5 ml) at 0°C, and stirred at room temperature for 1 hour. 6 ml of the aqueous solution was added thereto, and washed with diethylether three times. The aqueous solution layer was acidified with 2 N hydrochloric acid solution, and the crystalline product thus formed was dried under vacuum to prepare 760 mg of Compound XXI (yield: 85%). The structure of the product was identified by $^1$H-NMR.
$^1$H-NMR (400 MHz, DMSO) : 12.798 (bs, 1H), 7.49-4.456 (d, 1H), 6.755-6.749 (d, 1H), 6.571 (s, 1H), 6.049 (s, 1H), 3.786(s,2H), 3.022(s,6H).

**Synthesis of ethyl 2-(7-(diethylamino)-2-oxo-2H-chromen-4-yl)acetate (XXII)**

3-(Diethylamino)phenol (100 mg), diethyl-1,3-acetonedicarboxylate (0.12 ml), and anhydrous zinc chloride (anhydrous $ZnCl_2$, 99 mg) were dissolved in ethanol (5 ml), and then refluxed with stirring for 12 hours. The crystalline product formed from the reactant in 4°C aqueous solution was recrystallized with chloroform and diethylether, and then dried under vacuum to prepare 17 mg of Compound XXII (yield: 9.3%). The structure of the product was identified by $^1$H-NMR.
$^1$H-NMR (400 MHz, $CDCl_3$): 7.477-7.454 (d, 1H), 6.806-6.778 (dd, 1H), 6.670 (s, 1H), 6.046 (s, 1H), 4.198-4.180(q,2H), 3.486-3.407(m,4H), 2.379(s,2H), 1.281-1.260(t,3H), 1.226-1.190(t,6H).

**<Fluorescence characterization of fluorescent molecules>**

The excitation and emission wavelengths of the fluorescent molecules of the present invention, that is, the compounds represented by Chemical Formulae I to XXII are described in Table 1, and graphs showing the fluorescent characteristics thereof are given in FIGs. 3 to 15.

**<u>Example 8: Preparation method of Chemical Formula XXIII</u>**

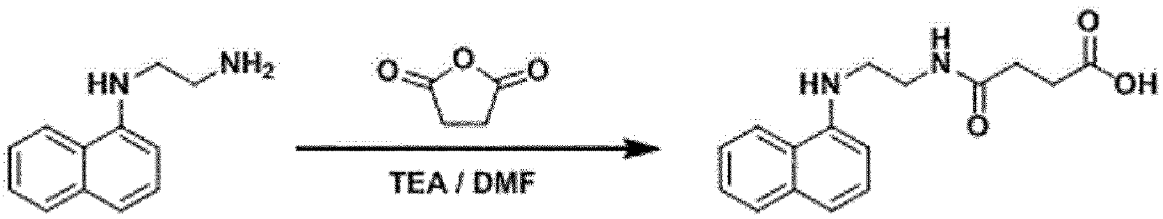


(A)

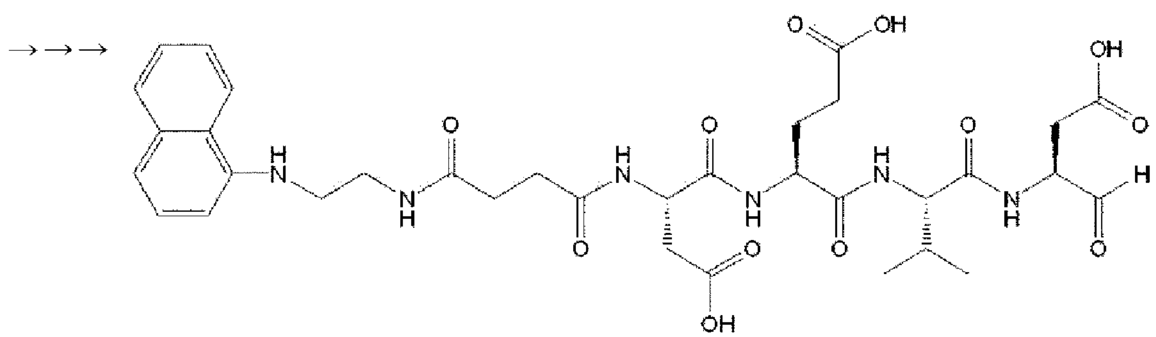

A linker was linked to the compound of Chemical Formula I (Compound I) as a fluorescent molecule to synthesize a fluorescent substance (A).

Through the following process, DEVD-CHO which is a peptide inhibitor inhibiting caspase activity was linked to the fluorescent substance (A) to prepare the compound of Chemical Formula XXIII.

4 mM N-(1-naphthyl)ethylenediamine dihydrochloride and 4.8 mM succinic anhydride were reacted on DMF, and at this time, approximately 12 mM triethylamine was used as a base. The termination of the reaction was confirmed by TLC, and the final product was recrystallized from methanol. The isolated substance was subjected to mass spectrometric analysis (FIG. 22).

LC/MS of Chemical Formula XXIII: 728.5 (M)

**<Detection of caspase-3 protein and Test of** its **activity using iFRET probe of Chemical Formula XXIII>**

Chemical Formula XXIII and caspase protein were used to detect iFRET signals. When caspase was present alone, fluorescence was observed at approximately 350 nm by excitation at 280 nm. Fluorescence of Chemical Formula XXIII was observed at approximately 445 nm by excitation at 280 nm.

As shown in the left graph of FIG. 16, the mixture of Chemical Formula XXIII (indicated by DEVD-Dye) and active caspase showed reduced fluorescence at 350 nm, and approximately 300% increased fluorescence at 445 nm, unlike caspase or Chemical Formula XXIII alone.

In contrast, when inactive caspase and Chemical Formula XXIII were excited at 280 nm, no iFRET signals were observed, as shown in the right graph, suggesting that the iFRET phenomenon results from selective binding of caspase and Chemical Formula XXIV.

<u>**Example 9: Preparation method of Chemical Formula XXIV**</u>

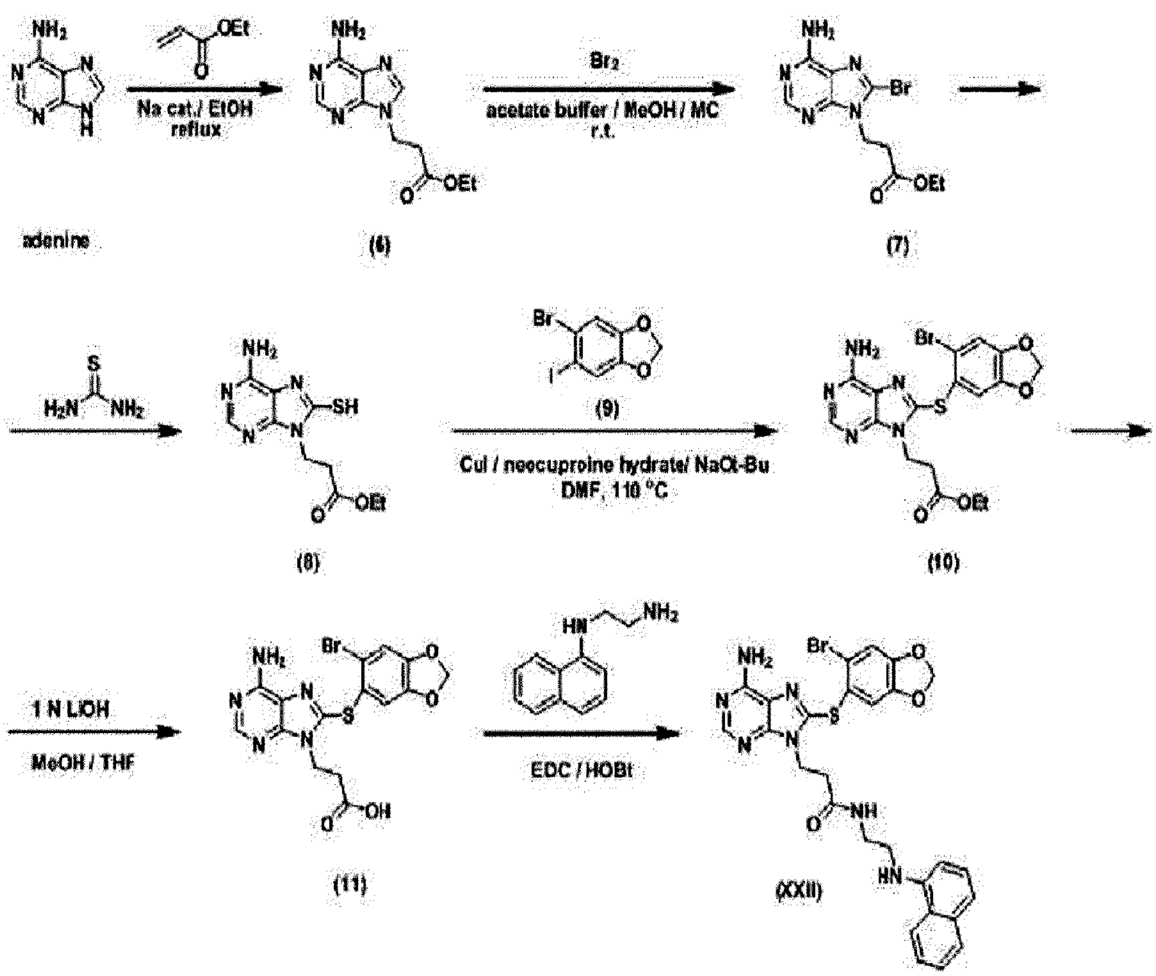


adenine (6) (7) (8) (9) (10) (11) (XXII)

**Synthesis of ethyl 3-(6-amino-9H-purin-9-yl)propanoate (6)**

Adenine (7 g), sodium (52 mg), and ethyl acrylate (16.8 ml) were added to ethanol (140 mL), and then refluxed with stirring for 4 hours. The solution was concentrated under reduced pressure, and the residue was purified by silica gel chromatography (dichloromethane:methanol = 50:1→30:1→10:1). The solution was recrystallized from ethanol to prepare 8.79 g of Compound 6 (yield: 73%). The structure of the product was identified by $^1$H-NMR.
$^1$H-NMR (600MHz, $CDCl_3$) (I) : 8.36 (s, 1H, H-2), 7.90 (s, 1H, H-8), 5.60 (b.s., 2H, $NH_2$), 4.51-4.49 t, 2H, $CH_2N$), 4.15-4.11 (q, 2H, $CH_2$) , 2.94-2.92 (t, 2H, $CH_2CO$), 1.23-1.21 (t, 3H, $CH_3$) .

**Synthesis of 5-bromo-6-iodo-benzo[1, 3]dioxole (9)**

5-Bromo-benzo [1,3] dioxole (1.5 g), trifluoroacetic acid (1.15 ml), and N-iodosuccinimide (5.06 g) were added to acetonitrile (23 ml), and then stirred at room temperature for 26 hours (under dark conditions). The solution was concentrated under reduced pressure, and then the residue was purified by silica gel chromatography (hexane) to prepare 2.126 g of Compound 9 (yield: 88%). The structure of the product was identified by $^1$H-NMR.
$^1$H-NMR (400 MHz, DMSO) (IA) : 7.478 (s, 1H), 7.363 (s, 1H), 6.092(s,2H)
$^1$H-NMR (400 MHz, $CDCl_3$) (IA): 7.26 (s, 1H), 7.10 (s, 1H), 5.99 (s, 2H)

**Synthesis of ethyl 3-(6-amino-8-bromo-9H-purin-9-yl)propanoate (7)**

Compound 6 (9.242 g) was added to acetate buffer (54 ml), methanol(6 ml), and dichloromethane (15 ml) and then bromine (2.86 ml) was added dropwise for 10 minutes. After solidification of the reactant with methanol (10 ml) was observed, an excessive amount of methanol was added, and stirred for 1 hour. The solution was concentrated under reduced pressure, and the residue was extracted with a mixed solution of dichloromethane and a small amount of methanol. The organic layer was washed with a saturated sodium thiosulfate aqueous solution, and then the desired product of the aqueous layer was extracted with dichloromethane. The organic layer was concentrated under reduced pressure, and then the residue was purified by silica gel chromatography (methanol:dichloromethane=100:1→50:1→40:1). After concentration under reduced pressure, the crystalline product was washed with ethyl acetate and hexane, and then dried under vacuum to prepare 9.517 g of Compound 7 (yield: 77%). The structure of the product was identified by $^1$H-NMR.
$^1$H-NMR (600 MHz, DMSO) (II) : 8.19 (s, 1H, H-2), 7.38 (b.s., 2H, $NH_2$) , 4.38-4.36 (t, 2H, $CH_2N$) , 4.03-3.99 (q, 2H, $CH_2$), 2.90-2.88 (t, 2H, $CH_2CO$), 1.12-1.09 (t, 3H, $CH_3$) .

**Synthesis of ethyl 3-(6-amino-8-macapto-9H-purin-9-yl)propanoate (8)**

Compound 7 (1 g) and thiourea (1.21 g) were added to butanol (n-butanol, 24 ml), and then refluxed with stirring for 5 hours. The reactant was cooled to room temperature and the solvent was removed under reduced pressure. Then, the desired crystalline product thus formed was recrystallized from methanol, and dried under vacuum to prepare 0.661 g of Compound 8 (yield: 75%). The structure of the product was identified by $^1$H-NMR.
$^1$H-NMR (600 MHz, DMSO) (III) : 12.35 (s, 1H, SH), 8.15 (s, 1H, H-2), 6.85 (b.s., 2H, $NH_2$), 4.37-4.34(t, 2H, $CH_2N$), 4.03-3.99 (q, 2H, $CH_2$), 2.81-2.79 (t, 2H, $CH_2CO$), 1.13-1.10 (t, 3H, $CH_3$) .

**Synthesis of ethyl 3-(6-amino-8-(6-bromobenzo[1,3]dioxol-5-ylthio)-9H-purin-9-yl)propanoate (10)**

Compound 8 (1.467 g), Compound 9 (2.81 g), copper iodide (0.1067 g), neocuproine hydrate (0.1156 g), and sodium butoxide (0.646 g) were dissolved in DMF (20 ml), and then stirred at 110°C for 25 hours. The solution was concentrated under reduced pressure and the residue was purified by silica gel chromatography (dichloromethane:methanol=200:1→100:1→50:1). After concentration under reduced pressure, the desired crystalline product thus formed was washed with a mixed solution of dichloromethane and hexane, and dried under vacuum to prepare 1.386 g of Compound 10 (yield: 54%).
The structure of the product was identified by $^1$H-NMR.
$^1$H-NMR (500 MHz, DMSO) (IV) : 8.15 (s, 1H, H-2) , 7.399 (b.s., 2H, $NH_2$) , 7.35 (s, 1H, CHBr), 6.82 (s, 1H, CHS), 6.08 (s, 2H, $OCH_2O$), 4.44-4.41 (t, 2H, $CH_2N$) , 4.02-3.97 (q, 4H, $CH_2O$), 2.86-2.82 (t, 2H, $CH_2CO$), 1.12-1.09 (t, 3H, $CH_3$) .
The structure of the product was identified by $^{13}$C-NMR.
$^{13}$C-NMR (400 MHz, DMSO)(IV): 14.459, 33.88, 40.025, 60.98, 103.29, 111.94, 113.68, 115.57, 120.24, 125.61, 143.86, 148.62, 149.09, 151.45, 153.71, 155.92, 170.85.
LC/MS: 466.3 (M+H, 98%)

**Synthesis of 3-(6-amino-8-(6-bromobenzo[1,3]dioxol-5-ylthio)-9H-purine-9yl)propionic acid (11)**

Compound 10 (540 mg) was dissolved in THF (13 ml), 1 N lithium hydroxide aqueous solution (32 ml), and methanol (32 ml) and then stirred at room temperature for 16 hours. The solution was concentrated under reduced pressure, and the residue was diluted with a small amount of aqueous solution, and then acidified with 1 N hydrochloric acid solution. The desired crystalline product thus formed was washed with the aqueous solution, and the dried under vacuum to prepare 397.4 mg of Compound 11 (yield: 76%). The structure of the product was identified by $^{1}$H-NMR.
$^{1}$H-NMR (600 MHz, DMSO) (V) : 12.50 (b.s, COOH) , 8.15 (s, 1H, H-2), 7.42 (bs, 2H,$NH_2$) , 7.36 (s, 1H, CHBr), 6.84 (s, 1H, CHS) , 6.09 (s, 2H, $OCH_2O$), 4.40-4.38 (t, 2H, $CH_2N$), 2.80-2.77 (t, 2H, $CH_2CO$) .
$^{13}$C-NMR (400 MHz, DMSO)(V): 34.605, 40.48, 103.272, 112.157, 113.666, 115.812, 120.274, 125.628, 144.146, 148.600, 149.120, 151.384, 153.61, 155.859, 172.641.
LC/MS: 438.2 (M+H, 98%)

**Synthesis of 3-(6-amino-8-(6-bromobenzo[1,3]dioxole-5-ylthio)-9H-purin-9-yl)-N-(2-(naphthalene-1-ylamino)ethyl)propaneamide (XXIV)**

Compound 11 (46 mg) and N-(1-naphtyl)ethylenediamine hydrochloride (30 mg) were dissolved in DMF (0.4 ml) and THF (1.6 ml) at 0°C and then stirred. HOBt (19 mg), EDC (24 mg) and N,N-diisopropylethylamine (0.054 ml) were added, and then stirred at room temperature for 12 hours. The solution was concentrated under reduced pressure, and then the residue was diluted with dichloromethane and then washed with the aqueous solution three times. The organic layer was further washed with the saturated sodium chloride solution once, and dried over anhydrous magnesium sulfate. The solution was concentrated under reduced pressure, and then the residue was purified by silica gel chromatography (dichloromethane:methanol=100:1→50:1→30:1) to prepare 40.3 mg of compound XXIV (yield: 92%). The structure of the product was identified by $^{1}$H-NMR.
$^{1}$H-NMR (600 MHz, DMSO)(VI): 8.23-8.21 (t, 1H, NHCO), 8.15 (s, 1H, H-2), 8.07-8.05 (d, 1H, Ar), 7.74-7.73 (d, 1H, Ar), 7.43-7.364 (m, 4H, Ar+$NH_2$), 7.33 (s, 1H, CHBr), 7.28-7.25 (t, 1H, Ar), 7.10-7.09 (d, 1H, Ar), 6.83 (s, 1H, CHS) , 6.54-6.52 (d, 1H, Ar), 6.20-6.18 (t, 1H, NH), 6.07 (s, 2H, $OCH_2O$), 4.44-4.42 (t, 2H, $CH_2N$), 3.35-3.29 (m, 2H, $CH_2NH$), 3.24-3.21 (m. 2H, $CH_2NH$), 2.699-2.67 (t, 2H, $CH_2CO$) .
$^{13}$C-NMR (400 MHz, DMSO)(VI): 35.455, 38.327, 40.84, 43.667, 103.24, 103.36, 112.208, 113.644, 115.812, 116.113, 120.303, 122.083, 123.577, 124.647, 125.731, 126.251, 127.482, 128.61, 134.689, 144.271, 144.469, 148.556, 149.069, 151.398, 153.537, 155.823, 170.378.
LC/MS : 608.5 (M+H, 98%)

References:

[1] JACS, 2000, 122, p.87-95
[2] WO 2008/ 115262
[3] JOC, 2005, 70, p.717-720


**<Detection of HSP 90 (heat shock protein 90) protein and Test of its activity using iFRET probe of Chemical Formula XXIV>**

The fluorescence characterization of the synthesized compound XXIV was performed and shown in FIG. 17. After excitation at 260, 280, 340, and 360 nm, the emitted fluorescence spectra were examined. When the compound was excited at 340 nm, it showed approximately 2-fold increased fluorescence intensity, compared to excitation at 280 nm.

The left graph of FIG. 18 shows the iFRET signals detected by using Chemical Formula XXIV and purified HSP90 (heat shock protein 90) protein. Compound XXIV was dissolved in DMSO, and then diluted with distilled water to a final concentration of 2 μM. When Compound XXIV or the protein was present alone, no fluorescence signals were newly observed around 450 nm. However, when both HSP90 protein and Compound XXIV were present together, new fluorescence signals were observed at approximately 450 nm, resulting from iFRET phenomenon.

The right graph of FIG. 18 is the result of reexamining iFRET signals using Compound XXIV, purified HSP90 protein, and a control group BSA (bovine serum albumin). 2 μM Compound XXIV was used, and HSP90 protein and BSA protein were used at the final concentration of 200 nM. At this time, the used buffer was phosphate buffered saline (PBS; pH 7.4). As shown in the figure, iFRET signals result from compound XXIV and HSP90 protein which are selectively binding. Compound XXIV and BSA made no iFRET signals.

FIG. 19 is a graph showing iFRET signals detected by using HSP90 protein and Chemical Formula XXIV

selectively binding thereto. When HSP90 was present alone, fluorescence was observed at approximately 350 nm by excitation at 280 nm. Fluorescence of Chemical Formula XXIV (indicated by Dye) was observed at approximately 430 nm by excitation at 280 nm. The mixture of Chemical Formula XXIV and HSP90 protein showed increased fluorescence at 430 nm, unlike that of HSP90 protein or Chemical Formula XXIV alone. As a control group, BSA (bovine serum albumin) was used. The mixture of BSA and Chemical Formula XXIV showed no iFRET signals, suggesting that the iFRET phenomenon results from selective binding of HSOP90 protein and Chemical Formula XXIV.

The iFRET phenomenon due to HSP90 and Chemical Formula XXIV was examined by FIGs 18 and 19. In order to demonstrate the iFRET phenomenon due to selective binding, geldanamycin and PU-H64 that are known to bind with HSP90 were used to perform competition assay. All of geldanamycin, PU-H64, and Chemical Formula XXIV bind to the ATP binding site of HSP90, and these compounds compete with each other when they co-exist. On this basis, competition of geldanamycin and Chemical Formula XXIV, and competition of PU-H64 and Chemical Formula XXIV were induced for the single binding site to induce a reduction in iFRET signals, respectively, as shown in the left and right figures of FIG. 20. Production of iFRET signals was observed by using HSP90 protein and Chemical Formula XXIV, and the reduction in iFRET signals was observed by adding the competitive inhibitor geldanamycin or PU-H64 at various concentrations, suggesting that Chemical Formula XXIV selectively binds to the ATP-binding site of HSP90 protein to produce iFRET signals.

FIG. 21 is the result of binding degree of the synthesized Chemical Formula XXIV and HSP90 protein. The core structure used in the synthesis of Chemical Formula XXIV is PU-H64 *(*J. Med. Chem. 2006, 49, 4953-4960), and this structure binds to HSP90. The iFRET probe used in this experiment is a structure in which a fluorescent nucleus is introduced into PU-H64. In order to examine the actual binding degree of this probe and HSP90 protein, analysis of enzyme kinetics was performed to confirm that its dissociation constant is approximately 160 nM. The concentration of Chemical Formula XXIV as the iFRET probe was fixed and HSP90 was reacted at various concentrations to measure emitted fluorescence intensity. Based on this measured values, the dissociation constant was calculated. The reciprocal of the HSP90 concentration and the reciprocal of the fluorescence value were plotted to calculate the dissociation constant.

**Example 10: Preparation of streptavidin-specific probes for iFRET of Chemical Formulae XXV to XXVIII**

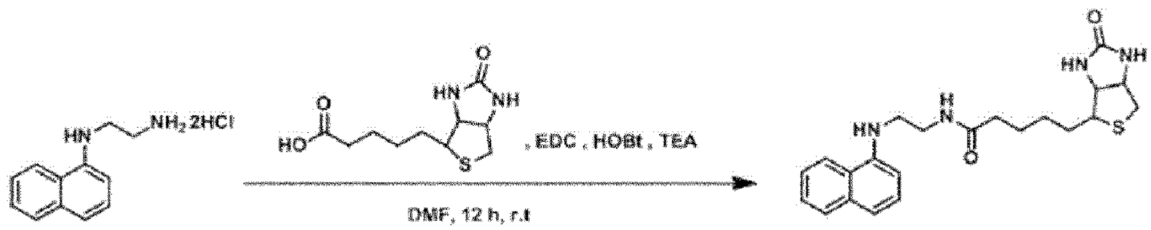


(XXV)

**Synthesis of N-biotinyl N'-(1-naphthyl)ethylenediamine (XXV)**

N-(1-naphthyl)ethylenediamine dihydrochloride (254 ng), biotin (200 mg) and triethylamine (0.58 ml) were dissolved in N,N-dimethylformamide (DMF, 10 ml), and 1-ethyl-3-(3-dimethylaminopropyl)carbodiimide (EDC, 236 mg) and hydroxybenzotriazole (HOBt, 166 mg) were added at 0°C and stirred at room temperature for 12 hours. The solution was concentrated under reduced pressure, and the residue was diluted with ethyl acetate and washed with distilled water. The organic layer was further washed with the saturated sodium chloride solution, and then dried over anhydrous magnesium sulfate (anhydrous $MgSO_4$). The solution was concentrated under reduced pressure, and then the residue was purified by silica gel chromatography (methanol:dichloromethane=1:10) to prepare Compound XXV (380 mg, yield: 85%). The structure of the product was identified by $^1$H-NMR.

$^1$H-NMR (500 MHz, DMSO-d6) : 8.17 (d, 1H) , 8.05 (d, 1H) 7.74 (d, 1H) , 7.44-7.36 (d, 1H) , 7.28 (t, 2H) , 7.10 (dd, 1H) , 6.54 (d, 1H) , 6.52 (d, 1H) , 4.23 (t, 1H) , 4.01 (dd, 1H) , 3.39(t,2H), 3.28 (m, 1H) , 3.27(t,2H), 2.74 (dd, 1H) , 2.55 (d, 1H) , 2.11 (t, 2H) , 1.56(m,2H), 1.55(m,2H), 1.27(m,2H).

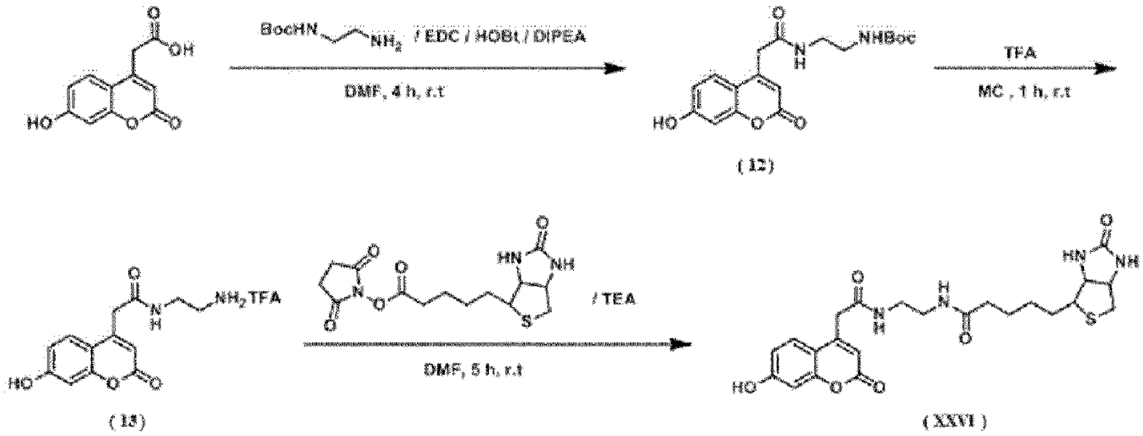

(12)
(13)
(XXVI)

**Synthesis of N-(7-hydroxycoumarin-4-acetyl)-N'-Boc-ethylenediamine (12)**

7-Hydroxycoumarin-4-acetic acid (2 g), N-Boc-1,2-diaminoethane (3 g) and N,N-diisopropylethylamine (8 ml) were dissolved in DMF (30 ml), and stirred at 0°C for 15 minutes. Then, EDC (2.6 g) and HOBt (1.37 g) were added, and further stirred at room temperature for 4 hours. The solution was concentrated under reduced pressure, and then the residue was diluted with ethyl acetate, and washed with a 10% citric acid aqueous solution and saturated sodium bicarbonate aqueous solution, respectively. The organic layer was further washed with the saturated sodium chloride solution, and then dried over anhydrous magnesium sulfate. The solution was concentrated under reduced pressure and then the residue was purified by silica gel chromatography (methanol:dichloromethane=1:10) to prepare Compound 12 (2.6 g, yield: 79%). The structure of the product was identified by $^1$H-NMR.
$^1$H-NMR (400 MHz, DMSO-d6): 7.55-7.52 (d, 1H), 6.82 (d, 1H) , 6.79 (s, 1H), 6.23 (s, 1H), 3.83(s,2H), 3.17-3.11(q,2H), 2.96-2.91 (q, 2H), 1.51 (s, 9H).

**Synthesis of (7-hydroxycoumarin-4-acetic acid)-2-aminoethylamide (13)**

Compound 12 (60 mg) was dissolved in MC:TFA (3:1, 1 ml), and stirred at room temperature for 1 hour. The reactant was dissolved in methylene chloride and then distilled under reduced pressure. This process was repeated twice, and recrystallization was performed in combination of methanol and diethylether. The desired crystalline product was dried under vacuum to prepare Compound 13 (40 mg, yield: 81%). The structure of the product was identified by $^1$H-NMR.
$^1$H-NMR (400 MHz, DMSO-d6): 8.38 (t, 1H), 7.60 (d, 1H) , 6.81 (d, 1H), 6.73 (s, 1H), 3.67(s,2H), 3.30-3.27(q,2H), 2.88-2.84(t,2H).

**Synthesis of N-(7-hydroxycoumarin-4-acetyl)-N'-(biotinyl)ethylenediamine (XXVI)**

Compound 13 (50 mg), biotin N-hydroxysuccinimide ester (55 mg) and TEA (0.036 ml) were dissolved in DMF (1 ml) at 0°C and stirred at room temperature for 5 hours. The solution was concentrated under reduced pressure, and then an excessive amount of water was added to the residue. The desired crystalline product thus formed was filtered and further purified by silica gel chromatography (methanol:dichloromethane=1:10) to prepare Compound XXVI (40 mg, yield: 63%). The structure of the product was identified by $^1$H-NMR.
$^1$H-NMR (400 MHz, DMSO-d6): 8.22 (s, 1H), 7.82 (s, 1H) , 7.59-7.56 (d, 1H), 6.80-6.77 (q, 1H), 6.71-6.71 (d, 1H), 6.43-6.37 (d, 2H) , 6.16 (s, 1H) , 4.32 (m, 1H) , 4.12 (m, 1H) , 3.63(s,2H), 3.10(s,5H), 2.84-2.79 (dd, 1H), 2.59 (d, 1H), 2.03 (t, 1H) , 1.48-1.28 (m, 6H) .

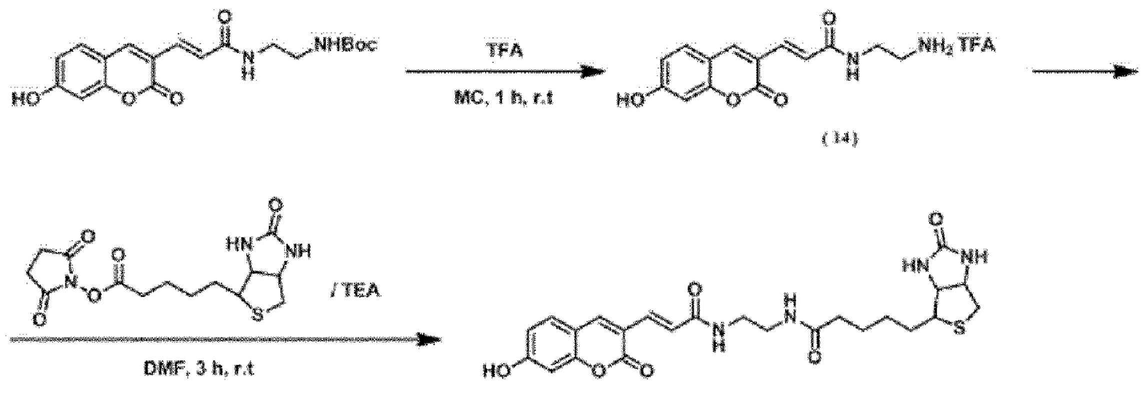

(14)

(XXVII)

**Synthesis of (E)-7-hydroxycoumarin-3-acrylic acid 2-aminoethylamide (14)**

N-((E)-7-hydroxycoumarin-3-acrylic acid)-N'-tert-butyl ethylenediamine (70 mg) was dissolved in MC:TFA (2:1, 1 ml), and stirred at room temperature for 1 hour. The reactant was further dissolved in methylene chloride, and then distilled under reduced pressure. This process was repeated twice, and then the desired crystalline product thus formed was dried under vacuum to prepare Compound 14 (65 mg, yield: 95%). The structure of the product was identified by $^1$H-NMR.

$^1$H-NMR (400 MHz, DMSO-d6) : 8.45 (t, 1H), 8.28 (s, 1H), 7.83(b.s,2H), 7.59-7.57 (d, 1H), 7.34-7.30 (d, 1H), 7.09-7.05 (d, 1H), 6.86-6.83 (dd, 1H), 6.76 (d, 1H), 3.42-3.37(q,2H), 2.94-2.89 (q, 2H) .

**Synthesis of N-((E)-7-hydroxycoumarin-3-acryl)-N'-(biotinyl)ethylenediamide (XXVII)**

Compound 14 (100 mg), biotin N-hydroxysuccinimide ester (89 mg) and TEA (0.06 ml) were dissolved in DMF (1 ml), and then stirred at room temperature for 3 hours. An excessive amount of ethyl acetate was added to the reactant, and the precipitate thus obtained was filtered, followed by recrystallization in combination of methanol and diethylether. The desired crystalline product thus formed was dried under vacuum to prepare Compound XXVII (59 mg, yield: 46%). The structure of the product was identified by $^1$H-NMR.

$^1$H-NMR (400 MHz, DMSO-d6) : 8.28 (d, 2H), 8.25 (s, 1H), 7.58-7.63 (d, 1H), 7.06-7.02 (d, 1H), 6.88-6.86 (d, 1H), 6.84-6.82 (d, 1H) , 6.74 (s, 1H), 6.54 (d, 1H), 6.52 (d, 1H), 4.23 (t, 1H), 4.01 (dd, 1H) , 3.27(t,2H), 3.19(q,2H), 3.02(q,2H), 2.74 (dd, 1H), 2.55 (d, 1H) , 2.11(t,2H), 1.56(m,2H), 1.55(m,2H), 1.27(m,2H).

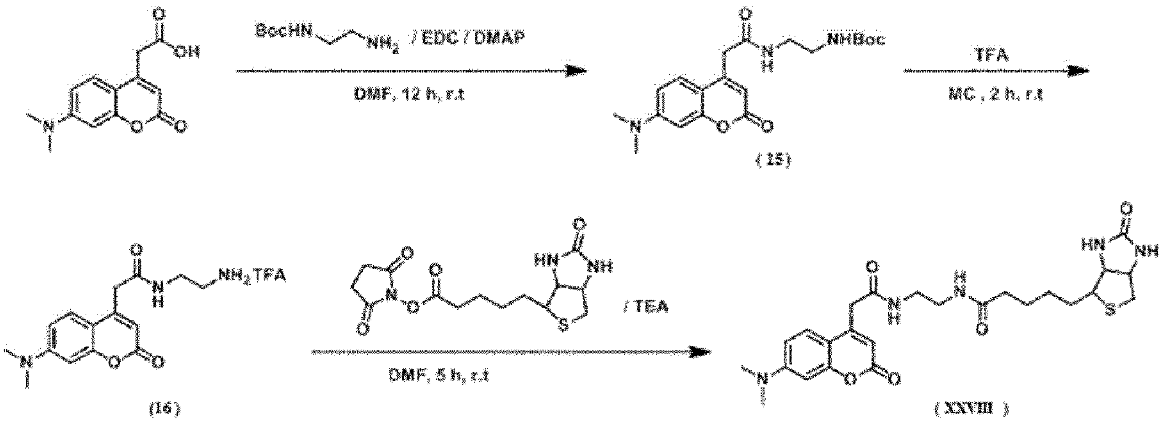

(15)

(16)

(XXVIII)

**Synthesis of N-(7-dimethylaminocoumarin-4-acetyl)-N'-Boc-ethylenediamine (15)**

7-Dimethylaminocoumarin-4-acetic acid (100 mg) and N-Boc-1,2-diaminoethane (71 mg) were dissolved in DMF (4 ml) at 0°C, and stirred for 15 minutes. Then, EDC (116 mg) and 4-(dimethylamino)pyridine (DMAP, 6 mg) were added and further stirred at room temperature for 12 hours. The solution was concentrated under reduced pressure, and then the residue was diluted with ethyl acetate, and washed with the 10% citric acid aqueous solution and saturated

sodium bicarbonate aqueous solution, respectively. The organic layer was further washed with the saturated sodium chloride solution, and then dried over anhydrous magnesium sulfate (anhydrous $MgSO_4$). The solution was concentrated under reduced pressure, and the residue was purified by silica gel chromatography (methanol:dichloromethane=1:20) to prepare Intermediate 15 (87 mg, yield: 55%). The structure of the product was identified by $^1$H-NMR.
$^1$H-NMR (400 MHz, $CDCl_3$): 7.48 (d, 1H), 6.63 (d, 1H), 6.61 (d, 1H) , 6.05 (s, 1H) , 4.84 (s, 1H) , 3.62 (s, 2H) , 3.35-3.31(q,2H), 3.23-3.20(q,2H), 3.06(s,6H), 1.41(s,9H).

**Synthesis of (7-dimethylaminocoumarin-4-acetic acid)-2-aminoethylamide (16)**

Compound 15 (80 mg) was dissolved in MC:TFA (2:1, 1 ml), and stirred at room temperature for 2 hours. The reactant was dissolved in methylene chloride, and distilled under reduced pressure. This process was repeated twice, and then the residue was recrystallized in combination of methanol and diethylether. The desired crystalline product thus formed was dried under vacuum to prepare Compound 16 (53 mg, yield: 67%). The structure of the product was identified by $^1$H-NMR.
$^1$H-NMR (400 MHz, $CDCl_3$) : 7.54 (d, 1H), 6.7 (d, 1H) , 6.57 (s, 1H), 6.04 (s, 1H) , 3.73(s,2H), 3.47(t,2H), 3.31(s,6H), 3.05 (t, 2H) .

**Synthesis of N-((E)-7-dimethylaminocoumarin-3-acryl)-N'-(biotinyl)ethylenediamide (XXVIII)**

Compound 16 (40 mg), biotin N-hydroxysuccinimide ester (41 mg) and TEA (0.03 ml) were dissolved in DMF (1 ml), and stirred at room temperature for 5 hours. The solution was concentrated under reduced pressure, and then excessive amount of water was added to the residue. The desired crystalline product thus formed was filtered, and further purified by silica gel chromatography (methanol:dichloromethane=1:10) to prepare Compound XXVIII (30 mg, yield: 52%). The structure of the product was identified by $^1$H-NMR.
$^1$H-NMR (400 MHz, DMSO-d6) : 8.23 (s, 1H), 7.83 (s, 1H) , 7.54-7.52 (d, 1H) , 6.74-6.71 (q, 1H) , 6.56-6.55 (d, 1H), 6.43-6.37(d,2H), 6.00 (s, 1H) , 4.32 (m, 1H) , 4.13 (m, 1H), 3.56 (s, 2H) , 3.02 (s, 5H) , 2.89 (s, 6H) , 2.84-2.79 (dd, 1H), 2.59 (d, 1H) , 2.03 (t, 1H) , 1.48-1.28 (m, 6H) .

**<Detection of streptavidin protein and Test of its activity using iFRET probes of Chemical Formulae XXV to XXVIII>**

The results of detecting streptavidin using Compounds of Chemical Formulae XXV to XXVIII as probes for iFRET are shown in FIGs. 23 to 26.

As in FIG. 23 showing the result of using the compound of Chemical Formula XXV as a probe for iFRET, the probe itself emitted low fluorescence (blue line) at 430 nm by UV irradiation at 280 nm; the protein (streptavidin) emitted fluorescence (green line) at 340 nm by UV irradiation at 280 nm. The mixture of the probe for iFRET and the desired protein streptavidin showed increased fluorescence at 430 nm by UV irradiation at 280 nm (black line). This result suggest that the compound itself of the probe developed in the present invention shows low fluorescence by UV irradiation of a particular wavelength, but FRET occurs resulting from energy transfer from tryptophan when the compound binds to streptavidin to be close to tryptophan as an energy donor present in streptavidin, indicating that the probe of the present invention is a binding sensitive turn-on fluorescence probe.

As in FIG. 24 showing the result of using the compound of Chemical Formula XXVI as a fluorescent probe, the probe itself emitted low fluorescence (blue line) at 460 nm by UV irradiation at 280 nm; the protein (streptavidin) emitted fluorescence (green line) at 340 nm by UV irradiation at 280 nm. The mixture of the probe for iFRET and streptavidin showed increased fluorescence at 460 nm longer than the emission wavelength of the probe XXV by UV irradiation at 280 nm (black line). Further, it was confirmed that the fluorescence increasing rate at the above emission wavelength was 600% or more, indicating production of effective iFRET signals.

As in FIG. 25 showing the result of using the compound of Chemical Formula XXVII as a probe, the mixture of the probe having different emission wavelength and streptavidin also showed increased fluorescence at 480 nm by UV irradiation at 280 nm (black line). Because the compound of Chemical Formula XXVII emitted fluorescence at the longer wavelength than the known iFRET probe, signal-to-noise ratio is minimized during real-time cell imaging, thereby providing more clear images.

FIG. 26 shows iFRET signals produced by binding of streptavidin and the compound of Chemical Formula XXVIII which is the synthesized probe for iFRET (black line in the figure). In order to examine target specificity of the probe having Chemical Formula XXVIII, the probe was exposed to BSA to examine whether iFRET signals were produced by binding of the probe to BSA. However, no signals were observed (dark blue line) when the fluorescent probe of the present invention was reacted with BSA. Therefore, it was confirmed that the biotin-conjugated iFRET fluorescent probe of the present invention selectively binds to streptavidin to produce iFRET signals.

Table 2 shows fluorescent characteristics and FRET efficiency of the fluorescent probes having biotin-conjugated Chemical Formulae XXV to XXVIII.

[Table 2]

| Compound (Biotin conjugated) | Quantum yield ($\Phi_F$) | FRET efficiency $E = (1 - I_{da}/ I_d)$ | $R_0$ (Fõrster distance) |
|---|---|---|---|
| XXV | 0.7 | 0.225 (308 nm ~ 373 nm, 50 nM) | 1.837 nm |
| XXVI | 0.7 | 0.141 ( 300 nm ~ 375 nm, 50 nM) | 1.675 nm |
| XXVII | 0.105 | 0.22 (300 nm ~ 420 nm, 150 nM) | 2.161 nm |
| XXVIII | 0.55 | 0.359 (300 nm ~ 420 nm, 150 nM) | 2.102 nm |

The quantum yield of each compound was measured in 0.1 M pH 10.0 phosphate buffer. 4-methyl-umbelliferone ($\Phi F = 0.63$) was used as a standard material, tryptophan ($\Phi F = 0.12$) was used as an FRET donor to calculate the quantum yield. FRET efficiency was calculated based on the results measured in PBS buffer at pH 7.4, and fluorescence was measured using an LS55 fluorescence spectrometer manufactured by Perkin Elmer. The quantum yield of each compound is shown in the table, and FRET efficiency of each synthesized compound showed high energy transfer efficiency of 0.225, 0.141, 0.22 and 0.359, respectively. Foster distance between the iFRET fluorescent probe and the protein can be confirmed by the values obtained from data, indicating that the distance between the iFRET fluorescent probe and streptavidin is within the distance range for effective production of iFRET signals.

**Example 11: Preparation of caspase-3 protein-specific probes for iFRET of Chemical Formulae IXXX to XXXII**

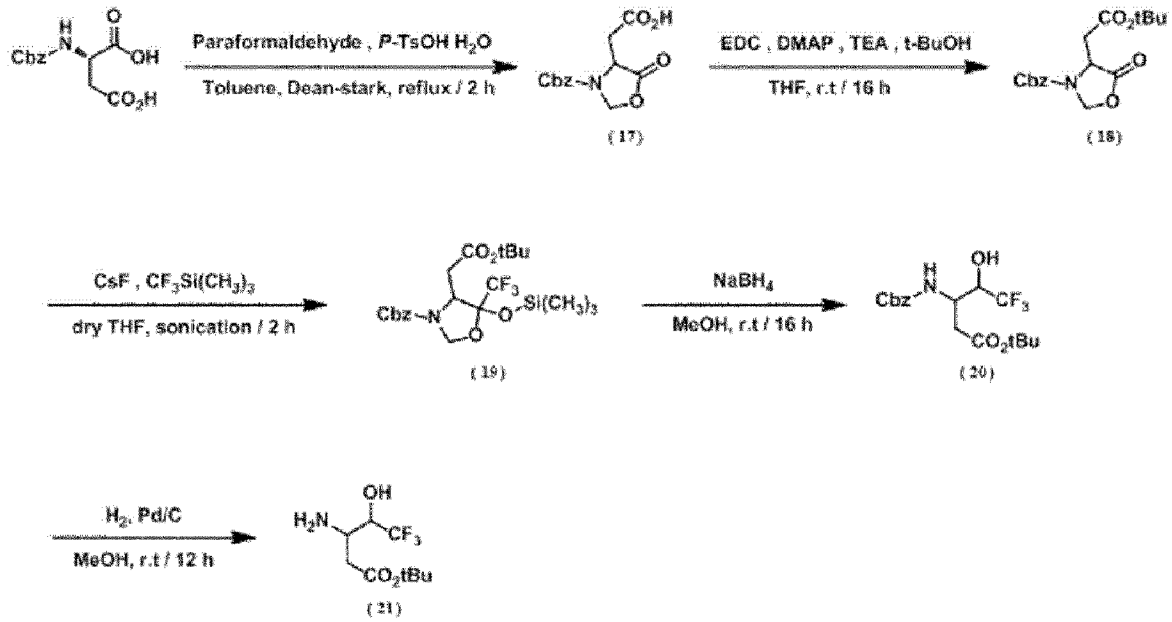

(17) (18) (19) (20) (21)

**Synthesis of 2-(3-(benzyloxycarbonyl-5-oxooxazolidin-4-yl)acetic acid (17)**

N-Cbz-L-aspartic acid (2.16 g), paraformaldehyde (1.53 g) and p-toluenesulfonic acid (0.16 g) were dissolved in toluene (150 ml), and then refluxed with stirring using a Dean-stark trap for 2 hours. The reactant was cooled at room temperature and the solvent was added to silica gel. After filtration with diethylether, concentration was performed under reduced pressure to prepare Compound 17 (2.04 g, 90 %). The structure of the product was identified by $^1H$-NMR.
$^1H$-NMR (600 MHz, $CDCl_3$) : 7.40-7.35(m,5H), 5.4 (br, 1H), 5.30-5.10 (m, 3H) , 4.38 (t, 1H) , 2.93(d,2H).

**Synthesis of tert-butyl-2-(3-(benzyloxycarbonyl-5-oxooxazolidin-4-yl)acetate (18)**

Compound 17 (1.8 g), t-BuOH (1.8 ml), EDC (1.5 g) and DMAP (0.35 g) were dissolved in dry THF (70 ml),

and then stirred for 5 minutes. TEA (0.9 ml) was added to the reactant and then stirred at room temperature for 16 hours. The solution was concentrated under reduced pressure and then the residue was diluted with ethyl acetate, and then washed with the 5% citric acid aqueous solution and 5% sodium bicarbonate aqueous solution. The organic layer was further washed with the saturated sodium chloride solution, and then dried over anhydrous magnesium sulfate. The solution was concentrated under reduced pressure, and the residue was purified by silica gel chromatography (hexane:ethyl acetate=1:9 → 1:4) to prepare Compound 18 (1.01 g, yield: 47%). The structure of the product was identified by $^1$H-NMR.
$^1$H-NMR (600 MHz, $CDCl_3$) : 7.38-7.34 (m, 5H) , 5.55 (br, 1H) , 5.34 (s, 1H) , 5.22-5.17 (q, 2H) , 4.29 (br, 1H) , 2.96-2.93(d,2H), 1.42(s,9H).

**Synthesis of benzyl 4-(2-(tert-butoxy)-2-oxoethyl)-5-(trifluoromethyl)-5-(trimethylsiloxy)oxazolidine-3-carboxylate (19)**

Compound 18 (0.1 g), cecium fluoride (0.006 g) and 2 M (trifluoromethyl)trimethylsilane (0.18 ml) were dissolved in dry THF (3 ml), and then sonicated at room temperature for 2 hours. The solution was concentrated under reduced pressure, and the residue was diluted with ethyl acetate, and washed with the aqueous solution. The organic layer was washed with the saturated sodium chloride solution, and then dried over anhydrous magnesium sulfate. The solution was concentrated under reduced pressure, and then the residue was purified by silica gel chromatography(hexane:ethyl acetate=1:10) to prepare Compound 19 (0.1 g, yield: 70%). The structure of the product was identified by $^1$H-NMR.
$^1$H-NMR (600 MHz, $CDCl_3$): 7.38-7.33(m,5H), 5.32-5.17 (br, 3H) , 4.95 (s, 1H) , 4.84 (q, 1H) , 2.46(d,2H), 1.43(s,9H), 0.21 (s, 9H) .

**Synthesis of tert-butyl 3-(benzyloxycarbonyl)amino)-5,5,5-trifluoro-4-hydroxypentanoate (20)**

Sodium borohydride (0.36 g) was slowly added dropwise to Compound 19 (0.46 g) in methanol (20 ml), and then stirred at room temperature for 16 hours. The aqueous solution (20 ml) was added to the reactant, and then extracted with ethyl acetate three times. The organic layer was washed with the saturated sodium chloride solution, and then dried over anhydrous magnesium sulfate. The solution was concentrated under reduced pressure, and then the residue was purified by silica gel chromatography (hexane:ethyl acetate=1:5) to prepare Compound 20 (0.22 g, yield: 61%). The structure of the product was identified by $^1$H-NMR.
$^1$H-NMR (600 MHz, $CDCl_3$) : 7.36-7.31 (m, 5H) , 5.55 (d, 1H), 5.13-5.07 (q, 2H) , 4.68 (s, 1H) , 4.65 (s, 1H), 4.25-4.11 (d, 2H) , 2.70 (q, 2H), 1.46 (s, 9H) .

**Synthesis of tert-butyl 3-amino-5,5,5-trifluoro-4-hydroxyhexapentanoate (21)**

Compound 20 (0.17 mg) and 10% Pd/C (Palladium on activated charcoal, 23 mg) were dissolved in methanol (10 ml), and then stirred under hydrogen atmosphere for 12 hours. The reactant was filtered using Celite, and then concentrated under reduced pressure to prepare Compound 21 (0.1 g, yield: 81%). The structure of the product was identified by $^1$H-NMR.
$^1$H-NMR (600 MHz, $CDCl_3$) : 5.22 (bs, 2H) , 4.26 (q, 1H), 3.82 (q, 1H) , 2.78(d,2H), 1.31(s,9H).

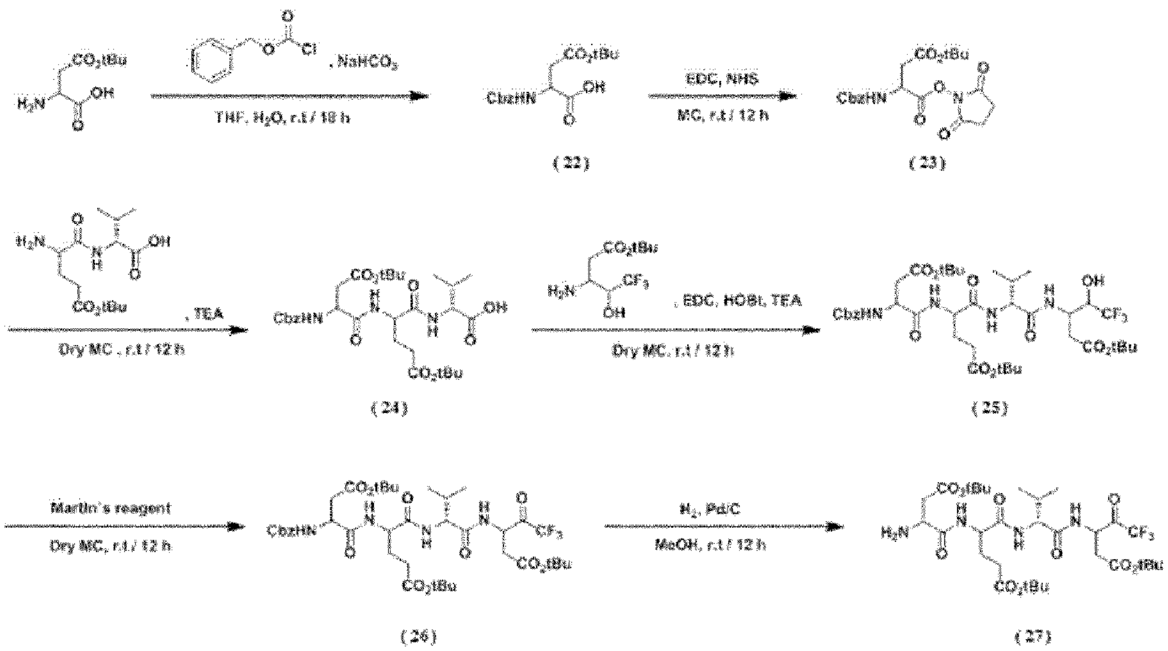


(22) (23) (24) (25) (26) (27)

**Synthesis of N-Cbz-aspartic acid 4-tert-butyl ester (22)**

L-Aspartic acid 4-tert-butyl ester (5 g) and $NaHCO_3$ (4.43 g) were dissolved in water (100 ml), and the stirred. A solution prepared by diluting benzyl chloroformate (4.14 ml) with THF (100 ml) was added dropwise to the reactant and then stirred at room temperature for 18 hours. The organic solvent was removed under reduced pressure, and then the aqueous solution layer was washed with ethyl acetate twice. The resultant was acidified with 10% citric acid aqueous solution, and then the desired product was extracted with ethyl acetate. The organic layer was washed with the saturated sodium chloride solution, and then dried over anhydrous magnesium sulfate. The resultant was dried under vacuum to prepare Compound 22 (7.2 g, yield: 85%). The structure of the product was identified by $^1$H-NMR.
$^1$H-NMR (600 MHz, $CDCl_3$) : 7.24-7.34 (m, 5H), 5.31-5.18 (s, 3H) , 4.58-4.67 (m, 1H) , 2.71(q,2H), 1.46(s,9H).

**Synthesis of N-Cbz-aspartic acid-N'-hydroxysuccinimide ester-4-tert-butyl ester (23)**

Compound 22 (3 g), EDC (1.95 g) and NHS (1.18 g) were dissolved in dichloromethane (40 ml), and then stirred at room temperature for 12 hours. The organic layer was washed with the saturated sodium bicarbonate aqueous solution, and the saturated sodium chloride solution, and then dried over anhydrous magnesium sulfate. Recrystallization was performed with methanol and diethylether, and the resultant was dried under vacuum to prepare Compound 23 (3 g, yield: 77%). The structure of the product was identified by $^1$H-NMR.
$^1$H-NMR (600 MHz, $CDCl_3$) : 7.24-7.34 (m, 5H) , 5.33-5.21(s,3H), 4.72 (m, 1H) , 2.94(s,4H), 2.67(q,2H), 1.46(s,9H).

**Synthesis of N-Cbz-Asp($CO_2$tBu)-L-Glu($CO_2$tBu)-L-Val (24)**

TEA (0.15 ml) was slowly added dropwise to Compound 23 (0.15 g) and amino acid EV (0.1 g) in dichloromethane (40 ml), and then stirred at room temperature for 12 hours. The reactant was washed with the 10% citric acid aqueous solution, the saturated sodium bicarbonate aqueous solution, and saturated sodium chloride solution, and then dried over anhydrous magnesium sulfate. The solution was concentrated under reduced pressure, and then the residue was purified by silica gel chromatography (methanol:dichloromethane = 1:10) to prepare Compound 24 (0.14 g, yield: 66%). The structure of the product was identified by $^1$H-NMR.
$^1$H-NMR (600 MHz, DMSO-d6): 10.52 (s, 1H), 7.38-7.33 (m, 5H), 5.33-5.21 (s, 3H), 4.60 (m, 1H) , 4.33 (m, 1H) , 4.12 (m, 1H) , 2.89 (s, 1H) , 2.73 (s, 1H), 2.20(m,2H), 2.06 (m, 1H) , 1.99 (m, 1H) , 1.74 (m, 1H) , 1.38 (s, 9H) , 1.35 (s, 9H) , 0.88 (q, 6H) .

**Synthesis of N-Cbz-Asp($CO_2$tBu)-L-Glu($CO_2$tBu)-L-Val-Asp($CO_2$tBu)-(OH)-CF3 (25)**

Compound 24 (0.13 g), Compound 21 (0.05 g) and TEA (0.15 ml) were dissolved in dichloromethane (6 ml), and then stirred for 5 minutes. EDC (0.05 g) and HOBt (0.06 g) were added to the reactant, and then further stirred at

room temperature for 12 hours. The reactant was washed with the 10% citric acid aqueous solution and the saturated sodium bicarbonate aqueous solution. The organic layer was further washed with the saturated sodium chloride solution, and then dried over anhydrous magnesium sulfate. The solution was concentrated under reduced pressure, and then the residue was purified by silica gel chromatography (methanol:dichloromethane = 1:10) to prepare Compound 25 (0.12 g, yield: 67%). The product was identified by mass spectrometric analysis.
LC/MS: 833.4 (M+H).

**Synthesis of N-Cbz-Asp($CO_2$tBu)-L-Glu($CO_2$tBu)-L-Val-Asp($CO_2$tBu)-(ketone)-CF3 (26)**

Compound 25 (0.2 g) and Dess-Martin's reagent (0.45 g) were dissolved in dichloromethane (12 ml), and then stirred at room temperature for 12 hours. The precipitate was filtered through Celite, and then filtrate was concentrated under reduced pressure. The residue was purified by silica gel chromatography (methanol:dichloromethane = 1:10) to prepare Compound 26 (0.17 g, yield: 86%). The product was identified by mass spectrometric analysis.
LC/MS: 831.4 (M+H).

**Synthesis of L-Asp($CO_2$tBu)-L-Glu($CO_2$tBu)-L-Val-Asp(CO2tBu)-(ketone)-CF3 (27)**

Compound 27 (0.05 g) and 10 % Pd/C (Palladium on activated charcoal, 22 mg) were dissolved in methanol (5 ml), and then stirred under hydrogen atmosphere for 12 hours. The reactant was filtered using Celite, and then concentrated under reduced pressure to prepare Compound 27 (0.03 g, yield: 71%). The product was identified by mass spectrometric analysis.
LC/MS: 697.4 (M+H).

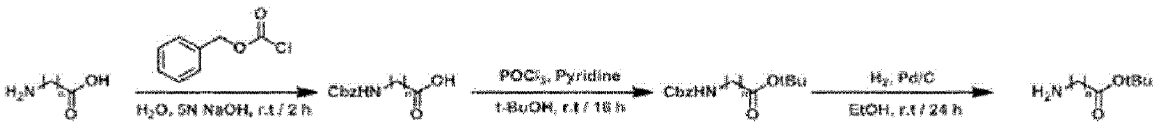


(28) (29) (30)

**Synthesis of Cbz-3-aminopropionic acid,Cbz-6-aminohexane acid (28)**

Aminocarboxylic acid (3-aminopropanoic acid or 6-aminohexanoic acid, 56 mmol or 38 mmol) was dissolved in water (15 ml), and then stirred at 0°C for 15 minutes. CbzCl (1.1 eq) and 5 N NaOH (1 eq) were slowly added dropwise to the reactant and maintained at pH 10.0. The aqueous solution layer was washed with diethylether twice and then acidified with 1 N hydrochloric acid, and the desired product was extracted with dichloromethane. The organic layer was washed with the saturated sodium chloride solution, and then dried over anhydrous magnesium sulfate. The resultant was recrystallized from diethylether at -70°C, and dried under vacuum to prepare Compound 28 (n =3, 10 g, yield: 77% / n = 5, 7.6 g, 75%). The structure of the product was identified by $^1$H-NMR.
$^1$H-NMR (400 MHz, $CDCl_3$, n=2): 7.34 (br, 5H), 5.33 (br, 1H), 5.09 (s, 2H) , 3.46 (m, 2H) , 2.58 (t, 2H) .
$^1$H-NMR (400 MHz, $CDCl_3$, n=5) : 7.30-7.40 (m, 5H), 5.09 (s, 2H) , 4.80 (br, 1H) , 3.19 (q, 2H) , 2.34 (t, 2H) , 1.64 (q, 2H) , 1.52(q,2H), 1.37(m,2H).

**Synthesis of tert-butyl Cbz-3-aminopropionate,tert-butyl Cbz-6-aminohexanonate (29)**

Compound 28 (1 eq) and pyridine (18 eq) were dissolved in tert-butanol, and then stirred at -10°C for 15 minutes. $POCl_3$ (1.1 eq) was slowly added dropwise to the reactant and then stirred at room temperature for 16 hours. The solution was concentrated under reduced pressure, and then the residue was diluted with ethyl acetate and then washed with water and saturated sodium carbonate aqueous solution. The organic layer was further washed with saturated sodium chloride solution, and then dried over anhydrous magnesium sulfate. The solution was concentrated under reduced pressure and the residue was purified by silica gel chromatography(methanol:dichloromethane = 1:50) to prepare Compound 29 (n =3, 1 g, yield: 60% / n = 5, 1.35 g, 56%). The structure of the product was identified by $^1$H-NMR.
$^1$H-NMR (400 MHz, $CDCl_3$, n=2): 7.33 (m, 5H), 5.34 (br, 1H), 5.10 (s, 2H) , 3.42(q,2H), 2.44(t,2H), 1.44(s,9H).
$^1$H-NMR (400 MHz, $CDCl_3$, n=5) : 7.30-7.36(m,5H), 5.09 (s, 2H) , 4.78 (br, 1H) , 3.19 (q, 2H) , 2.20 (t, 2H) , 1.65-1.30(m,6H), 1.43(s,9H).

**Synthesis of tert-butyl 3-aminopropionate,tert-butyl 6-aminohexanonate (30)**

Compound 29 (1 eq) and 10% Pd/C (Palladium on activated charcoal, catalytical amount) were dissolved in ethanol, and then stirred under hydrogen atmosphere for 24 hours. The reactant was filtered through Celite and then concentrated under reduced pressure. The precipitate was diluted with ethyl acetate, and then further filtered, and concentrated under reduced pressure. The product was dried under vacuum to prepare Compound 30 (n =3, 0.32 g, yield: 95% / n = 5, 0.51 g, 92%). The structure of the product was identified by $^1$H-NMR.
$^1$H-NMR (400 MHz, DMSO-d6, n=2): 2.73(t,2H), 2.27(t,2H), 1.93(br,2H), 1.39(s,9H).
$^1$H-NMR (400 MHz, $CDCl_3$, n=5): 2.67(t,2H), 2.21(t,2H), 1.65(s,2H), 1.60(m,2H), 1.40-1.50(m,2H), 1.44(s,9H), 1.36(m,2H).

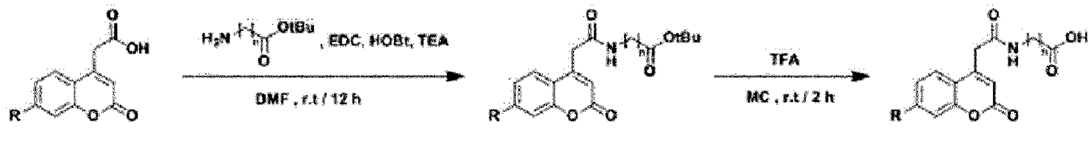


(31) (32)

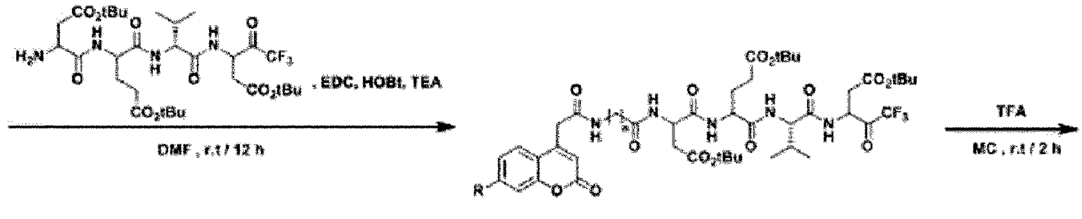


(33)

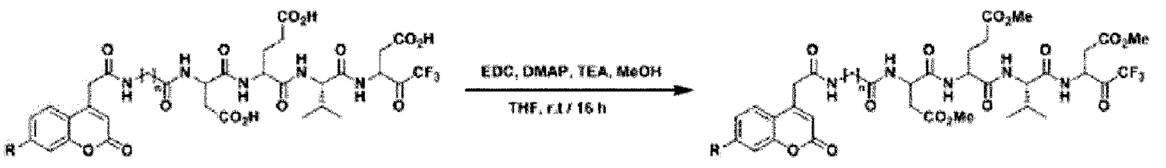


(XXIX) (XXX)

**Synthesis of tert-butyl 3-(N-(7-hydroxycoumarin-4-acetyl))propionate, tert-butyl 3-(N-(7-dimethylaminocoumarin-4-acetyl))propionate (31)**

7-R-coumarin-4-acetic acid (R = dimethylamino, hydroxy, 1.1 eq), Intermediate 30 (0.1g) and TEA (5 eq) were dissolved in DMF, and then stirred at 0 °C for 15 minutes. EDC (1.5 eq) and HOBt (1.5 eq) were added thereto, and the mixture was further stirred at room temperature for 12 hours. The solution was concentrated under reduced pressure, and the residue was diluted with ethyl acetate and washed with 10% citric acid aqueous solution and saturated sodium bicarbonate aqueous solution, respectively. The organic layer was further washed with the saturated sodium chloride solution, and then dried over anhydrous magnesium sulfate (anhydrous $MgSO_4$). The solution was concentrated under reduced pressure and the residue was purified by silica gel chromatography (methanol:dichloromethane=1:10) to prepare Intermediate 31 (R = -OH, n = 2, 0.14 g, yield:61 %) (R = -OH, n = 5, 0.15 g, yield: 74%), (R = $-NMe_2$, n = 2, 0.10 g, yield:39 %) (R = $-NMe_2$, n = 5, 0.11 g, yield: 43%). The structure of the product was identified by $^1$H-NMR.
$^1$H-NMR (600 MHz, DMSO-d6, R=-OH, n=2): 8.68(d,1H), 7.63(d,1H), 6.81(d,1H), 6.75(s,1H), 6.39(s,1H), 3.79(t,2H), 3.73(s,2H), 2.65(t,2H), 1.42(s,9H).
$^1$H-NMR (600 MHz, DMSO-d6, R=-OH, n=5): 8.66(s,1H), 7.61(d,1H), 6.76(d,1H), 6.74(s,1H), 6.24(s,1H), 3.70(s,2H), 3.19(q,2H), 2.20(t,2H), 1.65-1.30(m,6H), 1.43(s,9H).
$^1$H-NMR (600 MHz, DMSO-d6, R=$-NMe_2$, n=2): 8.01(s,1H), 7.48(d,1H), 6.63(d,1H), 6.61(d,1H), 6.51(s,1H), 3.77(t,2H), 3.61(s,2H), 3.05(s,6H), 2.62(t,2H), 1.42(s,9H).
$^1$H-NMR (600 MHz, DMSO-d6, R=$-NMe_2$, n=5): 8.03(s,1H), 7.45(d,1H), 6.62(d,1H), 6.60(d,1H), 6.49(s,1H), 3.71(s,2H), 3.23(q,2H), 3.19(s,6H), 2.24(t,2H), 1.66-1.34(m,6H), 1.42(s,9H).

**Synthesis of 3-(N-(7-hydroxycoumarin-4-acetyl))propionamide (32)**

Compound 31 (0.1 g) was dissolved in MC:TFA (2:1, 1 ml), and stirred at room temperature for 2 hours. The reactant was dissolved in methylene chloride, and then distilled under reduced pressure. This process was repeated twice, and then the residue was recrystallized in combination of methanol and diethylether. The desired crystalline product thus formed was dried under vacuum to prepare Compound 32 (R = -OH, n = 2, 0.060 g, yield: 78%) (R = -OH, n = 5, 0.080 g, yield: 84%), (R = $-NMe_2$, n = 2, 0.051 g, yield:67 %) (R = $-NMe_2$, n = 5, 0.081 g, yield: 88%). The structure of the product was identified by $^1$H-NMR.
$^1$H-NMR (600 MHz, DMSO-d6, R=-OH, n=2): 8.68(d,1H), 7.63(d,1H), 6.81(d,1H), 6.75(s,1H), 6.39(s,1H), 3.73(s,2H), 3.79(t,2H), 2.65(t,2H), 1.42(s,9H).
$^1$H-NMR (600 MHz, DMSO-d6, R=-OH, n=5): 8.66(s,1H), 7.61(d,1H), 6.76(d,1H), 6.74(s,1H), 6.24(s,1H), 3.70(s,2H), 3.19(q,2H), 2.20(t,2H), 1.65-1.30(m,6H).
$^1$H-NMR (600 MHz, DMSO-d6, R=$-NMe_2$, n=2): 8.22(s,1H), 7.53(d,1H), 6.74(d,1H), 6.56(s,1H), 6.43(s,1H), 3.75(t,2H), 3.61(s,2H), 3.05(s,6H), 2.62(t,2H).
$^1$H-NMR (600 MHz, DMSO-d6, R=$-NMe_2$, n=5): 8.20(s,1H), 7.47(d,1H), 6.64(d,1H), 6.60(d,1H), 6.49(s,1H), 3.71(s,2H), 3.23(q,2H), 3.19(s,6H), 2.24(t,2H), 1.68-1.32(m,6H).

**Synthesis of 3-(N-(7-hydroxycoumarin-4-acetyl))propionamide-Asp ($CO_2$tBu)-L-Glu ($CO_2$tBu)-L-Val-Asp($CO_2$tBu)-(ketone)-$CF_3$ (33)**

Compound 32 (1.2 eq), Compound 27 (0.03 g) and TEA (5 eq) were dissolved in DMF and stirred at 0°C for 15 minutes. Then, EDC (1.5 eq) and HOBt (1.5 eq) were added and further stirred at room temperature for 12 hours. The solution was concentrated under reduced pressure, and then the residue was diluted with ethyl acetate, and washed with 10% citric acid aqueous solution and the saturated sodium bicarbonate aqueous solution, respectively. The organic layer was further washed with the saturated sodium chloride solution, and then dried over anhydrous magnesium sulfate. The solution was concentrated under reduced pressure and then the residue was purified by silica gel chromatography(methanol:dichloromethane=1:10) to prepare Compound 33 (R = -OH, n = 2, 0.013 g, yield: 32%) (R = -OH, n = 5, 0.017 g, yield: 40%), (R = $-NMe_2$, n = 2, 0.016 g, yield:39 %) (R = $-NMe_2$, n = 5, 0.013 g, yield:30 %). The product was identified by mass spectrometric analysis.
LC/MS (R = -OH, n = 2): 970.4(M+H).
LC/MS (R = -OH, n = 5): 1012.5(M+H).
LC/MS(R = -NMe2, n = 2): 997.5(M+H).
LC/MS(R = -NMe2, n = 5): 1039.5(M+H).

**Synthesis of 3-(N-(7-hydroxycoumarin-4-acetyl))propionamide-Asp-L-Glu-L-Val-Asp-(ketone)-CF3 (XXIX)**

Compound 33 was dissolved in MC:TFA (2:1, 1 ml) and stirred at room temperature for 2 hours. The reactant was further dissolved in methylene chloride, and distilled under vacuum. This process was repeated twice, and then the residue was recrystallized in combination of methanol and diethylether. The desired crystalline product thus formed was dried under vacuum to prepare Compound XXIX.

**Synthesis of 3-(N-(7-hydroxycoumarin-4-acetyl))propionamide-Asp($CO_2$Me)-L-Glu($CO_2$Me)-L-Val-Asp($CO_2$Me)-(ketone)-$CF_3$ (XXX)**

Compound XXIX, MeOH, EDC and DMAP were dissolved in a proper amount of THF, and then stirred at 0°C for 15 minutes. TEA was slowly added dropwise to the reactant, and then stirred at room temperature for 16 hours. The solution was concentrated under reduced pressure, and then the residue was diluted with ethyl acetate and washed with 10% citric acid aqueous solution and the sodium bicarbonate aqueous solution, respectively. The organic layer was further washed with the saturated sodium chloride solution, and then dried over anhydrous magnesium sulfate. The solution was concentrated under reduced pressure, and the residue was purified by silica gel chromatography to prepare Compound XXX.

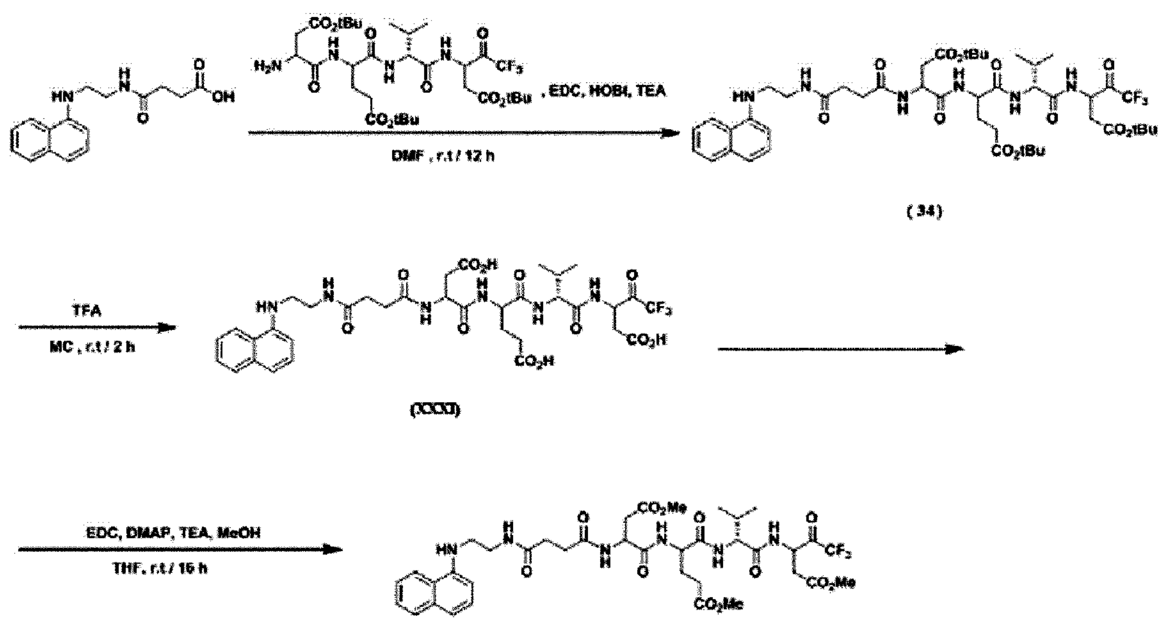


(34)

(XXXI)

( XXXII )

**Synthesis of N-(1-naphthyl)ethyleneamide-4-butanamide-Asp ($CO_2$tBu)-L-Glu ($CO_2$tBu)-L-Val-Asp ($CO_2$tBu)-(ketone)-$CF_3$ (34)**

N-(1-naphthyl)ethyleneamide-4-butanoic acid (1.1 eq)), Compound 27 (1 eq) and TEA (5 eq) were dissolved in DMF and stirred at 0°C for 15 minutes. EDC (1.5 eq) and HOBt (1.5 eq) were added and stirred at room temperature for 12 hours. The solution was concentrated under reduced pressure and then the residue was diluted with ethyl acetate and washed with 10% citric acid aqueous solution and the saturated sodium bicarbonate aqueous solution, respectively. The organic layer was further washed with the saturated sodium chloride solution, and then dried over anhydrous magnesium sulfate. The solution was concentrated under reduced pressure and the residue was purified by silica gel chromatography to prepare Compound 34.

**Synthesis of N-(1-naphthyl)ethyleneamide-4-butaneamide-Asp-L-Glu-L-Val-Asp-(ketone)-CF3 (XXXI)**

Compound 34 was dissolved in MC:TFA(2:1), and stirred at room temperature for 2 hours. The reactant was dissolved in methylene chloride, and distilled under reduced pressure. This process was repeated twice and the residue was recrystallized in combination of methanol and diethylether. The desired crystalline product thus formed was dried under vacuum to prepare Compound XXXI.

**Synthesis of N-(1-naphthyl)ethyleneamide-4-butaneamide-Asp ($CO_2$Me)-L-Glu ($CO_2$Me)-L-Val-Asp ($CO_2$Me)-(ketone)-$CF_3$ (XXXII)**

Compound XXXI (1 eq), MeOH (5 eq), EDC (1.2 eq) and DMAP (0.1 eq) were dissolved in a proper amount of THF and then stirred at 0°C for 15 minutes. TEA (3 eq) was slowly added dropwise to the reactant, and further stirred at room temperature for 16 hours. The solution was concentrated under reduced pressure, and then the residue was diluted with ethyl acetate and washed with 10% citric acid aqueous solution and the sodium bicarbonate aqueous solution, respectively. The organic layer was further washed with the saturated sodium chloride solution, and dried over anhydrous magnesium sulfate. The solution was concentrated under reduced pressure, and then the residue was purified by silica gel chromatography to prepare Compound XXXII.

For real-time imaging of cell apoptosis that is induced by particular intracellular signal transduction, cell permeable caspase iFRET probe was synthesized. The widely known peptide inhibitor (27) selectively binding to caspase was synthesized using Compound (21). The iFRET fluorescent nucleus was introduced into the N-terminal region of (27). In order to introduce the compound serving as a fluorescent material into DEVD-CF3 which is a peptide inhibitor inhibiting caspase activity, Linker 20 (n = 2 to 5) was synthesized. The cell permeable trifluoromethyl aspartic acid (21) has a -$CF_3$ group at its end, which binds to the target protein caspase-3 to inhibit enzymatic function. To prepare the fluorescent probe for emitting various fluorescence wavelengths, two groups of (-OH) and (-$N(CH_3)_2$) were introduced

as R groups of the fluorescent probe, and a fluorescent probe having Chemical Formula XXXII emitting fluorescence at 430 nm was also synthesized.

**Effect of the invention**

The probe for iFRET according to the present invention utilizes an amino acid in a protein as a fluorescent donor, unlike the conventional FRET method. Therefore, an artificial label (fluorescent protein, etc.) for the target protein is not required, only one fluorescent material can be used, and its emission wavelength is distinct from the intrinsic fluorescence of the protein. Thus, high specificity and sensitivity are ensured, and the quantity, activity and mechanism of various proteins can be analyzed in an easy and accurate manner. Further, the probe can be used for the development of diagnostic and therapeutic agents for target protein-associated diseases.

**Claims**

1. A method for searching for a target protein-specific binding site or a molecule having the binding site, comprising:

   the first step of preparing a probe for intrinsic fluorescence resonance energy transfer (iFRET) by selecting the target protein-specific binding site or the molecule having the binding site from various candidate groups and linking the same with a fluorescent molecule acting as an acceptor for protein intrinsic fluorescence through a direct bond or a linker;
   the second step of treating the target protein with the probe for iFRET prepared in the first step; and
   the third step of irradiating light comprising a wavelength for excitation of the amino acids in the target protein and detecting the light-emitting probe for iFRET.

2. The method according to claim 1, wherein the target protein-specific binding site or the molecule having the binding site, which has been identified by the above of searching, is used as a substance for preventing, ameliorating, or treating the target protein-associated diseases or as a part thereof.

3. The method according to claim 1, wherein the amino acid exhibiting the intrinsic fluorescence of the protein is tryptophan, tyrosine, phenylalanine or a combination thereof.

4. The method according to claim 1, wherein the second step is performed in a solvent selected from the group consisting of water, buffer solution, lower alcohol having 1 to 6 carbon atoms and mixtures thereof; on a biochip, microparticles, or nanoparticles; or in cells or tissues.

5. The method according to claim 1, wherein the target protein is excited by light at a wavelength of 260 to 300 nm and emits light at a wavelength of 290 to 400 nm, and
   the probe for iFRET is selectively excited by light at a wavelength of 300 to 400 nm.

6. The method according to claim 1, wherein the molecule acting as an acceptor for the intrinsic fluorescence of the protein is selected from the group consisting of the compounds represented by the following Chemical Formulae I to XXII:

[Chemical Formula I]

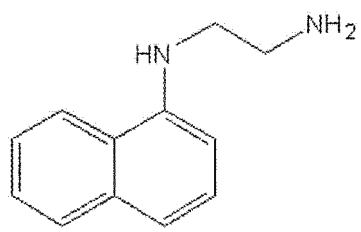

[Chemical Formula II]

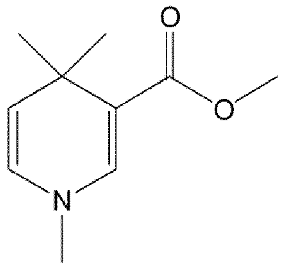

[Chemical Formula III]

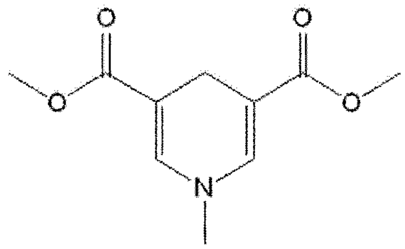

[Chemical Formula IV]

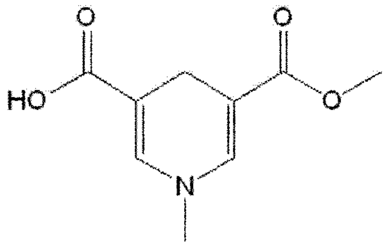

[Chemical Formula V]

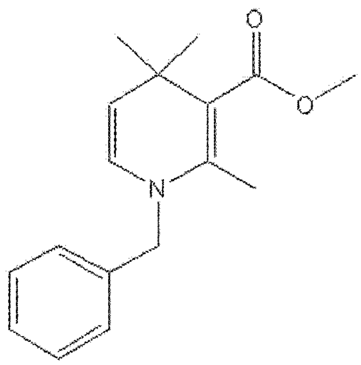

[Chemical Formula VI]

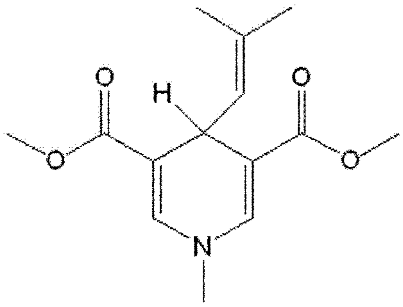

[Chemical Formula VII]

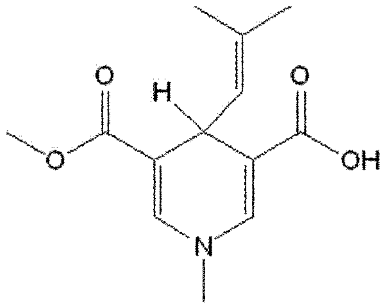

[Chemical Formula VIII]

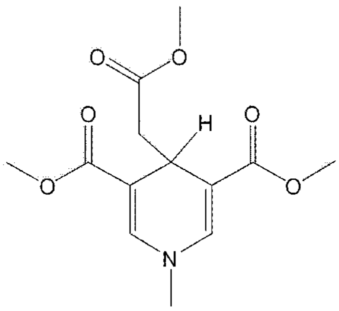

[Chemical Formula IX]

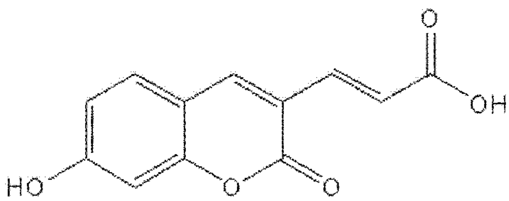

[Chemical Formula X]

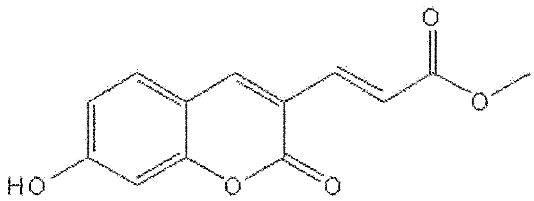

[Chemical Formula XI]

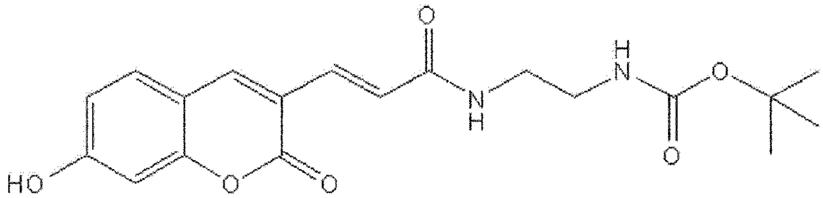

[Chemical Formula XII]

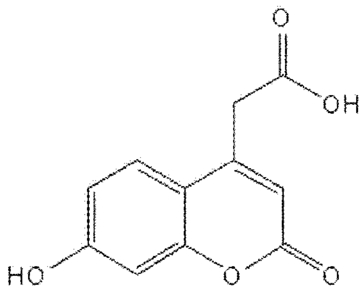

[Chemical Formula XIII]

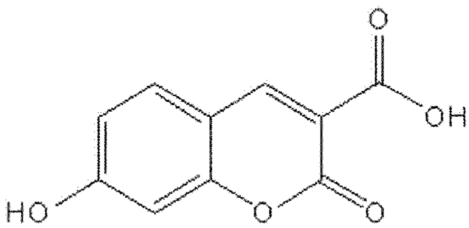

[Chemical Formula XIV]

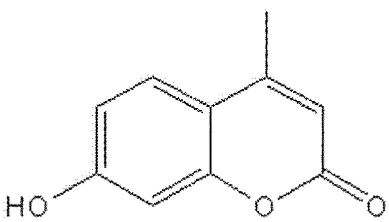

[Chemical Formula XV]

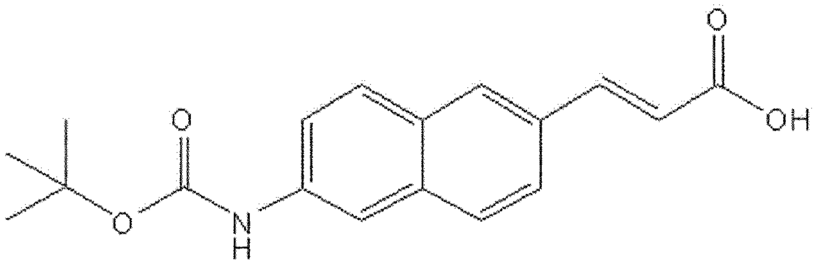

[Chemical Formula XVI]

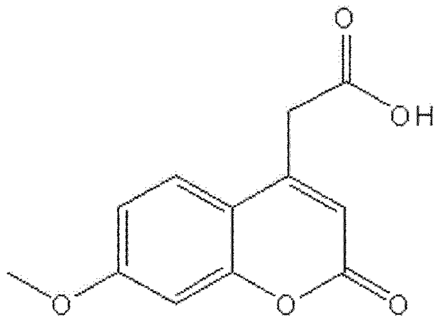

[Chemical Formula XVII]

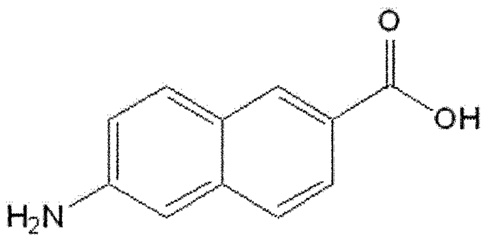

[Chemical Formula XVIII]

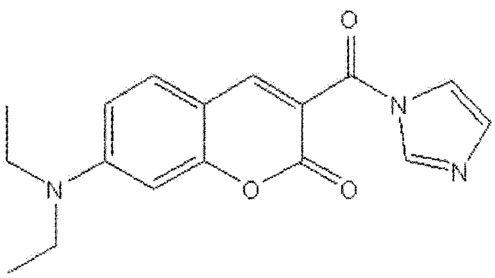

[Chemical Formula XIX]

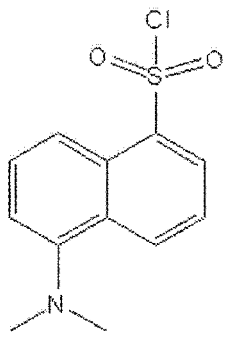

[Chemical Formula XX]

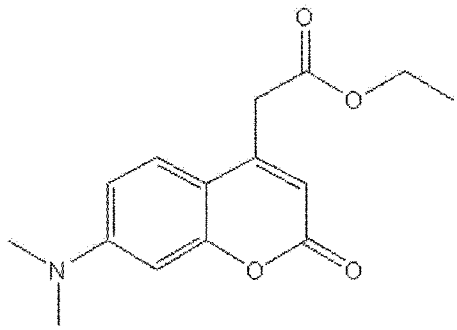

[Chemical Formula XXI]

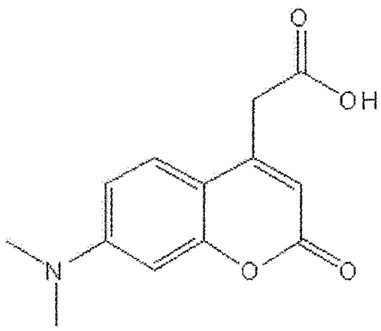

[Chemical Formula XXII]

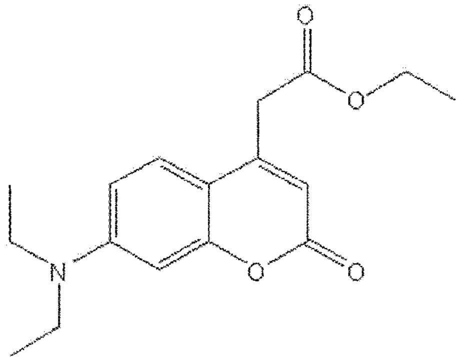

7. A method for preparing a probe for iFRET, comprising:

the first step of preparing a first probe for iFRET by obtaining the target protein-specific binding site or the molecule having the binding site from various candidate groups and linking the same with a first fluorescent molecule acting as an acceptor for protein intrinsic fluorescence through a direct bond or a linker;
the second step of treating the target protein with the first probe for iFRET;
the third step of irradiating light comprising a wavelength for excitation of the amino acids in the target protein and detecting the light-emitting probe for iFRET; and
the fourth step of preparing a second probe for iFRET by linking the target protein-specific binding site or the molecule having the binding site, composing the first probe for iFRET light-emitting from the third step, with a second fluorescent molecule acting as an acceptor for protein intrinsic fluorescence through a direct bond or a linker.

8. The method according to claim 7, wherein the first fluorescent molecule and the second fluorescent molecule are the same as or different from each other.

9. The method according to claim 7, wherein the amino acid exhibiting the intrinsic fluorescence of the protein is tryptophan, tyrosine, phenylalanine or a combination thereof.

10. The method according to claim 7, wherein the third step is performed in a solvent selected from the group consisting of water, buffer solution, lower alcohol having 1 to 6 carbon atoms and mixtures thereof; on a biochip, microparticles, or nanoparticles; or in cells or tissues.

11. The method according to claim 7, wherein the second fluorescent molecule acting as an acceptor for the intrinsic fluorescence of the protein is excited by itself or by the emitted light from the excited amino acids in the target protein in the second probe for iFRET, and emits light at a 450nm or longer wavelength.

12. The method according to claim 7, wherein the target protein is excited by light at a wavelength of 260 to 300 nm and emits light at a wavelength of 290 to 400 nm, and the first probe for iFRET is selectively excited by light at a wavelength of 300 to 400 nm.

13. The method according to claim 7, wherein the first fluorescent molecule, the second fluorescent molecule acting as an acceptor for the intrinsic fluorescence of the protein, or both of them, are selected from the group consisting of the compounds represented by the following Chemical Formulae I to XXII:

[Chemical Formula I]

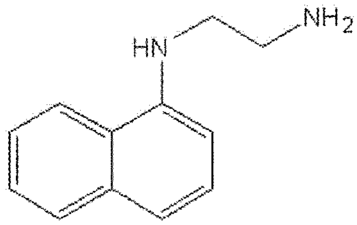

[Chemical Formula II]

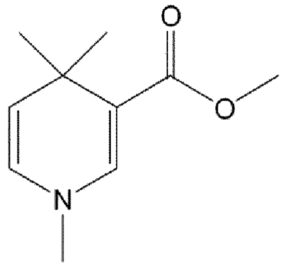

[Chemical Formula III]

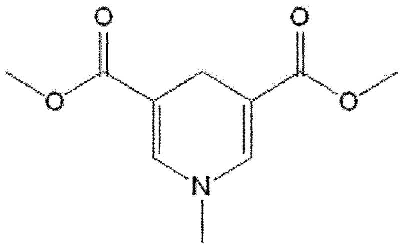

[Chemical Formula IV]

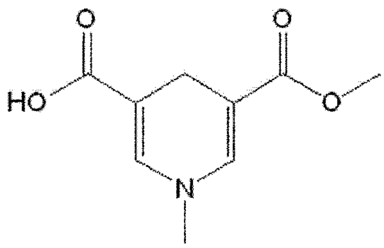

[Chemical Formula V]

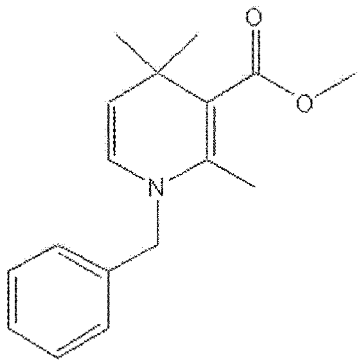

[Chemical Formula VI]

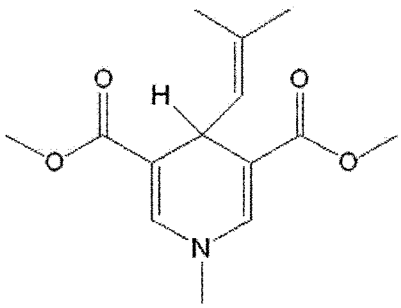

[Chemical Formula VII]

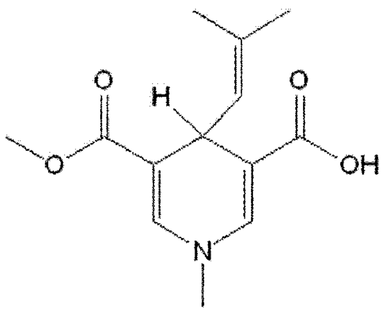

[Chemical Formula VIII]

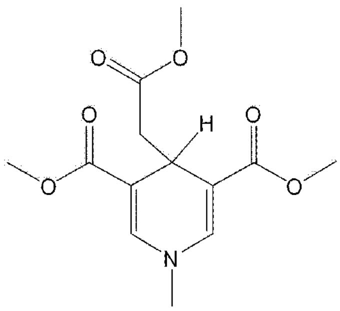

[Chemical Formula IX]

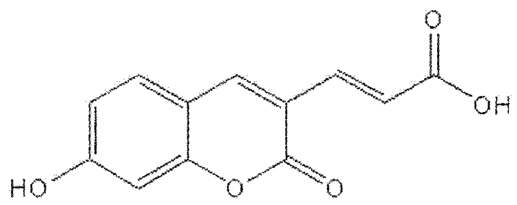

[Chemical Formula X]

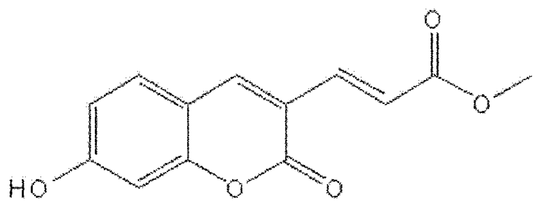

[Chemical Formula XI]

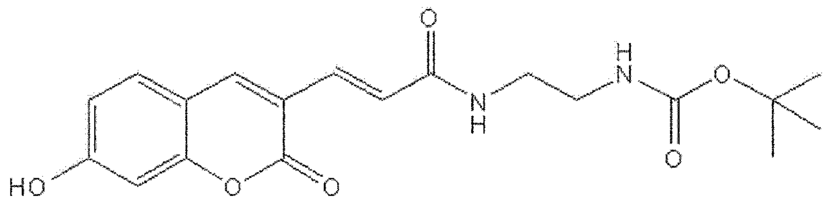

[Chemical Formula XII]

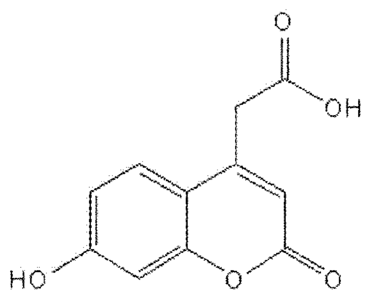

[Chemical Formula XIII]

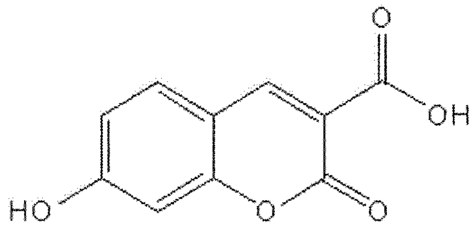

[Chemical Formula XIV]

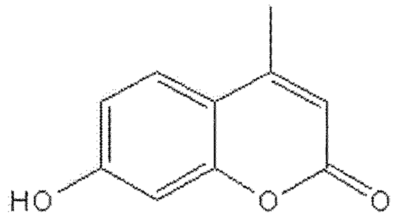

[Chemical Formula XV]

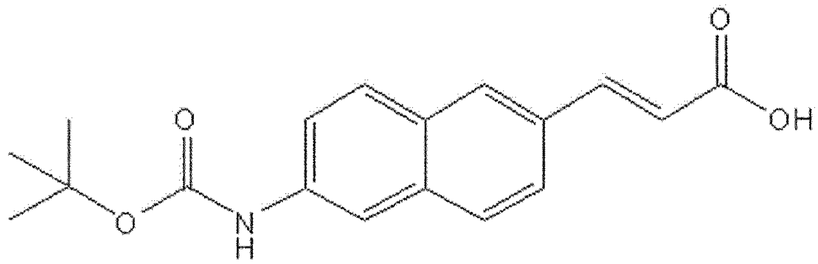

[Chemical Formula XVI]

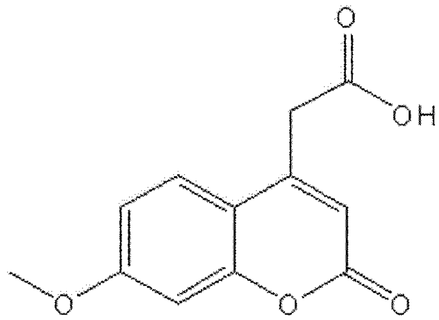

[Chemical Formula XVII]

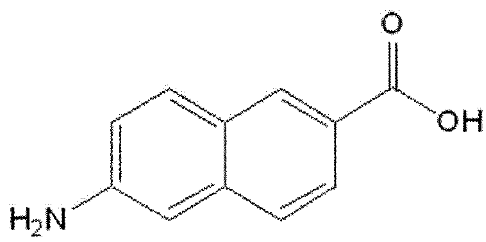

[Chemical Formula XVIII]

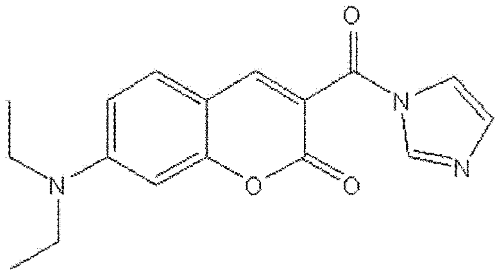

[Chemical Formula XIX]

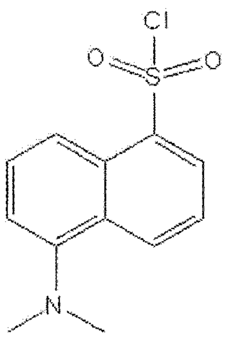

[Chemical Formula XX]

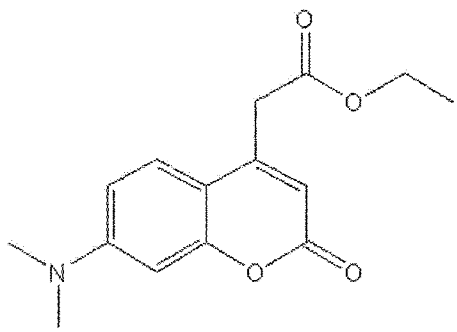

[Chemical Formula XXI]

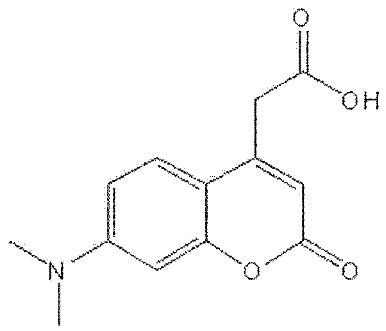

[Chemical Formula XXII]

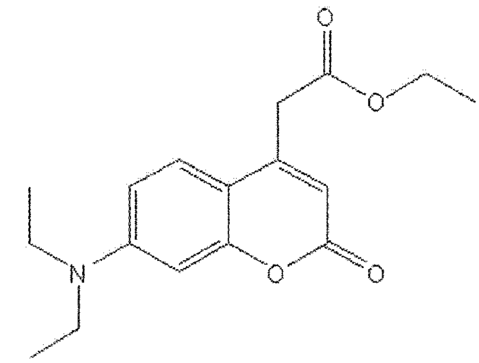

**14.** A probe for iFRET that is prepared by linking a target protein-specific binding site or a molecule having the binding site with a fluorescent molecule acting as an iFRET acceptor selected from the group consisting of the compounds represented by the following Chemical Formulae I to XXII, through a direct bond or a linker:

[Chemical Formula I]

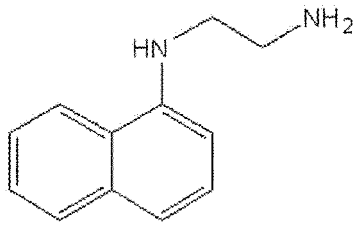

[Chemical Formula II]

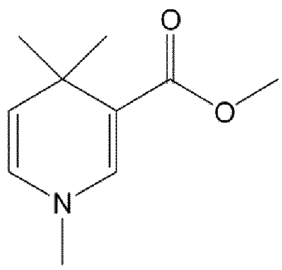

[Chemical Formula III]

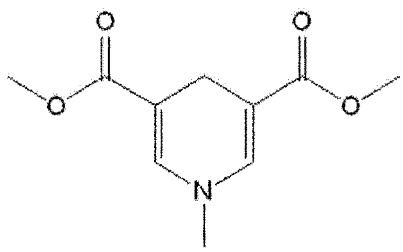

[Chemical Formula IV]

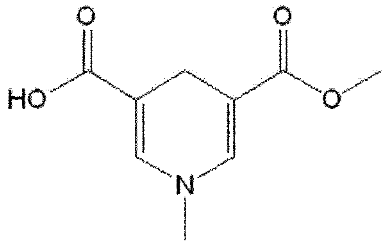

[Chemical Formula V]

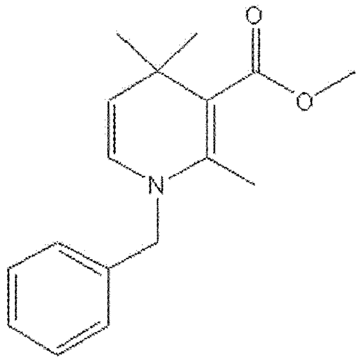

[Chemical Formula VI]

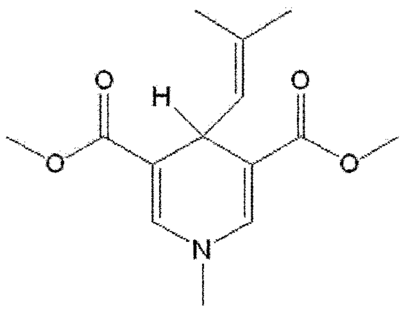

[Chemical Formula VII]

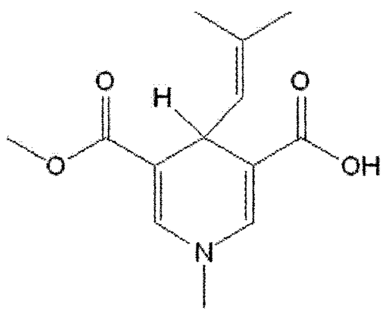

[Chemical Formula VIII]

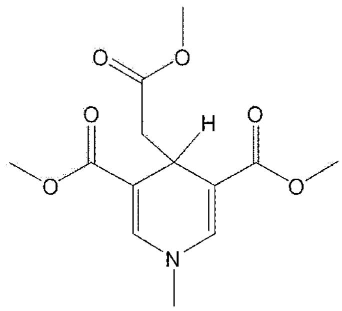

[Chemical Formula IX]

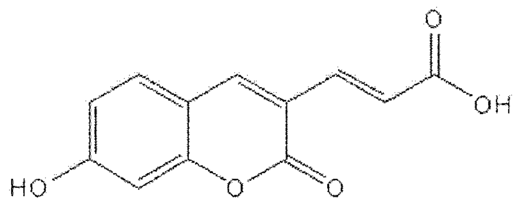

[Chemical Formula X]

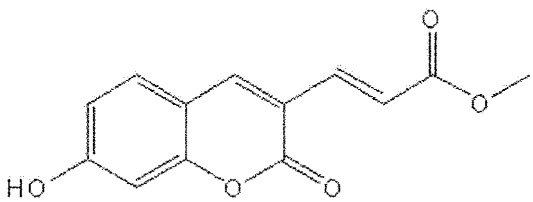

[Chemical Formula XI]

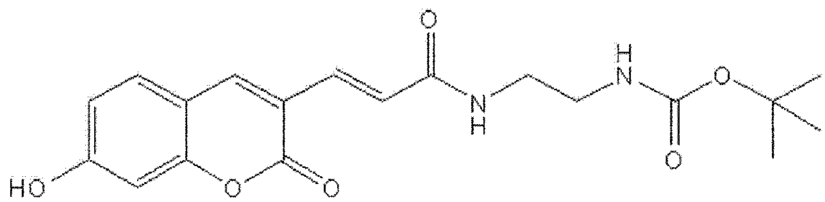

[Chemical Formula XII]

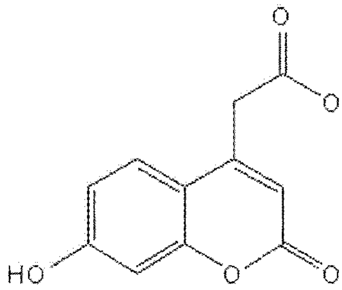

[Chemical Formula XIII]

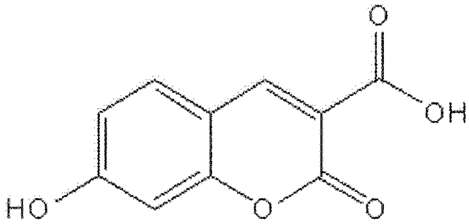

[Chemical Formula XIV]

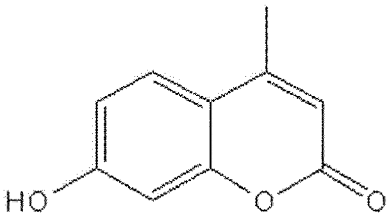

[Chemical Formula XV]

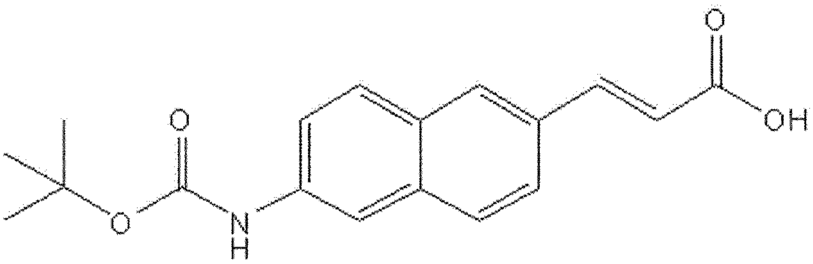

[Chemical Formula XVI]

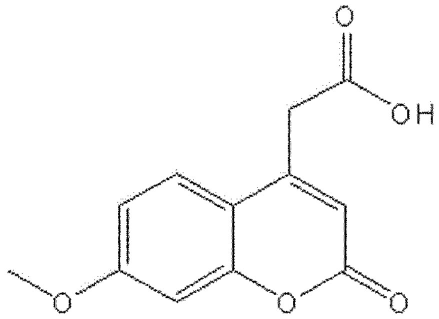

[Chemical Formula XVII]

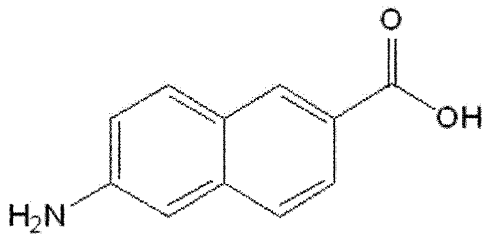

[Chemical Formula XVIII]

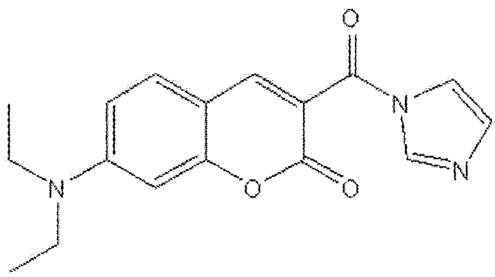

[Chemical Formula XIX]

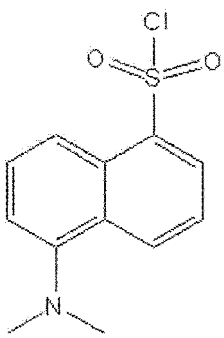

[Chemical Formula XX]

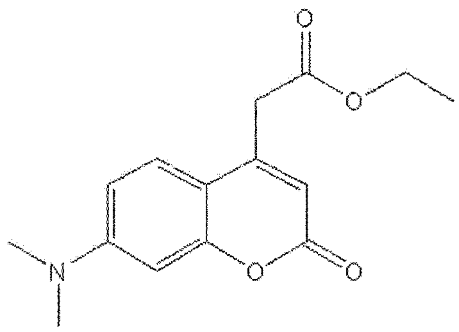

[Chemical Formula XXI]

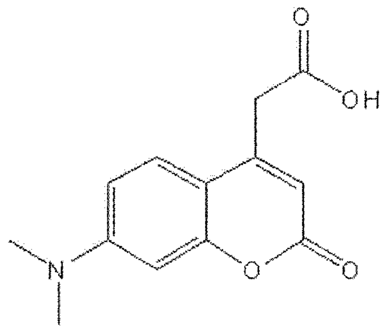

[Chemical Formula XXII]

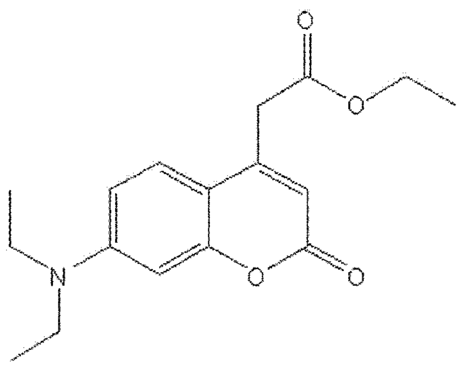

**15.** The probe for iFRET according to claim 14, wherein the probe emits light at 450 nm or longer wavelength.

**16.** The probe for iFRET according to claim 14, wherein the target protein is caspase-3, HSP90 (heat shock protein 90) or streptavidin.

**17.** The probe for iFRET according to claim 14, wherein the probe is represented by Chemical Formula selected from the group consisting of the following Chemical Formulae XXIII, XXIV, or XXVI to XXXII:

[Chemical Formula XXIII]

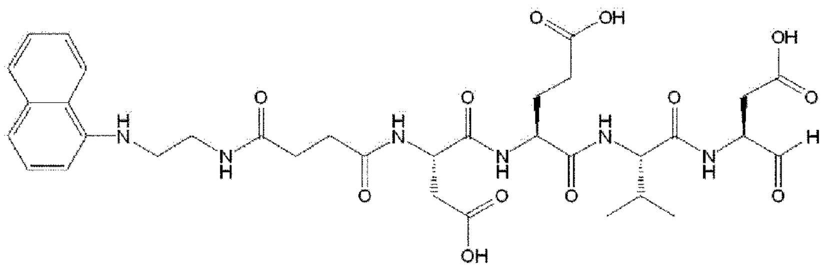

[Chemical Formula XXIV]

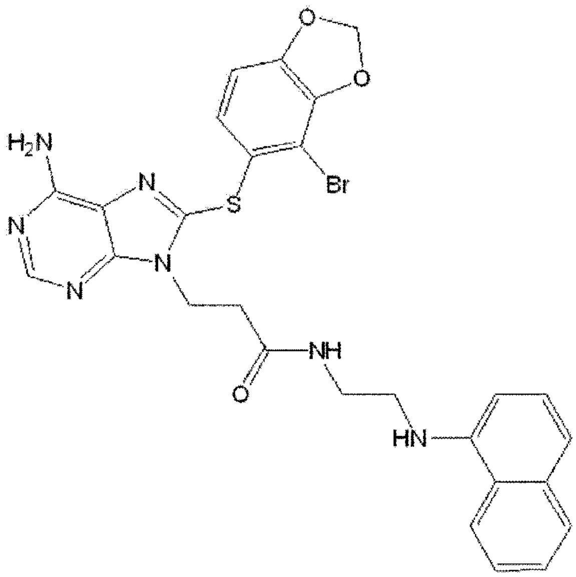

[Chemical Formula XXVI]

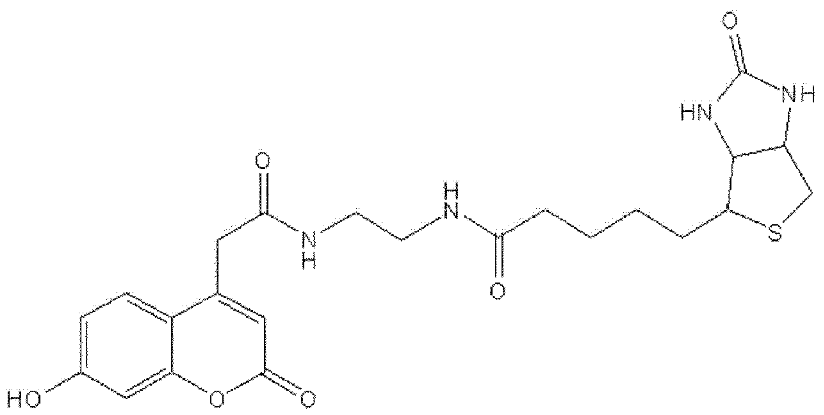

[Chemical Formula XXVII]

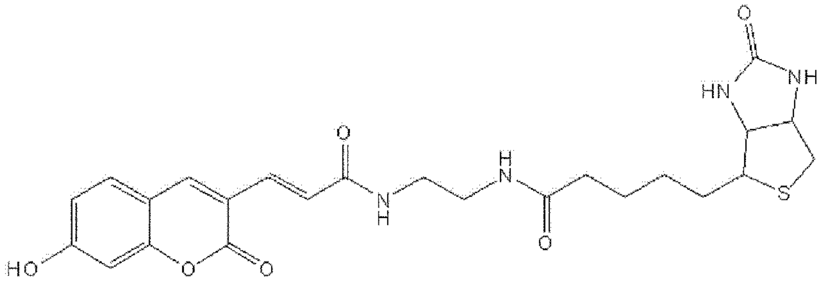

[Chemical Formula XXVIII]

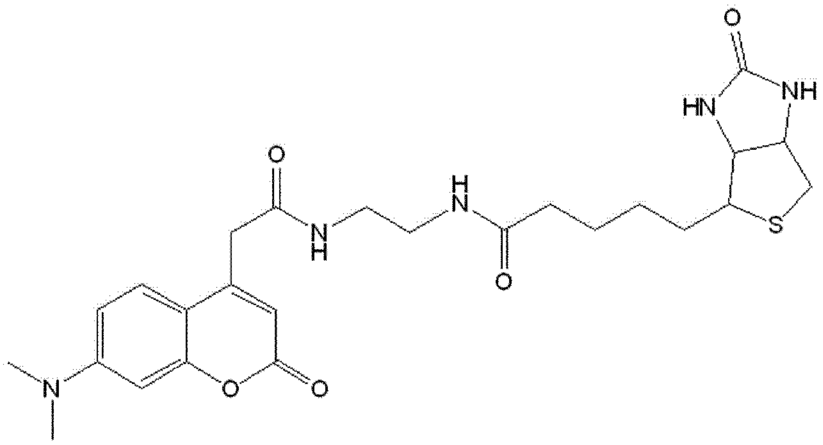

[Chemical Formula XXIX]

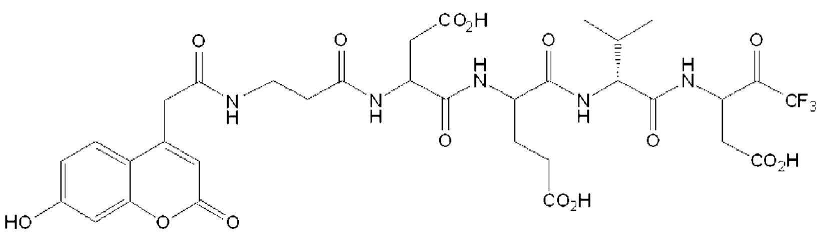

[Chemical Formula XXX]

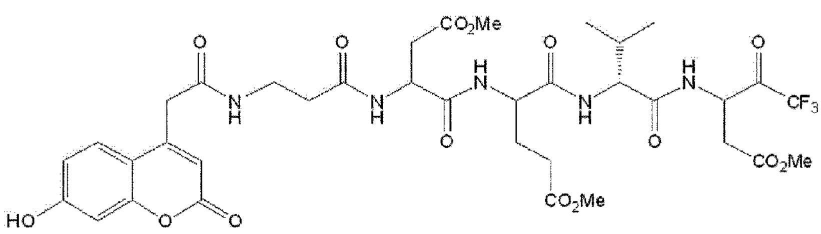

[Chemical Formula XXXI]

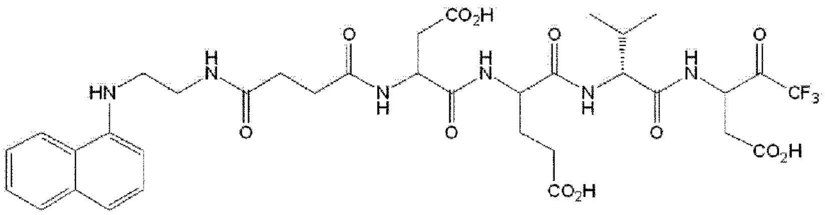

[Chemical Formula XXXII]

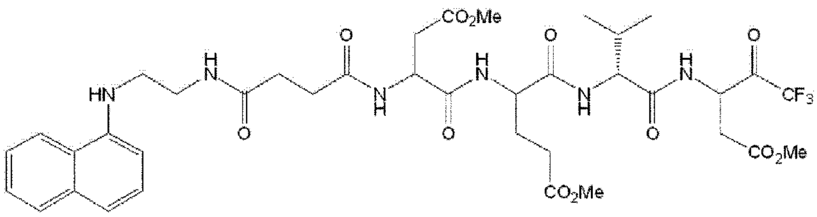

**18.** A probe for iFRET that is prepared by linking a target protein-specific binding site or a molecule having the binding site with a fluorescent molecule acting as an iFRET acceptor through a direct bond or a linker, wherein $R_0$ value, a distance between fluorescence donor and fluorescence acceptor at which the FRET efficiency is 50%, ranges from 1 to 4 nm.

**19.** A method for detecting or imaging a target protein by iFRET, comprising:

the first step of adding the second probe for iFRET prepared by the method of any one of claims 7 to 13 or the probe for iFRET of any one of claims 14 to 18 to a sample containing the target protein; and
the second step of irradiating light comprising a wavelength for excitation of the amino acids in the target protein and detecting or tracking the emitted light from the probe for iFRET.

**20.** The method according to claim 19, wherein the method is used for measuring the quantity of the target protein or activity of the target protein, or for tracking the action mechanism or migration route of the target protein.

**21.** The method according to claim 19, further comprising the third step of adding a particular material in combination with the probe for iFRET to the sample, irradiating light comprising a wavelength for excitation of the amino acids in the target protein and detecting or tracking the emitted light from the probe for iFRET,
and, according to the presence of the particular material, screening the particular material having a predetermined function for the target protein or examining the function of the particular material for the target protein by irradiating light with a wavelength for excitation of the amino acids in the target protein and then examining changes in the emitted light from the probe for iFRET.

**22.** The method according to claim 21, wherein the particular material is a drug or a food additive for preventing, treating or ameliorating the target protein-associated diseases.

**23.** The method according to claim 19, wherein the sample is selected from the group consisting of a target protein itself, cell, tissue, blood, feces, water, soil, air, food, waste product, animal/plant intestinal material, animal/plant tissue, and an organism itself.

**24.** The method according to claim 20, wherein the method is used as a method for examining the functions of the target protein, a method for detecting disease-associated target proteins in various specimens for disease diagnosis, or a method for detecting microorganisms or target proteins derived from microorganisms in food, feed, or drink.

**25.** The method according to claim 19, wherein in the second step, the light with 450 nm or longer wavelength among the emitted lights from the probe for iFRET is detected or tracked.

**26.** The method according to claim 19, wherein the method is performed by automated workstation.

**27.** A compound represented by the following Chemical Formula XXIII or a salt thereof:

[Chemical Formula XXIII]

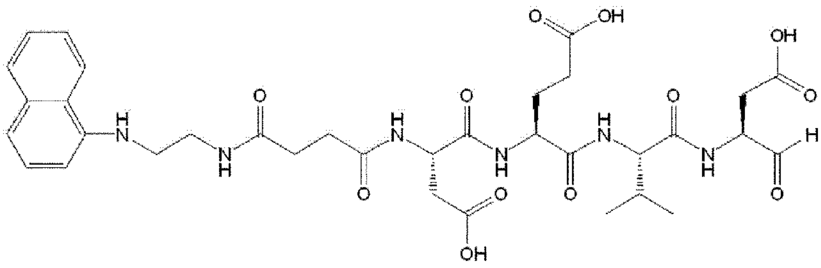

**28.** A compound represented by the following Chemical Formula XXIV or a salt thereof:

[Chemical Formula XXIV]

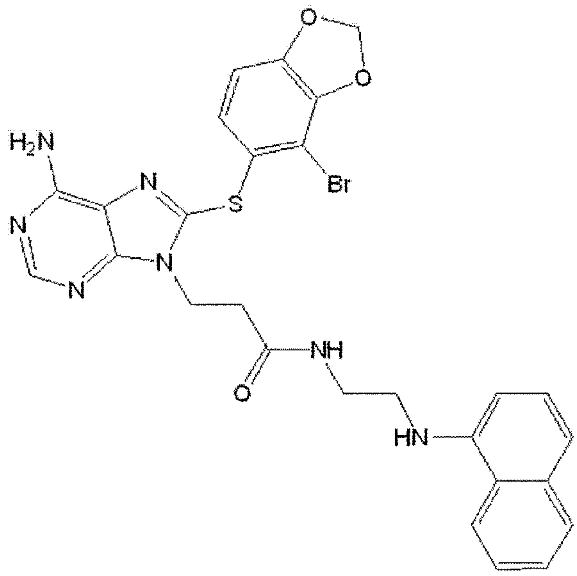

**29.** A compound represented by the following Chemical Formula XXVI or a salt thereof:

[Chemical Formula XXVI]

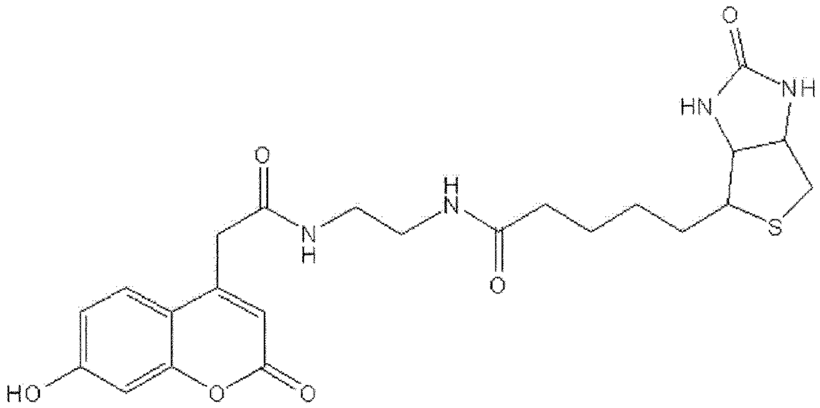

**30.** A compound represented by the following Chemical Formula XXVII or a salt thereof:

[Chemical Formula XXVII]

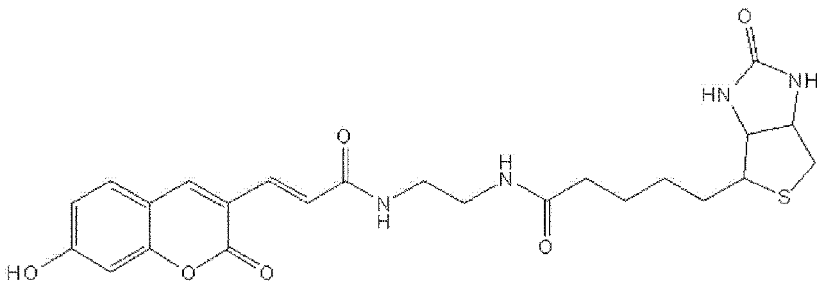

**31.** A compound represented by the following Chemical Formula XXVIII or a salt thereof:

[Chemical Formula XXVIII]

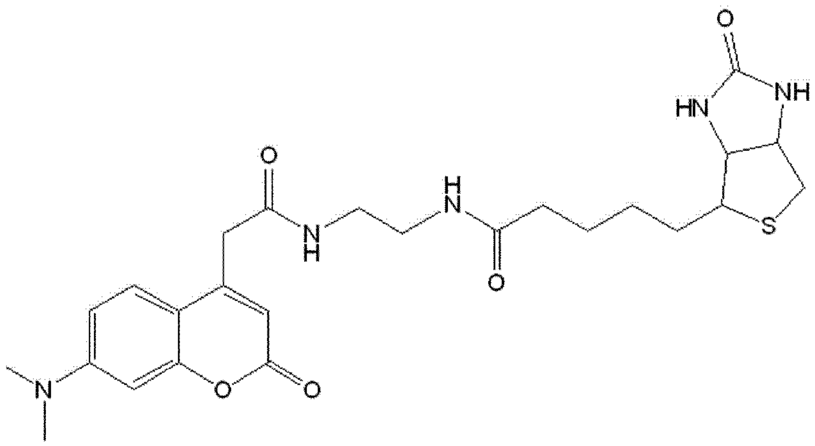

**32.** A compound represented by the following Chemical Formula XXIX or a salt thereof:

[Chemical Formula XXIX]

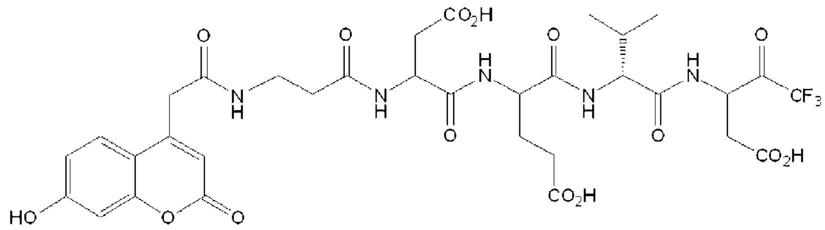

**33.** A compound represented by the following Chemical Formula XXX or a salt thereof:

[Chemical Formula XXX]

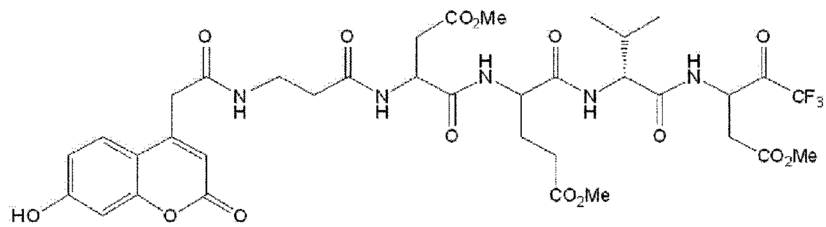

**34.** A compound represented by the following Chemical Formula XXXI or a salt thereof:

[Chemical Formula XXXI]

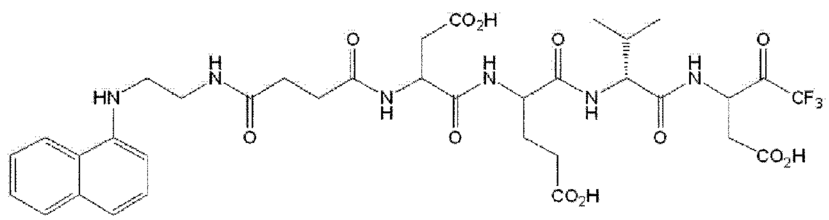

**35.** A compound represented by the following Chemical Formula XXXII or a salt thereof:

[Chemical Formula XXXII]

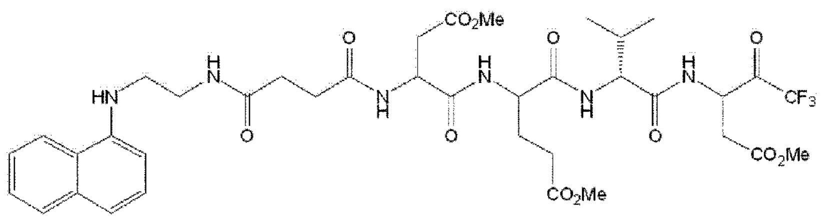

**36.** A method for detecting or imaging a target protein using a probe for intrinsic fluorescence resonance energy transfer (iFRET),
wherein the probe for iFRET is prepared by linking a target protein-specific binding site or a molecule having the binding site with a fluorescent molecule acting as an acceptor for the intrinsic fluorescence of the target protein through a direct bond or a linker, and
the method comprises the first step of introducing the probe for iFRET into a sample containing the target protein; the second step of alternately irradiating a first light with a wavelength for excitation of the amino acids in the target protein and a second light with a wavelength for excitation of the fluorescent molecule of the probe for iFRET onto

the sample prepared in the first step; and
the third step of calculating a third emission signal by ratiometric measurement of the first and second emission signals, by analyzing a first emission signal that is generated from the probe for iFRET by irradiating the first light with a wavelength for excitation of the amino acids in the target protein to the sample and a second emission signal that is generated from the probe for iFRET by irradiating the second light with a wavelength for excitation of the fluorescent molecule of the probe for iFRET.

**37.** The method according to claim 36, wherein the first emission signal, the second emission signal, and the third emission signal are two-dimensional images corresponding to each two-dimensional point of the sample.

**38.** The method according to claim 36, wherein the wavelength of the first light is in the range of 260 nm to 300 nm, and the wavelength of the second light is in the range of 300 nm to 400 nm.

**39.** The method according to claim 36, wherein the first light with a wavelength for excitation of the amino acids in the target protein and the second light with a wavelength for excitation of the fluorescent molecule of the probe for iFRET are alternately irradiated at a predetermined time interval, and
the third emission signals are calculated and accumulated by ratiometric measurement at a predetermined time interval from the first emission signals that are generated from the probe for iFRET by irradiating the first light with a wavelength for excitation of the amino acids in the target protein to the sample and the second emission signals that are generated from the probe for iFRET by irradiating the second light with a wavelength for excitation of the fluorescent molecule of the probe for iFRET,
thereby monitoring changes in the localization or quantity of the target protein or both of them over time.

**40.** The method according to claim 36, wherein changes of the target protein over time are given as moving images.

**41.** The method according to claim 36, wherein the method is used for measuring the quantity of the target protein or activity of the target protein, or for tracking the action mechanism or migration route of the target protein.

**42.** The method according to claim 36, further comprising the fourth step of adding a particular material in combination with the probe for iFRET to the sample, irradiating light with a wavelength for excitation of the amino acids in the target protein, and detecting or tracking the emitted light from the probe for iFRET,
and, according to the presence of the particular material, screening the particular material having a predetermined function for the target protein or examining the function of the particular material for the target protein by irradiating light with a wavelength for excitation of the amino acids in the target protein and then examining changes in the emitted light from the probe for iFRET.

**43.** The method according to claim 42, wherein the particular material is a drug or a food additive for preventing, treating or ameliorating the target protein-associated diseases.

**44.** The method according to claim 36, wherein the sample is selected from the group consisting of a target protein itself, cell, tissue, blood, feces, water, soil, air, food, waste product, animal/plant intestinal material, animal/plant tissue, and an organism itself.

**45.** The method according to claim 42, wherein the method is used as a method for examining the functions of the target protein, a method for detecting disease-associated target proteins in various specimens for disease diagnosis, or a method for detecting microorganisms or target proteins derived from microorganisms in food, feed, or drink.

**46.** The method according to claim 42, wherein the method is performed by an automated workstation.

[FIG. 1]

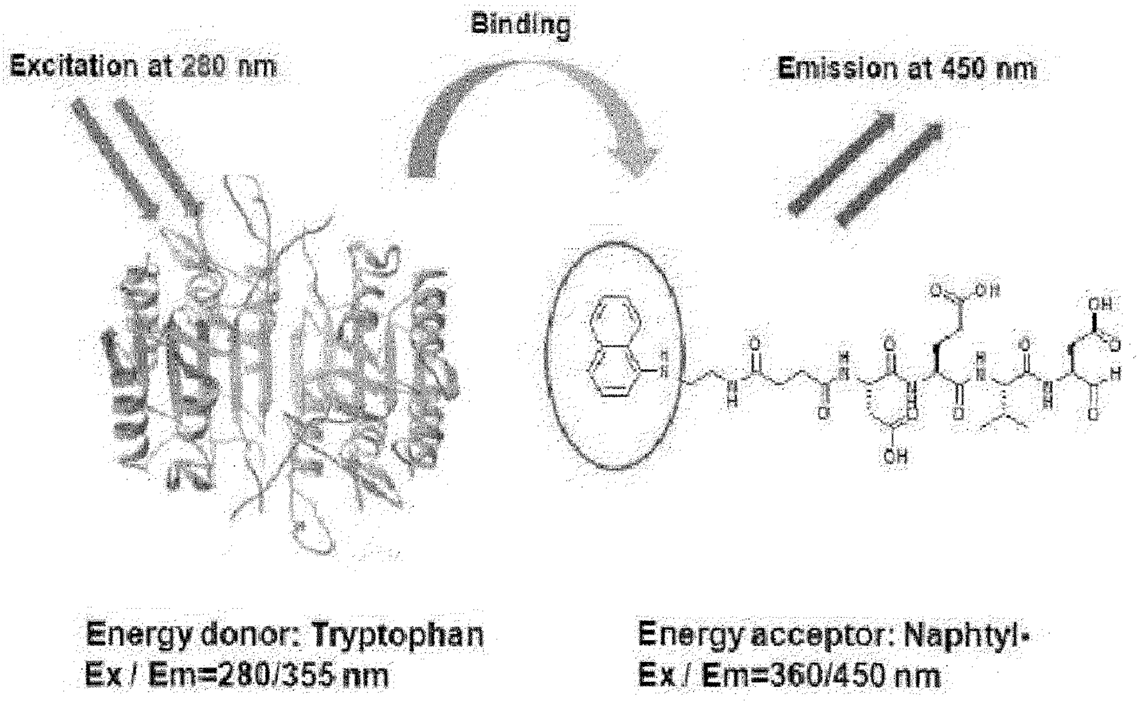
Binding
Excitation at 280 nm
Emission at 450 nm
Energy donor: Tryptophan
Ex / Em=280/355 nm
Energy acceptor: Naphtyl-
Ex / Em=360/450 nm

[FIG. 2]

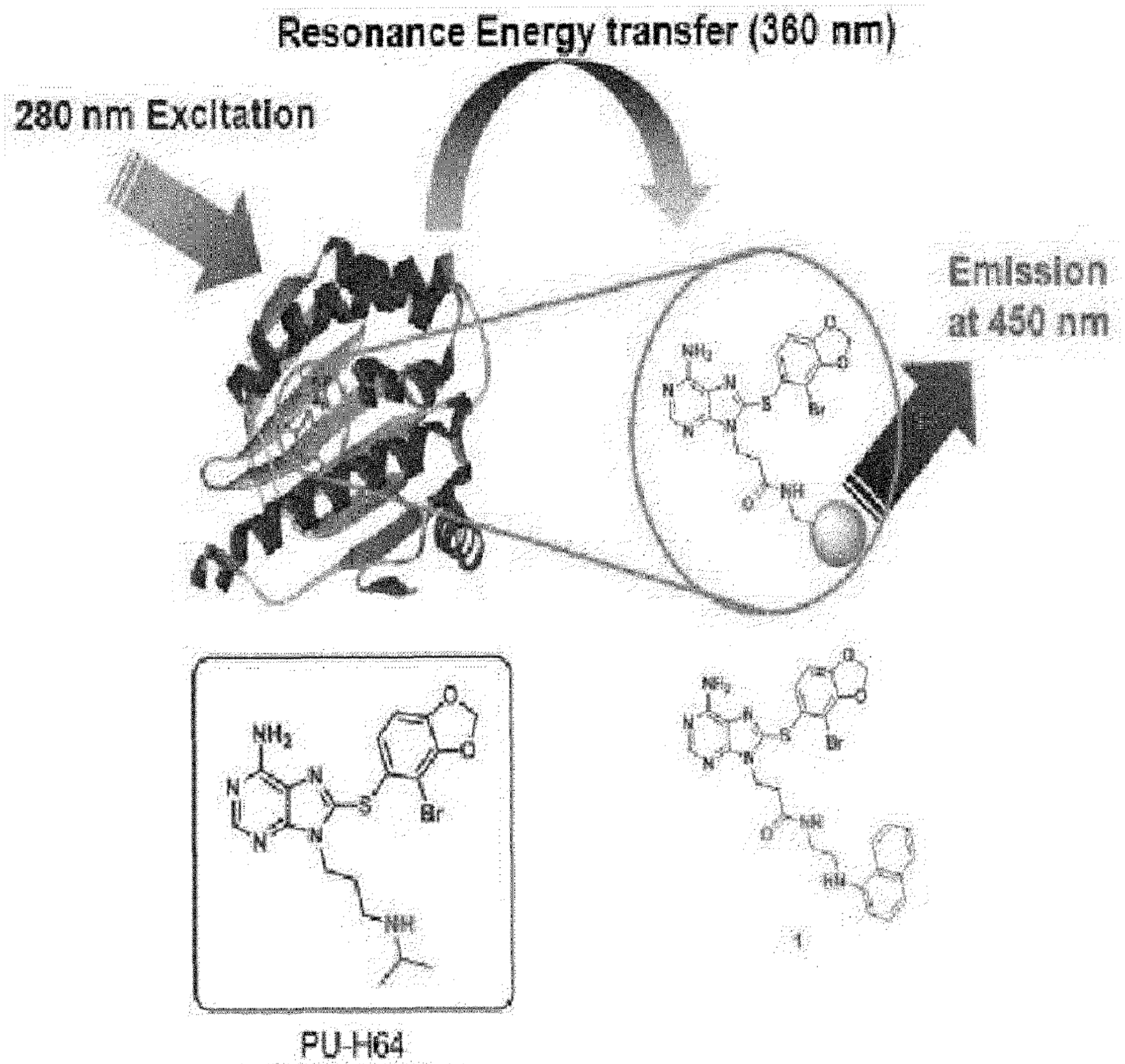


PU-H64
(HSP90 Inhibitor)

[FIG. 3]

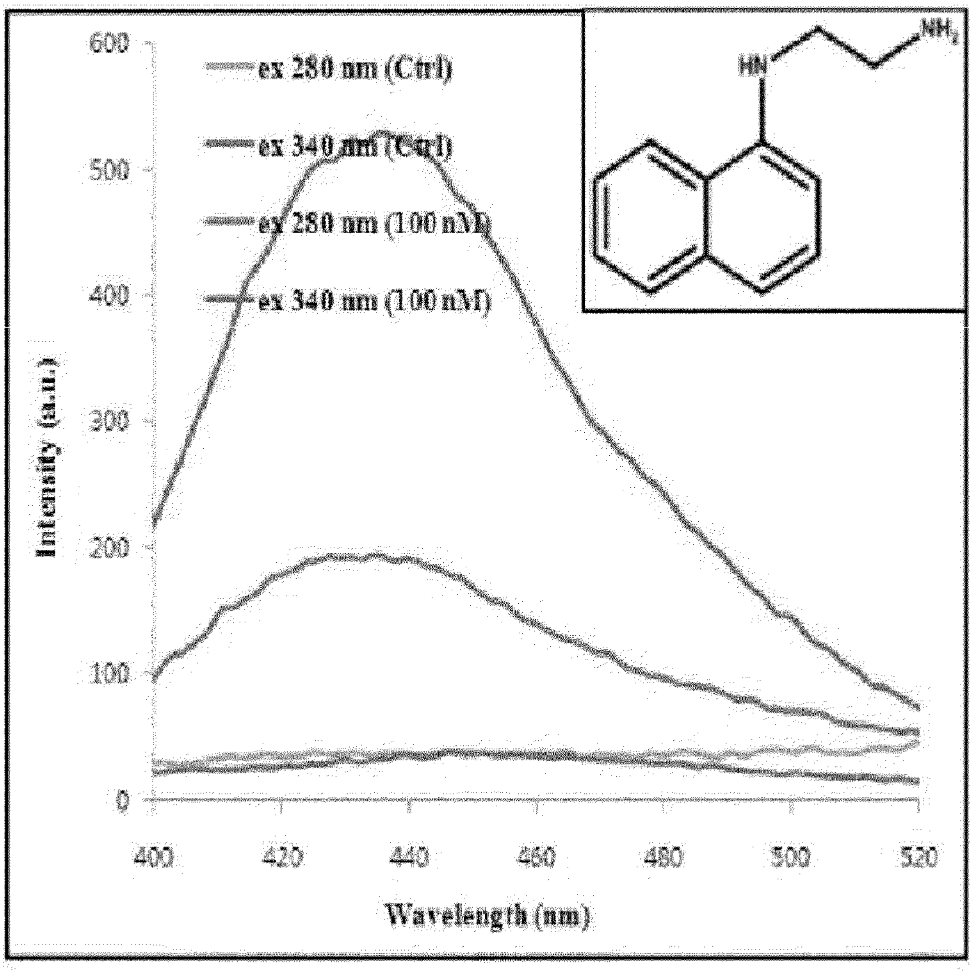
ex 280 nm (Ctrl)
ex 340 nm (Ctrl)
ex 280 nm (100 nM)
ex 340 nm (100 nM)
Intensity (a.u.)
600
500
400
300
200
100
0
400
420
440
460
480
500
520
Wavelength (nm)

[FIG. 4]

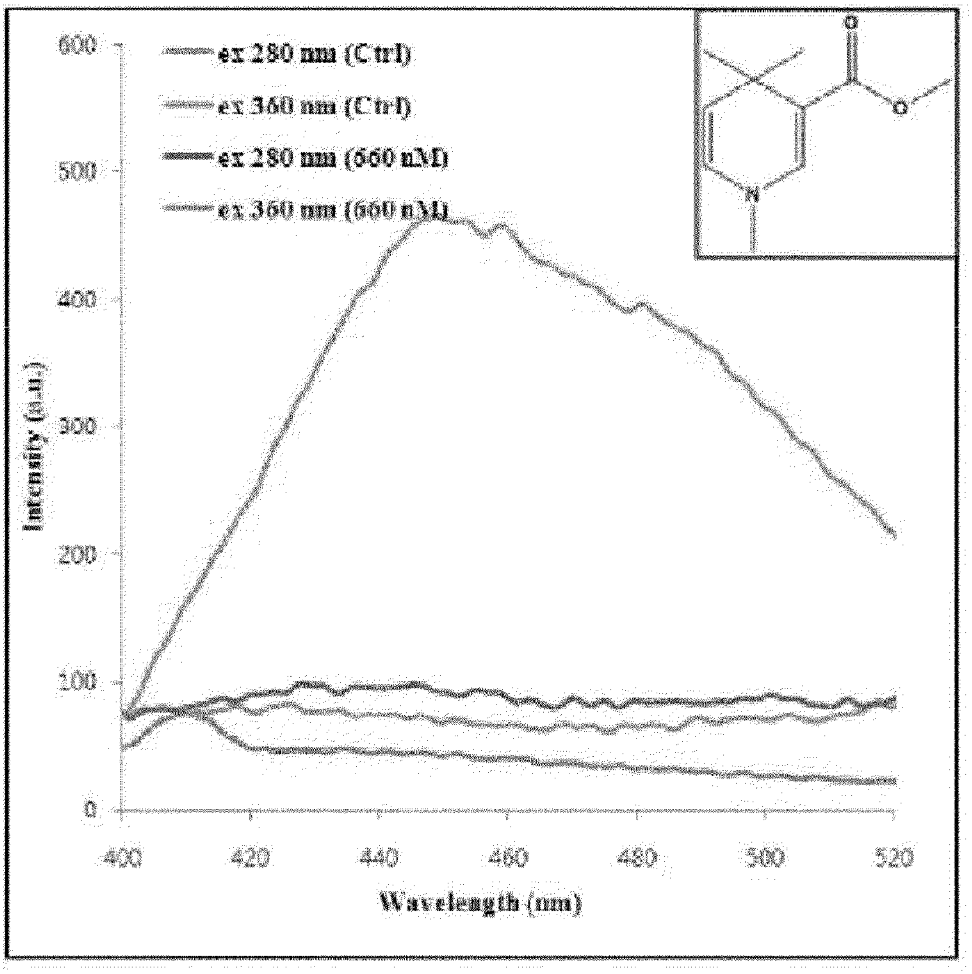
ex 280 nm (Ctrl)
ex 360 nm (Ctrl)
ex 280 nm (660 nM)
ex 360 nm (660 nM)
Intensity (a.u.)
0
100
200
300
400
500
600
Wavelength (nm)
400
420
440
460
480
500
520

[FIG. 5]

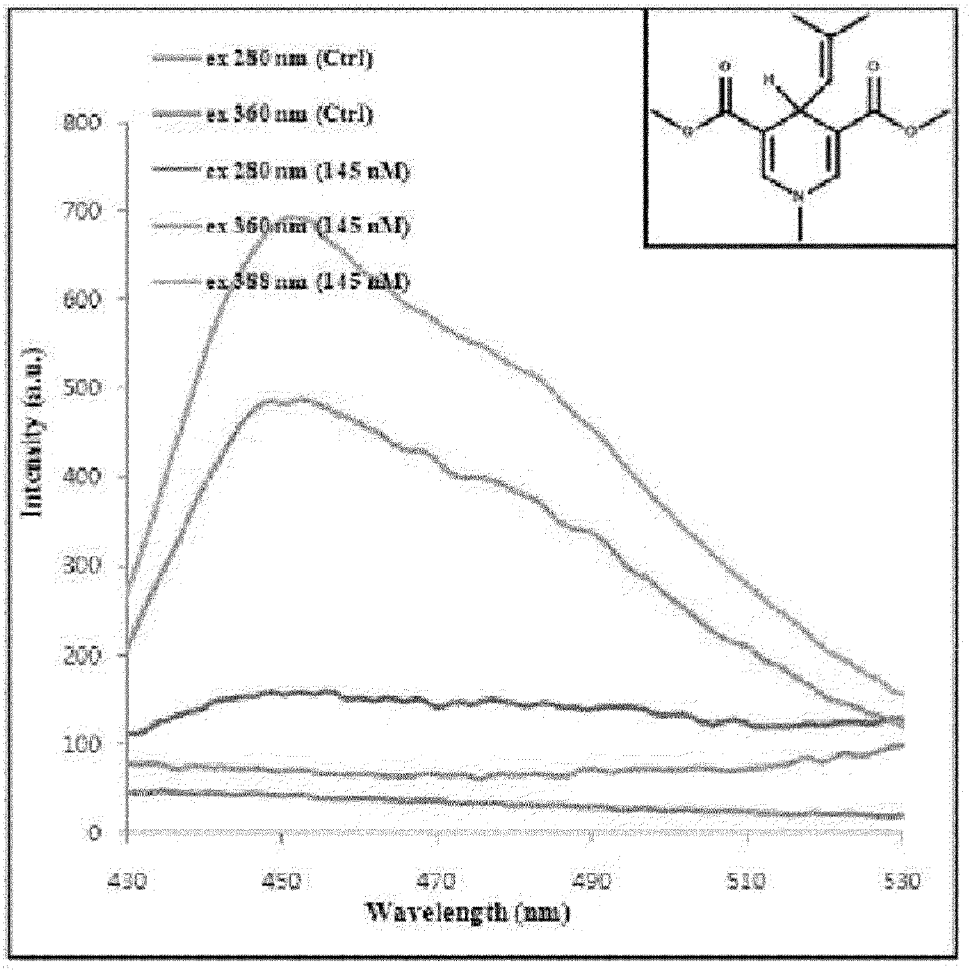
ex 280 nm (Ctrl)
ex 360 nm (Ctrl)
ex 280 nm (145 nM)
ex 360 nm (145 nM)
ex 388 nm (145 nM)
Intensity (a.u.)
0
100
200
300
400
500
600
700
800
430
450
470
490
510
530
Wavelength (nm)

[FIG. 6]

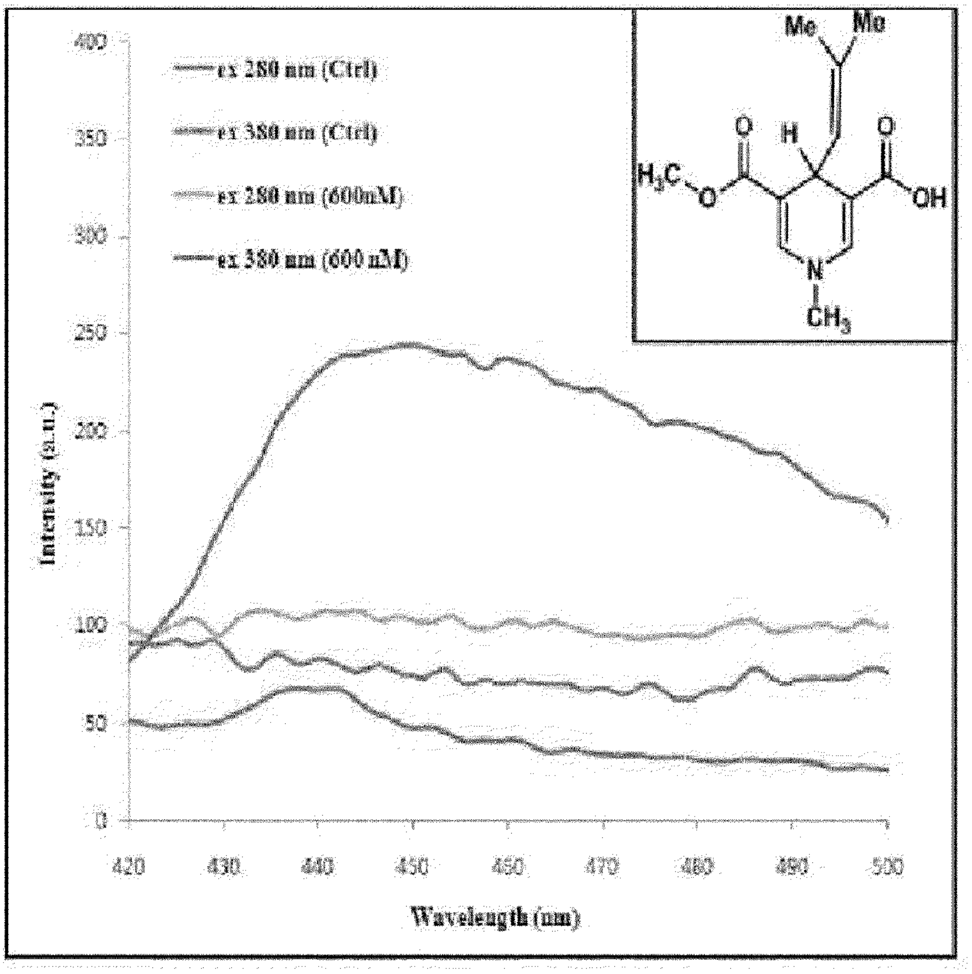
ex 280 nm (Ctrl)
ex 380 nm (Ctrl)
ex 280 nm (600nM)
ex 380 nm (600 nM)
Intensity (a.u.)
Wavelength (nm)

[FIG. 7]

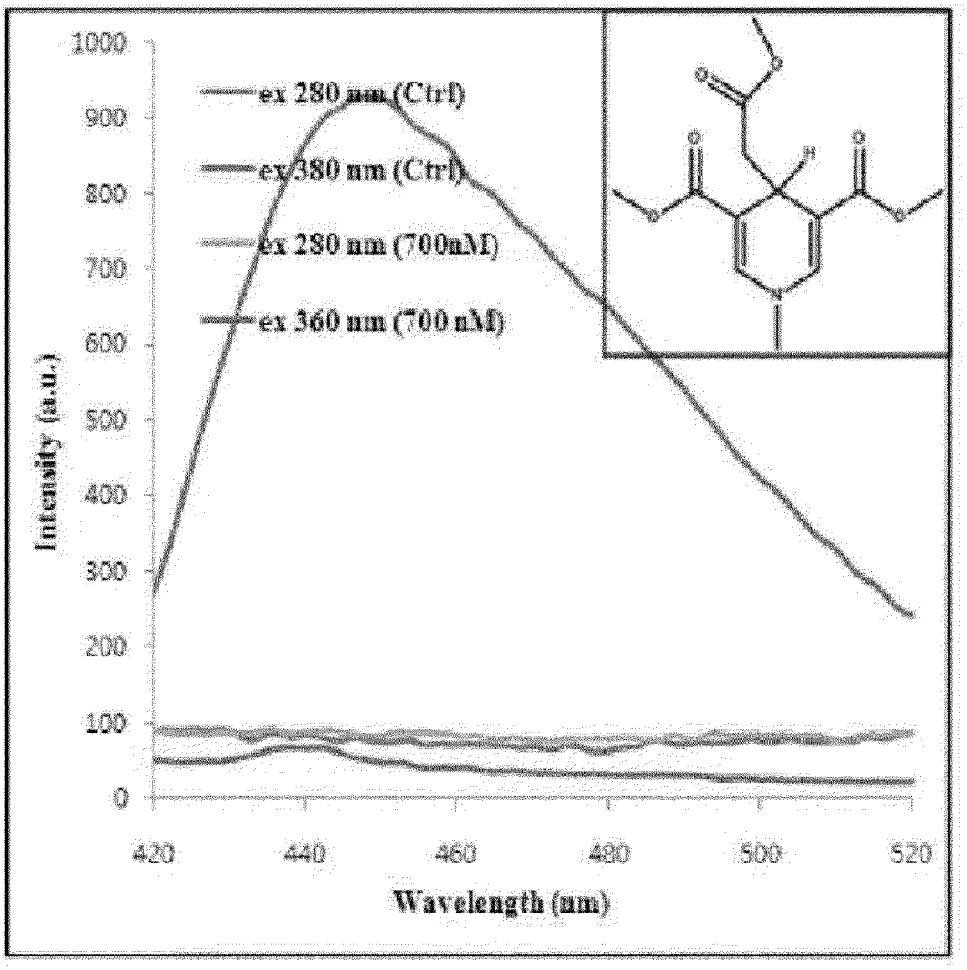
ex 280 nm (Ctrl)
ex 380 nm (Ctrl)
ex 280 nm (700nM)
ex 360 nm (700 nM)
Intensity (a.u.)
Wavelength (nm)
0
100
200
300
400
500
600
700
800
900
1000
420
440
460
480
500
520

[FIG. 8]

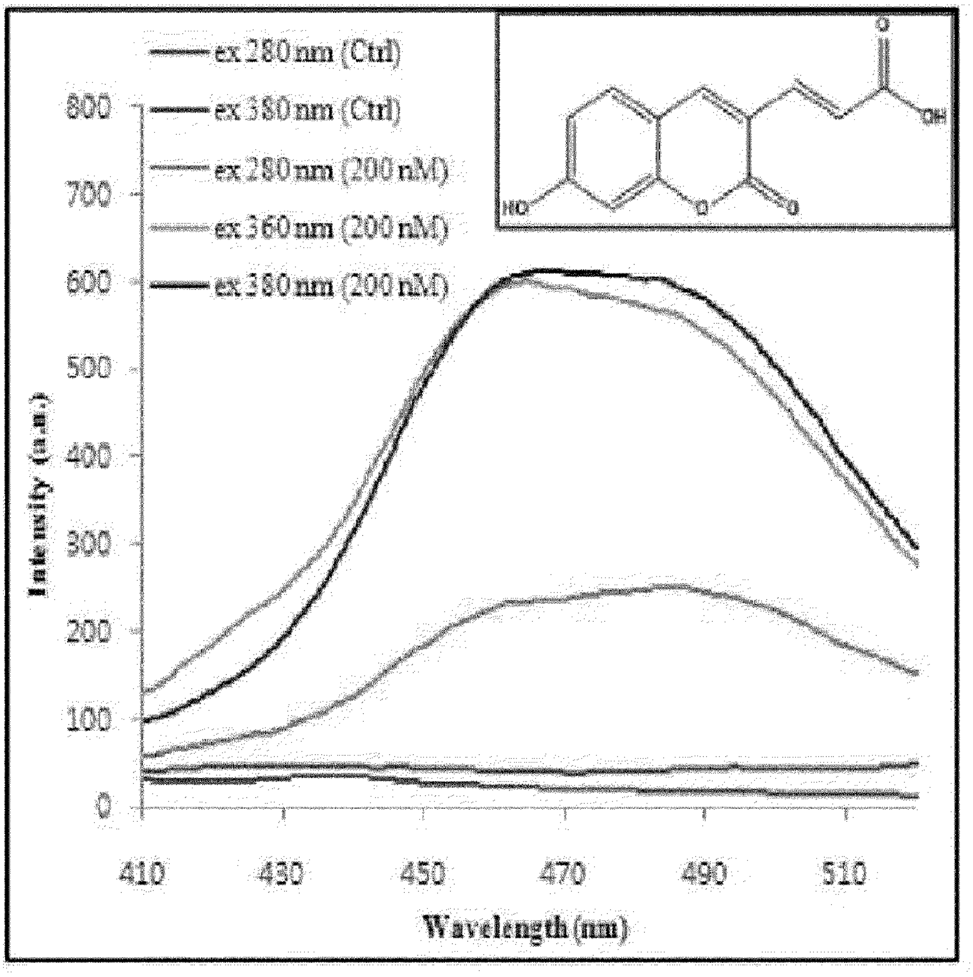
ex 280 nm (Ctrl)
ex 380 nm (Ctrl)
ex 280 nm (200 nM)
ex 360 nm (200 nM)
ex 380 nm (200 nM)
Intensity (a.u.)
0
100
200
300
400
500
600
700
800
Wavelength (nm)
410
430
450
470
490
510

[FIG. 9]

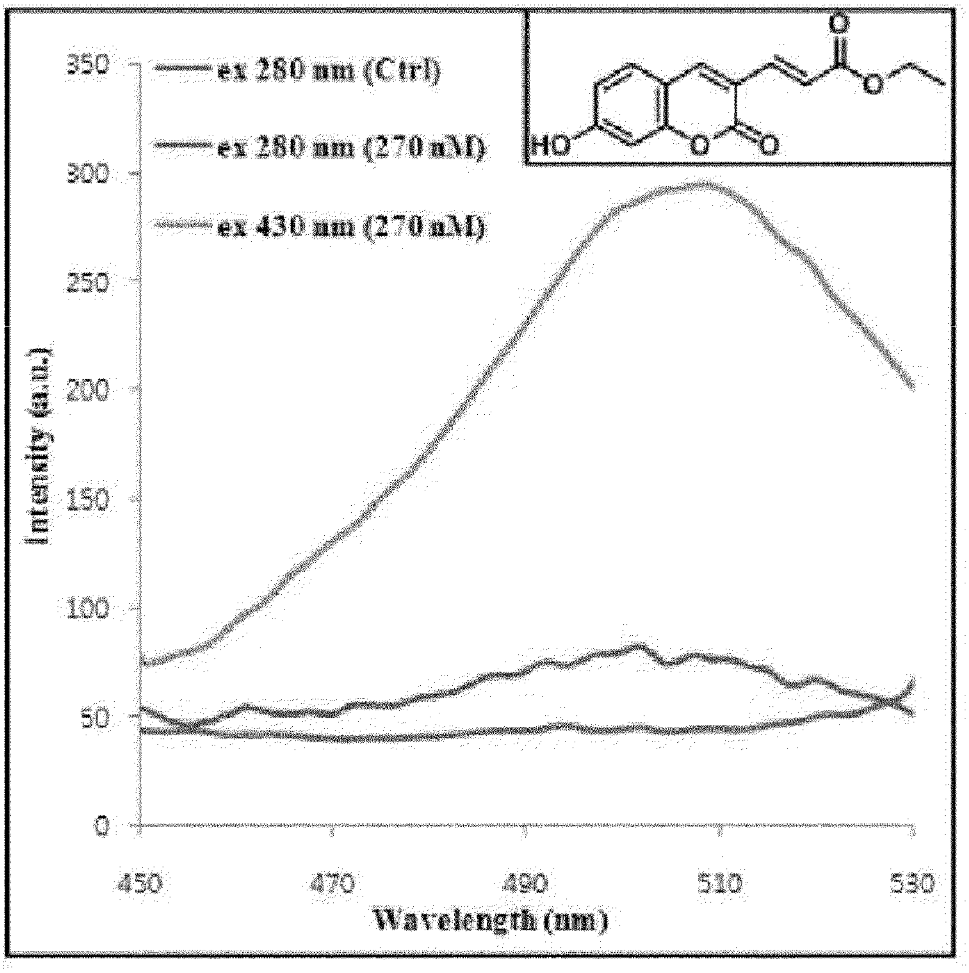
ex 280 nm (Ctrl)
ex 280 nm (270 nM)
ex 430 nm (270 nM)
Intensity (a.u.)
350
300
250
200
150
100
50
0
450
470
490
510
530
Wavelength (nm)

[FIG. 10]

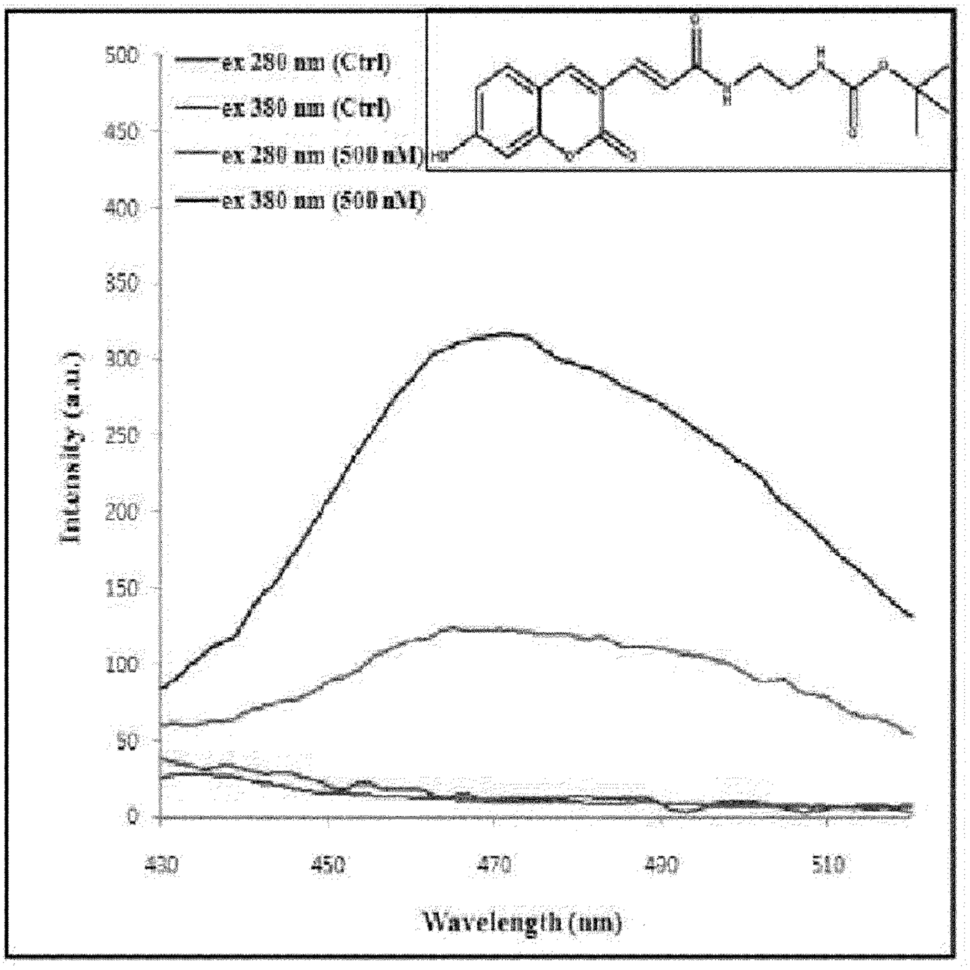
ex 280 nm (Ctrl)
ex 380 nm (Ctrl)
ex 280 nm (500 nM)
ex 380 nm (500 nM)
Intensity (a.u.)
0
50
100
150
200
250
300
350
400
450
500
430
450
470
490
510
Wavelength (nm)

[FIG. 11]

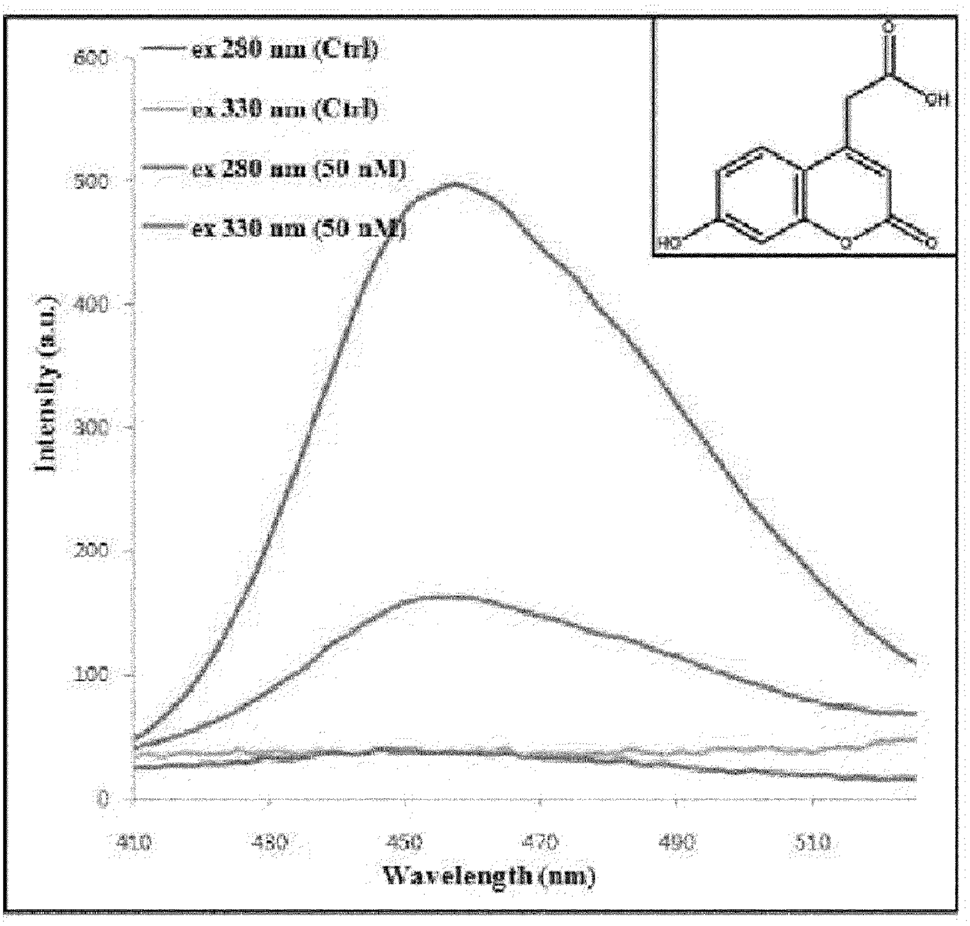
ex 280 nm (Ctrl)
ex 330 nm (Ctrl)
ex 280 nm (50 nM)
ex 330 nm (50 nM)
Intensity (a.u.)
Wavelength (nm)
0
100
200
300
400
500
600
410
430
450
470
490
510
HO
O
OH

[FIG. 12]

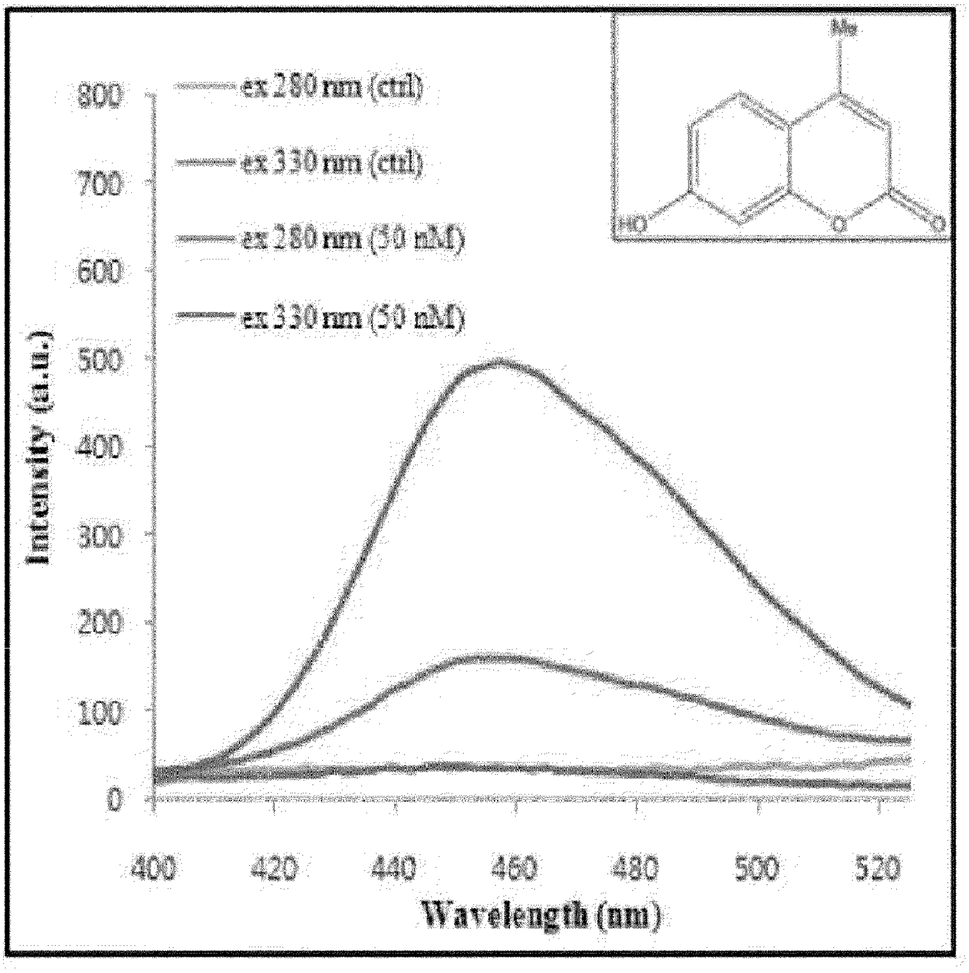
ex 280 nm (ctrl)
ex 330 nm (ctrl)
ex 280 nm (50 nM)
ex 330 nm (50 nM)
Intensity (a.u.)
0
100
200
300
400
500
600
700
800
Wavelength (nm)
400
420
440
460
480
500
520

[FIG. 13]

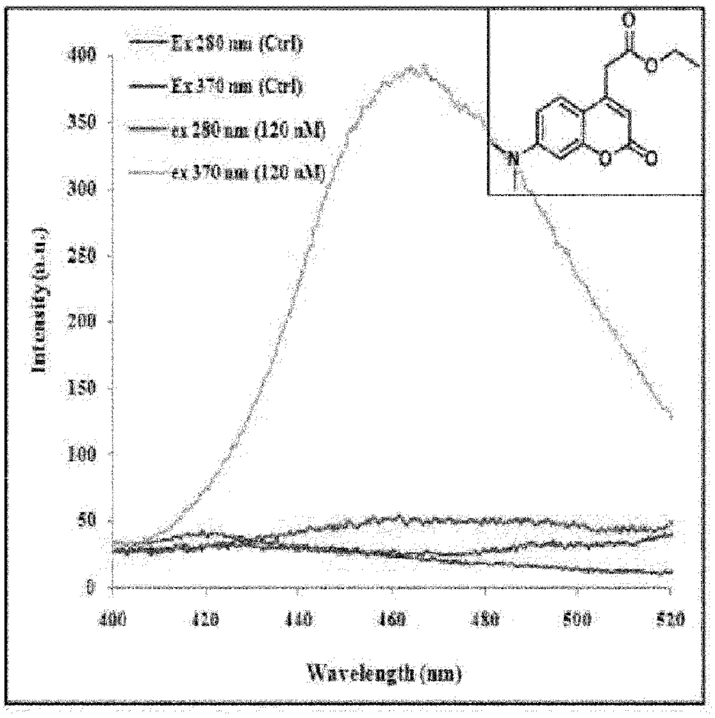
Ex 280 nm (Ctrl)
Ex 370 nm (Ctrl)
ex 280 nm (120 nM)
ex 370 nm (120 nM)
Intensity (a.u.)
0
50
100
150
200
250
300
350
400
400
420
440
460
480
500
520
Wavelength (nm)

[FIG. 14]

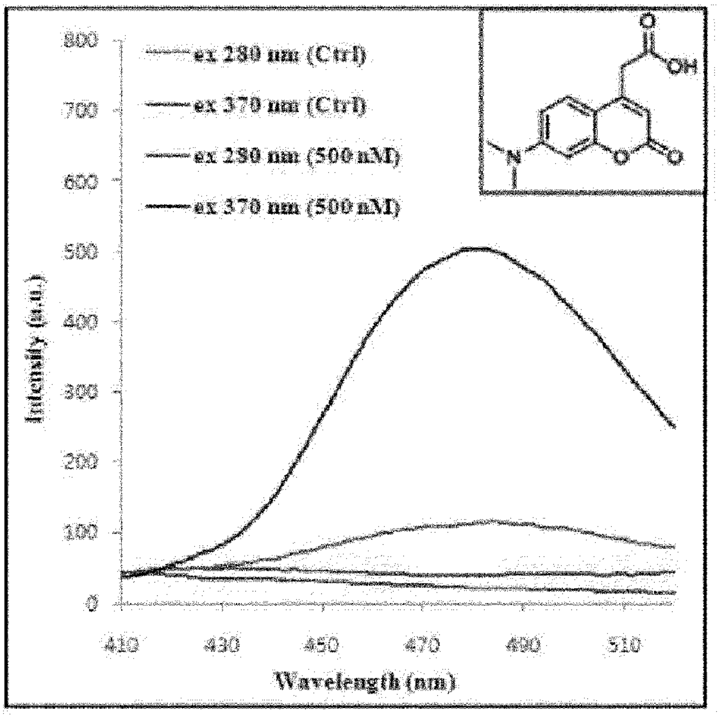
ex 280 nm (Ctrl)
ex 370 nm (Ctrl)
ex 280 nm (500 nM)
ex 370 nm (500 nM)
Intensity (a.u.)
0
100
200
300
400
500
600
700
800
410
430
450
470
490
510
Wavelength (nm)

[FIG. 15]

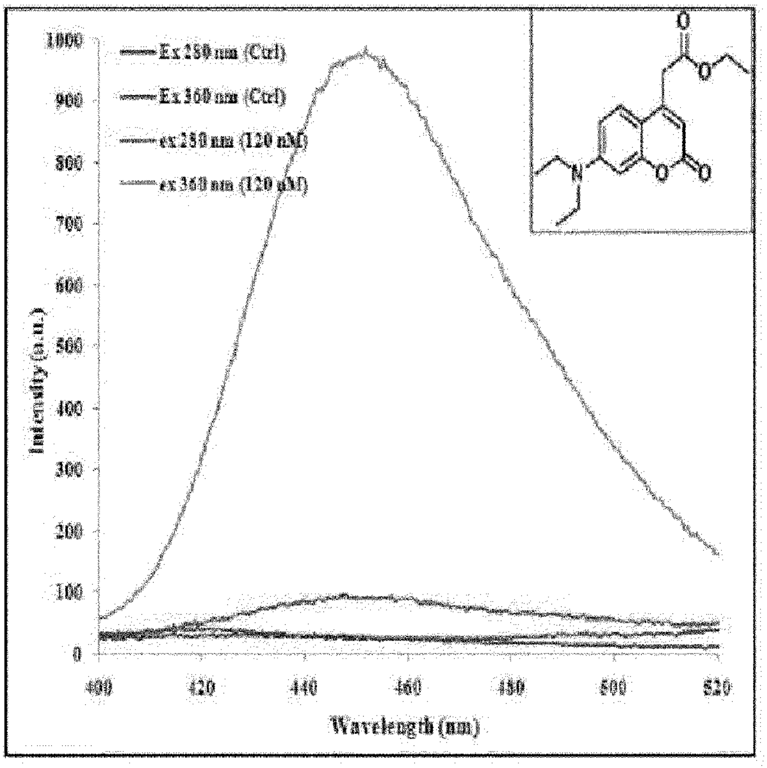
Ex 280 nm (Ctrl)
Ex 360 nm (Ctrl)
ex 280 nm (120 nM)
ex 360 nm (120 nM)
Intensity (a.u.)
Wavelength (nm)

[FIG. 16]

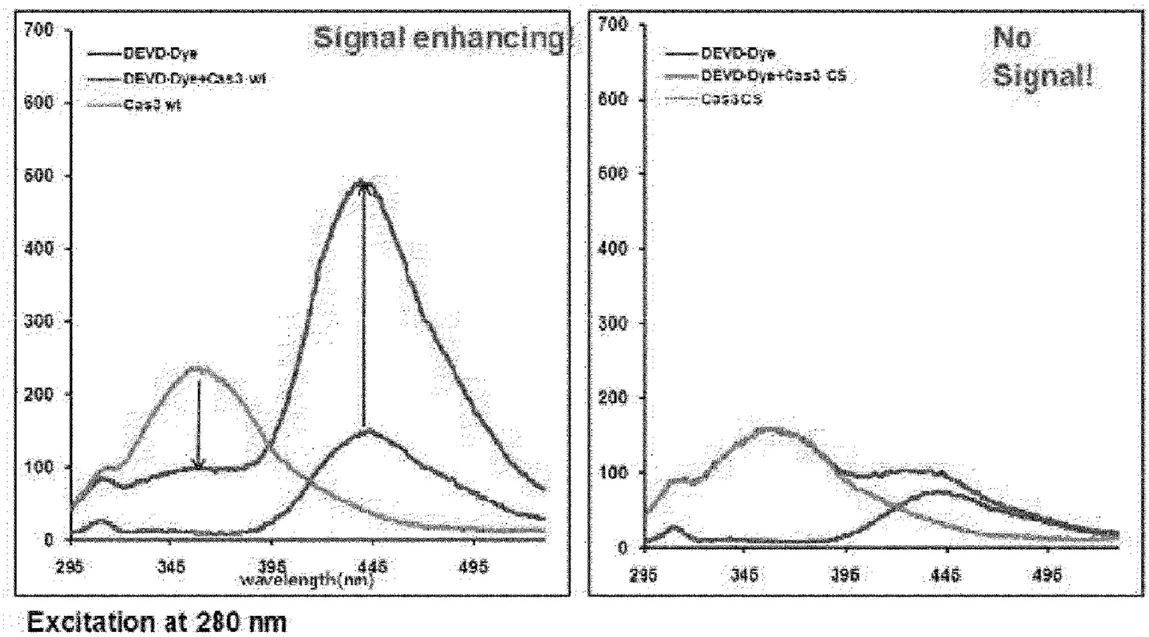
Signal enhancing!
DEVD-Dye
DEVD-Dye+Cas3 wt
Cas3 wt
wavelength(nm)
No Signal!
DEVD-Dye
DEVD-Dye+Cas3 CS
Cas3 CS
Excitation at 280 nm

[FIG. 17]

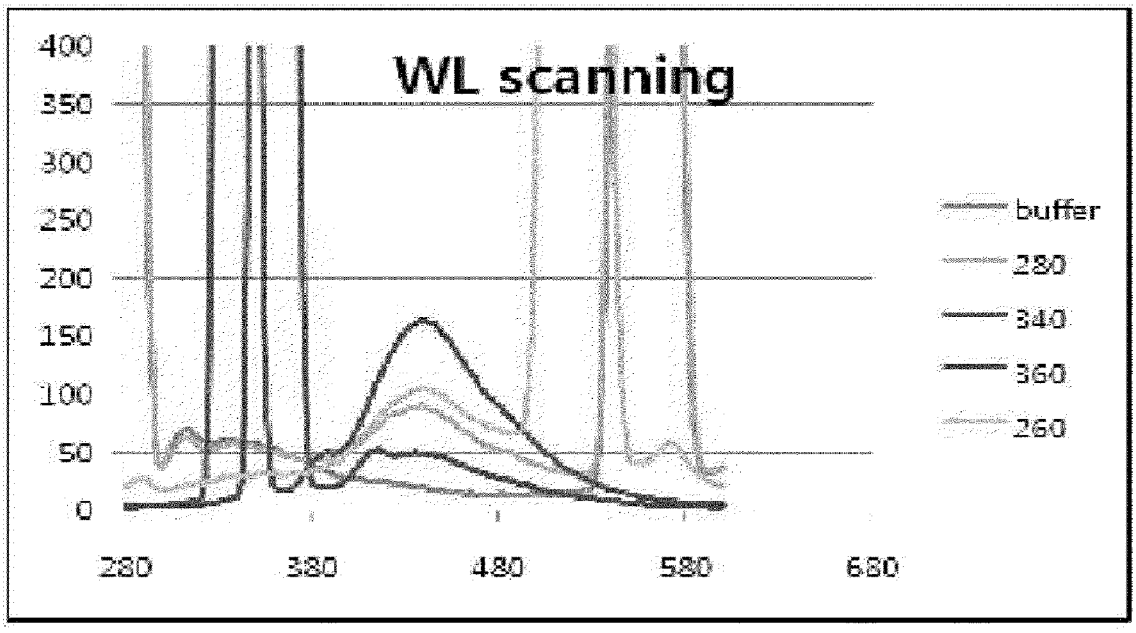
WL scanning
400
350
300
250
200
150
100
50
0
280
380
480
580
680
buffer
280
340
360
260

[FIG. 18]

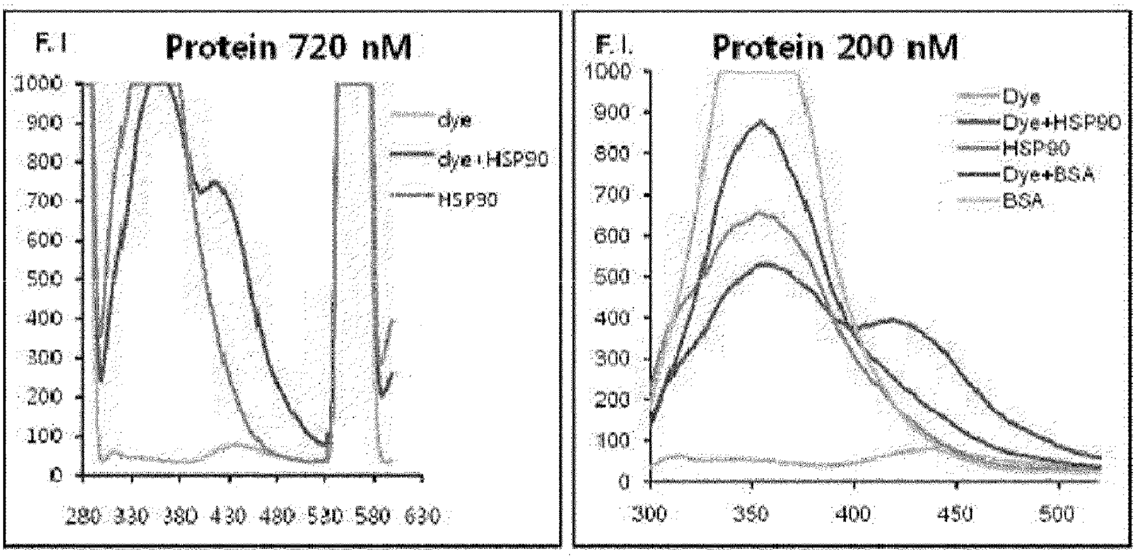
F.I
Protein 720 nM
1000
900
800
700
600
500
400
300
200
100
0
280 330 380 430 480 530 580 630
dye
dye+HSP90
HSP90
F.I.
Protein 200 nM
1000
900
800
700
600
500
400
300
200
100
0
300
350
400
450
500
Dye
Dye+HSP90
HSP90
Dye+BSA
BSA

[FIG. 19]

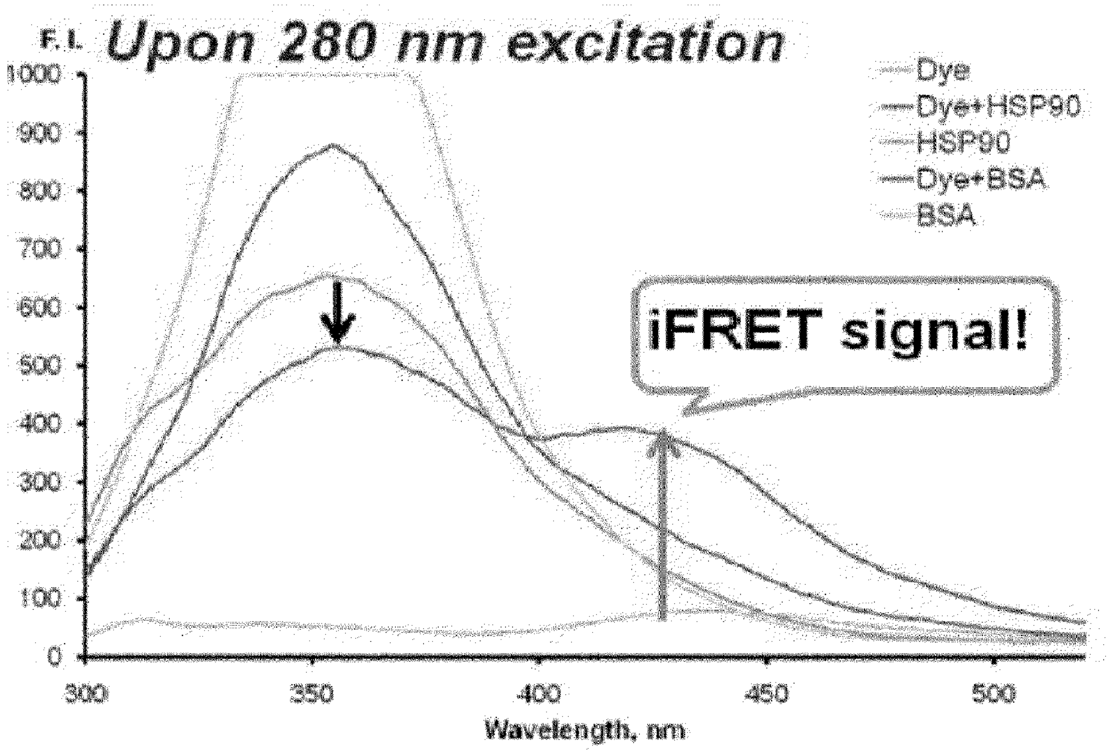
F.I.
Upon 280 nm excitation
1000
900
800
700
600
500
400
300
200
100
0
300
350
400
450
500
Wavelength, nm
Dye
Dye+HSP90
HSP90
Dye+BSA
BSA
iFRET signal!

[FIG. 20]

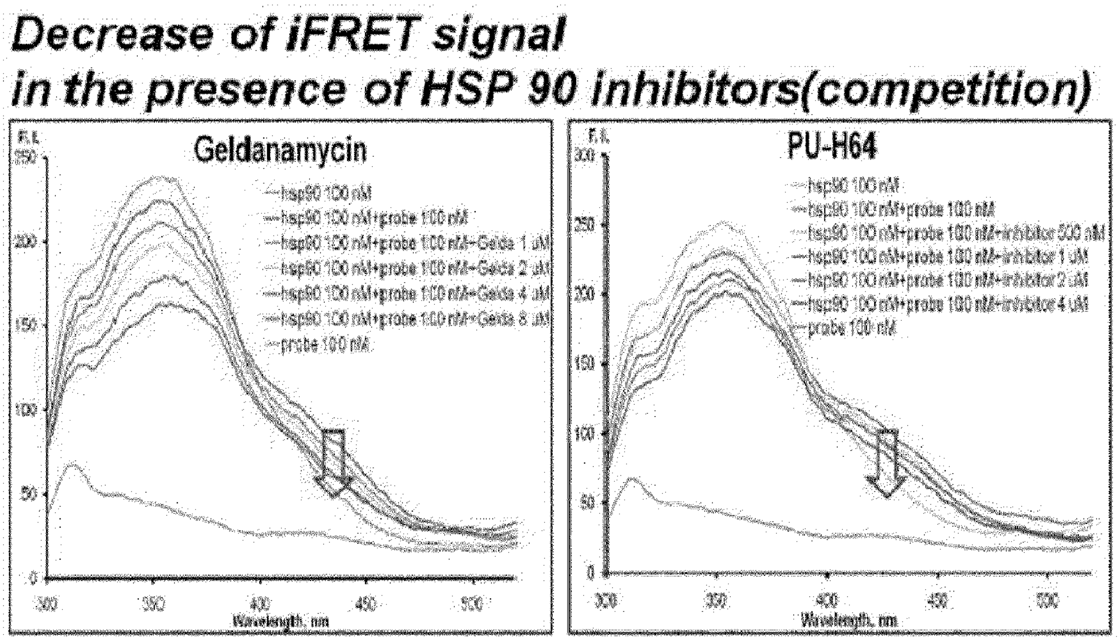
Decrease of iFRET signal
in the presence of HSP 90 inhibitors(competition)
Geldanamycin
F.I.
hsp90 100 nM
hsp90 100 nM+probe 100 nM
hsp90 100 nM+probe 100 nM+Gelda 1 uM
hsp90 100 nM+probe 100 nM+Gelda 2 uM
hsp90 100 nM+probe 100 nM+Gelda 4 uM
hsp90 100 nM+probe 100 nM+Gelda 8 uM
probe 100 nM
Wavelength, nm
PU-H64
F.I.
hsp90 100 nM
hsp90 100 nM+probe 100 nM
hsp90 100 nM+probe 100 nM+inhibitor 500 nM
hsp90 100 nM+probe 100 nM+inhibitor 1 uM
hsp90 100 nM+probe 100 nM+inhibitor 2 uM
hsp90 100 nM+probe 100 nM+inhibitor 4 uM
probe 100 nM
Wavelength, nm

[FIG. 21]

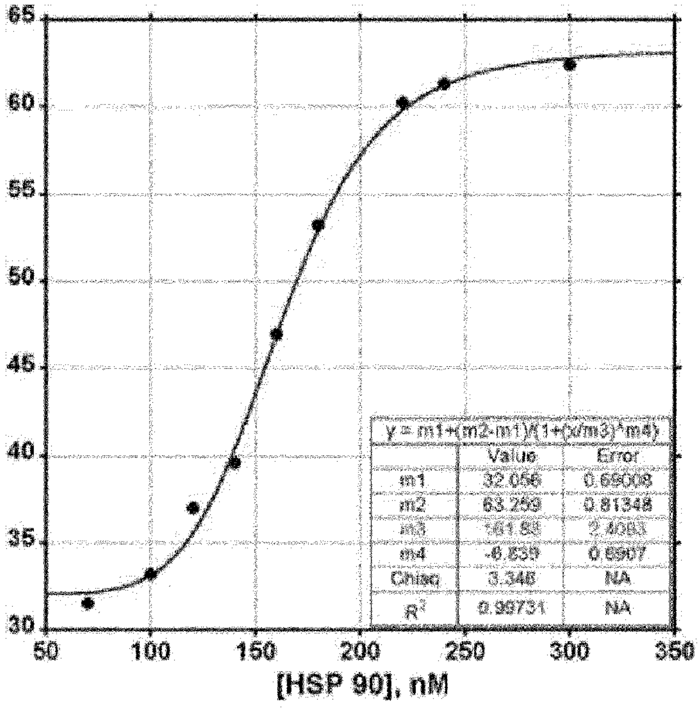
65
60
55
50
45
40
35
30
50
100
150
200
250
300
350
[HSP 90], nM
y = m1+(m2-m1)/(1+(x/m3)^m4)
Value
Error
m1
32.056
0.69008
m2
63.259
0.81348
m3
161.83
2.4093
m4
-6.839
0.6907
Chisq
3.348
NA
R^2
0.99731
NA

[FIG. 22]

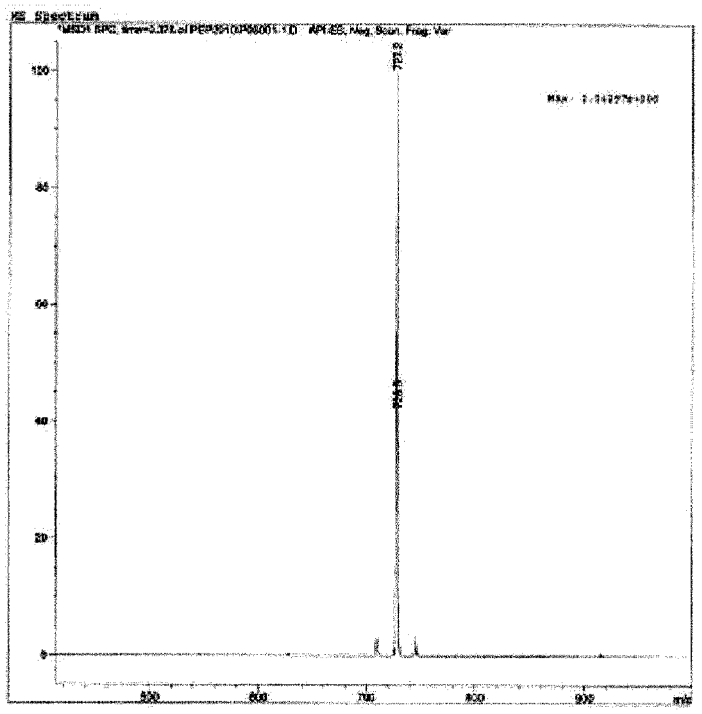
MS Spectrum
100
80
60
40
20
0
500
600
700
800
900
m/z

[FIG. 23]

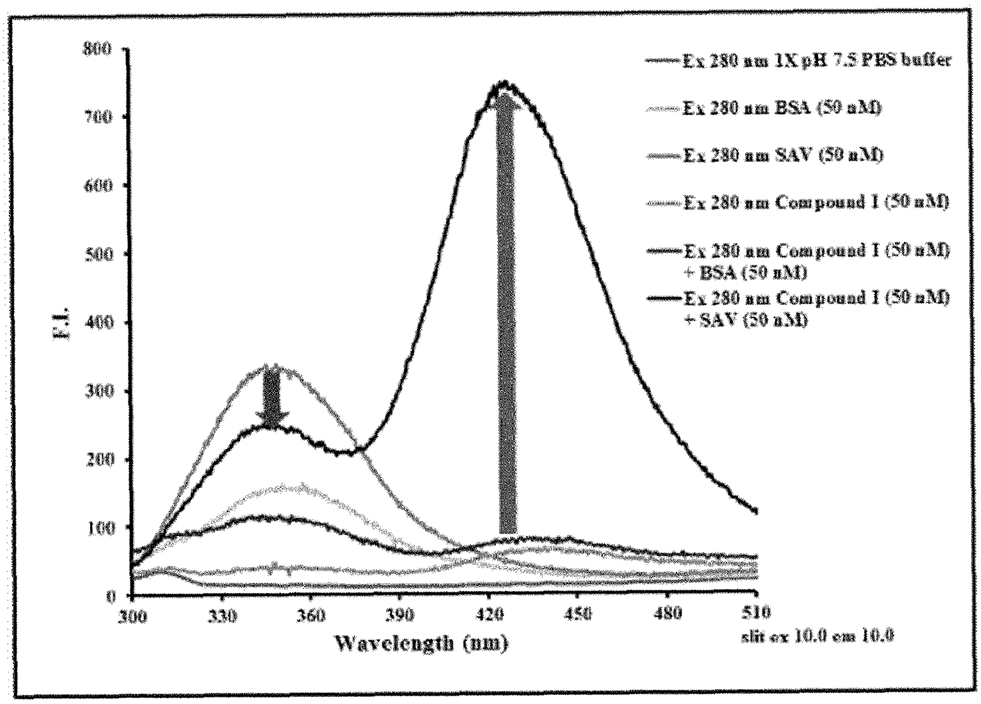
Ex 280 nm 1X pH 7.5 PBS buffer
Ex 280 nm BSA (50 nM)
Ex 280 nm SAV (50 nM)
Ex 280 nm Compound I (50 nM)
Ex 280 nm Compound I (50 nM) + BSA (50 nM)
Ex 280 nm Compound I (50 nM) + SAV (50 nM)
F.I.
800
700
600
500
400
300
200
100
0
300
330
360
390
420
450
480
510
Wavelength (nm)
slit ex 10.0 em 10.0

[FIG. 24]

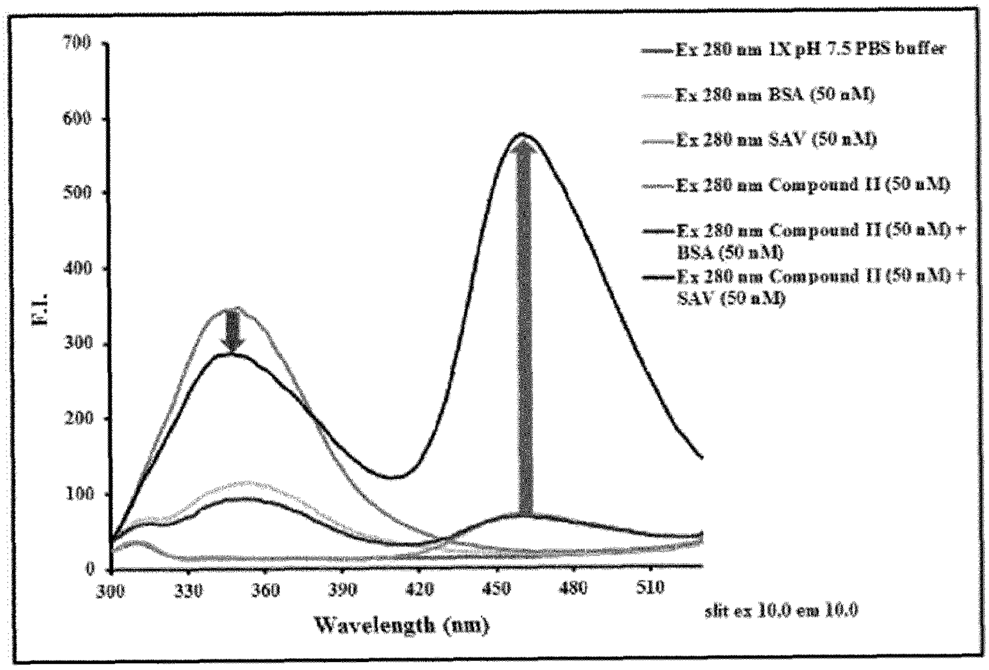
Ex 280 nm 1X pH 7.5 PBS buffer
Ex 280 nm BSA (50 nM)
Ex 280 nm SAV (50 nM)
Ex 280 nm Compound II (50 nM)
Ex 280 nm Compound II (50 nM) + BSA (50 nM)
Ex 280 nm Compound II (50 nM) + SAV (50 nM)
F.I.
700
600
500
400
300
200
100
0
300
330
360
390
420
450
480
510
Wavelength (nm)
slit ex 10.0 em 10.0

[FIG. 25]

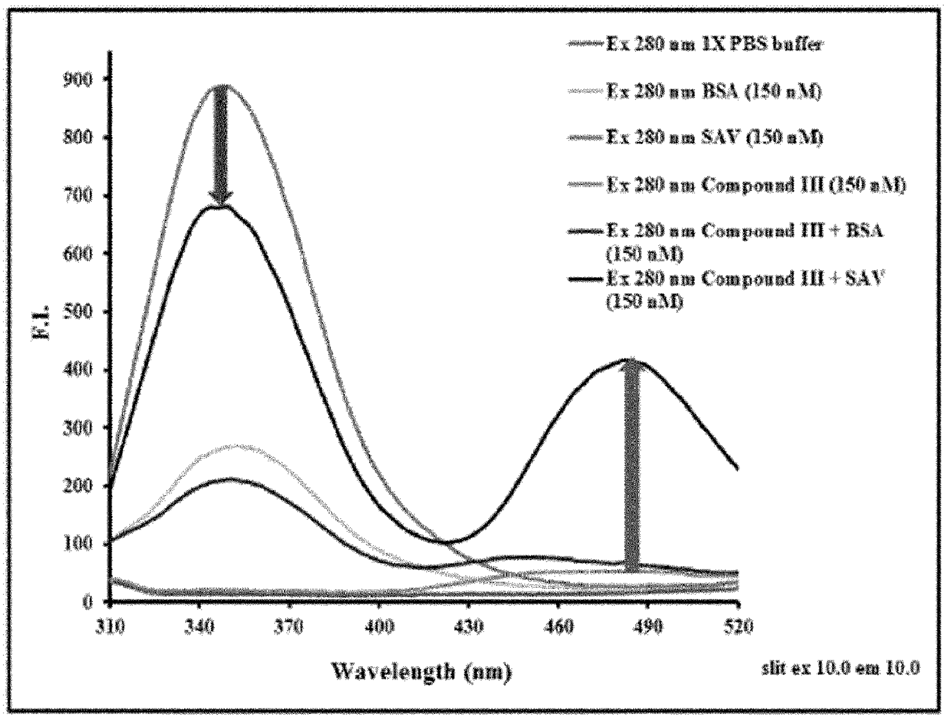
Ex 280 nm 1X PBS buffer
Ex 280 nm BSA (150 nM)
Ex 280 nm SAV (150 nM)
Ex 280 nm Compound III (150 nM)
Ex 280 nm Compound III + BSA (150 nM)
Ex 280 nm Compound III + SAV (150 nM)
F.I.
900
800
700
600
500
400
300
200
100
0
310
340
370
400
430
460
490
520
Wavelength (nm)
slit ex 10.0 em 10.0

[FIG. 26]

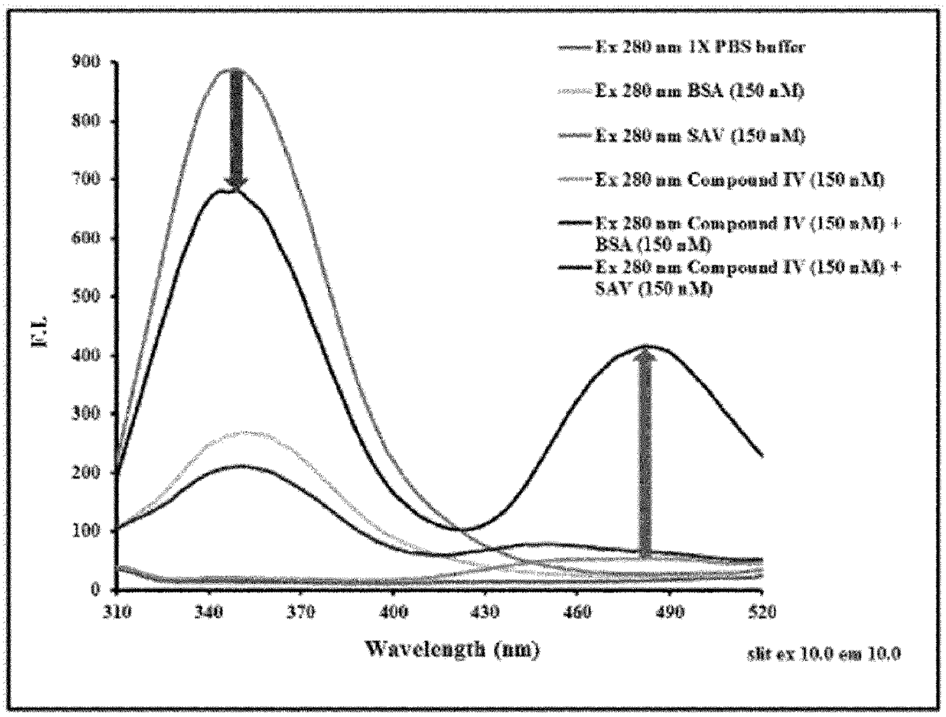
Ex 280 nm 1X PBS buffer
Ex 280 nm BSA (150 nM)
Ex 280 nm SAV (150 nM)
Ex 280 nm Compound IV (150 nM)
Ex 280 nm Compound IV (150 nM) + BSA (150 nM)
Ex 280 nm Compound IV (150 nM) + SAV (150 nM)
F.I.
900
800
700
600
500
400
300
200
100
0
310
340
370
400
430
460
490
520
Wavelength (nm)
slit ex 10.0 em 10.0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080311047 A
- WO 2008115262 A


### Non-patent literature cited in the description


- **MIYAWAKI et al.** *Nature,* 1997, vol. 388, 882-887
- *Angew. Chem. Int. Ed.,* 2006, vol. 45, 4562-4588
- **LAKOWICZ, J.R.** Principles of Fluorescence Spectroscopy. Plenum Press, 1999
- **PATTERSON et al.** *Anal. Biochem.,* 2000, vol. 284, 438
- **PATTERSON et al.** *J. of Cell Sci.,* 2001, vol. 114, 837
- *JACS,* 2000, vol. 122, 87-95
- *JOC,* 2005, vol. 70, 717-720
- *J. Med. Chem.,* 2006, vol. 49, 4953-4960